# EUROPÄISCHE PATENTSCHRIFT

(11) **EP 0 793 644 B1**
(45) Veröffentlichungstag und Bekanntmachung des Hinweises auf die Patenterteilung: **24.03.1999**
(21) Anmeldenummer: 95937887.8
(22) Anmeldetag: 10.11.1995
(51) Int. Cl.: C07C 271/12, A01N 47/24

(54) **2-(4-BIPHENYL-OXYMETHYLEN)-ANILIDE, VERFAHREN UND ZWISCHENPRODUKTE ZU IHRER HERSTELLUNG UND IHRE VERWENDUNG ALS SCHÄDLINGSBEKÄMPFUNGSMITTEL**
2-(4-BIPHENYL-OXYMETHYLENE)ANILIDES, PROCESS AND INTERMEDIATE PRODUCTS FOR PREPARING THE SAME AND THEIR USE AS PESTICIDES
2-(4-BIPHENYL-OXYMETHYLENE)ANILIDES, PROCEDE ET PRODUITS INTERMEDIAIRES DE PREPARATION DE CELLES-CI ET LEUR UTILISATION COMME PESTICIDES

(30) Priorität: 23.11.1994 DE 4441673
(43) Veröffentlichungstag der Anmeldung: 10.09.1997
(73) Patentinhaber: BASF AKTIENGESELLSCHAFT, 67056 Ludwigshafen (DE)
(72) Erfinder: MÜLLER, Bernd, D-67227 Frankenthal (DE); GRAMMENOS, Wassilios, D-67063 Ludwigshafen (DE); SAUTER, Hubert, D-68167 Mannheim (DE); RÖHL, Franz, D-67105 Schifferstadt (DE); AMMERMANN, Eberhard, D-64646 Heppenheim (DE); LORENZ, Gisela, D-67434 Hambach (DE); GÖTZ, Norbert, D-67547 Worms (DE)
(86) Internationale Anmeldenummer: EP9504429
(87) Internationale Veröffentlichungsnummer: WO9616029

(56) Entgegenhaltungen:
- EP-A- 0 619 301

## Beschreibung

Die vorliegende Erfindung betrifft 2-[4-Biphenyl-oxymethylen]-anilide der Formel I 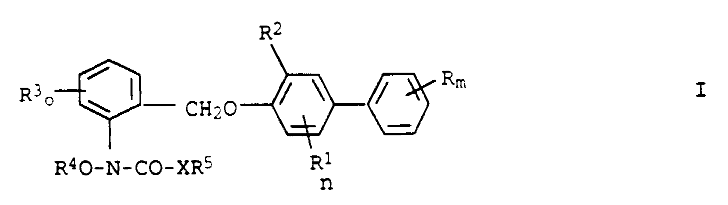 in der die Indices und die Substituenten die folgende Bedeutung haben:
- R: Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino, C₁-C₄-Alkylcarbonyl, C₁-C₄-Alkoxycarbonyl, C₁-C₄-Alkylaminocarbonyl, Di-(C₁-C₄-alkyl)aminocarbonyl, C₁-C₄-Alkylcarbonylamino, C₁-C₄-Alkylcarbonyl-(C₁-C₄-alkylamino), C₂-C₄-Alkenyl, C₃-C₄-Alkenyloxy, C₂-C₄-Alkinyl, C₃-C₄-Alkinyloxy, C₃-C₆-Cycloalkyl oder C(R^{a})=NOR^{b};
R^{a},R^{b} Wasserstoff, Alkyl, Alkenyl, Alkinyl, Cycloalkyl oder Cycloalkenyl;
- m: 1, 2, 3, 4 oder 5, wobei die Reste R verschieden sein können, wenn m größer als 1 ist;
- R¹: Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino, C₁-C₄-Alkylcarbonyl, C₁-C₄-Alkoxycarbonyl, C₁-C₄-Alkylaminocarbonyl, Di-(C₁-C₄-alkyl)aminocarbonyl, C₁-C₄-Alkylcarbonylamino, C₁-C₄-Alkylcarbonyl-(C₁-C₄-alkylamino), C₂-C₄-Alkenyl, C₃-C₄-Alkenyloxy, C₂-C₄-Alkinyl, C₃-C₄-Alkinyloxy, C₃-C₆-Cycloalkyl oder C(R^{a})=NOR^{b};
- n: 0, 1 oder 2, wobei die Reste R¹ verschieden sein können, wenn n für 2 steht;
- R²: Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino, C₁-C₄-Alkylcarbonyl, C₁-C₄-Alkoxycarbonyl, C₁-C₄-Alkylaminocarbonyl, Di-(C₁-C₄-alkyl)aminocarbonyl, C₁-C₄-Alkylcarbonylamino, C₁-C₄-Alkylcarbonyl-(C₁-C₄-alkylamino), C₂-C₄-Alkenyl, C₃-C₄-Alkenyloxy, C₂-C₄-Alkinyl, C₃-C₄-Alkinyloxy, C₃-C₆-Cycloalkyl oder C(R^{a})=NOR^{b};
- R³: Nitro, Cyano, Halogen,
ggf. subst. Alkyl, Alkenyl, Alkinyl, Alkoxy, Alkenyloxy, Alkinyloxy oder
C₁-C₄-Alkylcarbonyl, C₁-C₄-Alkoxycarbonyl, C₁-C₄-Alkylaminocarbonyl, Di-(C₁-C₄-alkyl)aminocarbonyl, C₁-C₄-Alkylcarbonylamino, C₁-C₄-Alkylcarbonyl-(C₁-C₄-alkylamino), C(R^{a})=NOR^{b} oder
für den Fall, daß o größer als 1 ist, zusätzlich eine an zwei benachbarte Ringatome gebundene ggf. subst. Brücke, welche drei bis vier Glieder aus der Gruppe 3 oder 4 Kohlenstoffatome, oder 2 oder 3 Kohlenstoffatome und 1 oder 2 Stickstoff-, Sauerstoff- und/oder Schwefelatome besitzt, wobei diese Brücke gemeinsam mit dem Ring an den sie gebunden ist einen partiell ungesättigten oder aromatischen Rest bilden kann;
- o: 0, 1, 2, 3 oder 4, wobei die Substituenten R³ verschieden sein können, wenn n größer als 1 ist;
- R⁴: Wasserstoff,
ggf. subst. Alkyl, Alkenyl, Alkinyl, Cycloalkyl, Cycloalkenyl, Alkylcarbonyl oder Alkoxycarbonyl;
- X: eine direkte Bindung oder CH₂, O oder NR^{c};
- R^{c}: Wasserstoff, Alkyl, Alkenyl, Alkinyl, Cycloalkyl oder Cycloalkenyl;
- R⁵: Alkyl, Alkenyl, Alkinyl, Cycloalkyl oder Cycloalkenyl, oder
für den Fall, daß X für NR^{c} steht, zusätzlich Wasserstoff.

Desweiteren betrifft die Erfindung Verfahren und Zwischenprodukte zur Herstellung dieser Verbindungen, sie enthaltende Mittel sowie ihre Verwendung zur Bekämpfung von tierischen Schädlingen oder Schadpilzen.

In der WO-A 93/15,046 sind 2-[4-Biphenyl-oxymethylen]-anilide in allgemeiner Form zur Bekämpfung von tierischen Schädlingen und Schadpilzen beschrieben, wobei die dort beschriebenen Wirkstoffe dieser Gruppe entweder am einen oder am anderen Phenylring des Biphenylsystems Substituenten tragen. Diese Verbindungen lassen jedoch hinsichtlich des Bekämpfungserfolgs bzw. der benötigten Aufwandmengen für die Praxis zu wünschen übrig.

Der vorliegenden Erfindung lagen daher Verbindungen mit verbesserten Eigenschaften als Aufgabe zugrunde.

Demgemäß wurden die eingangs definierten Verbindungen I gefunden. Außerdem wurden Verfahren und Zwischenprodukte zu ihrer Herstellung, sie enthaltende Mittel und ihre Verwendung zur Bekämpfung von tierischen Schädlingen oder Schadpilzen gefunden.

Die Verbindungen I sind auf verschiedenen Wegen erhältlich.

Man erhält diejenigen Verbindungen I, in denen R⁴ Wasserstoff bedeutet und X für eine direkte Bindung oder Sauerstoff steht, beispielsweise dadurch, daß man ein Benzylderivat der Formel II in Gegenwart einer Base mit einem 4-Biphenol der Formel III in das entsprechende 2-[4-Biphenyl-oxymethylen]-nitrobenzol der Formel IV überführt, IV anschließend zum N-Hydroxylanilin der Formel Va reduziert und Va mit einer Carbonylverbindung der Formel VI in I umwandelt.

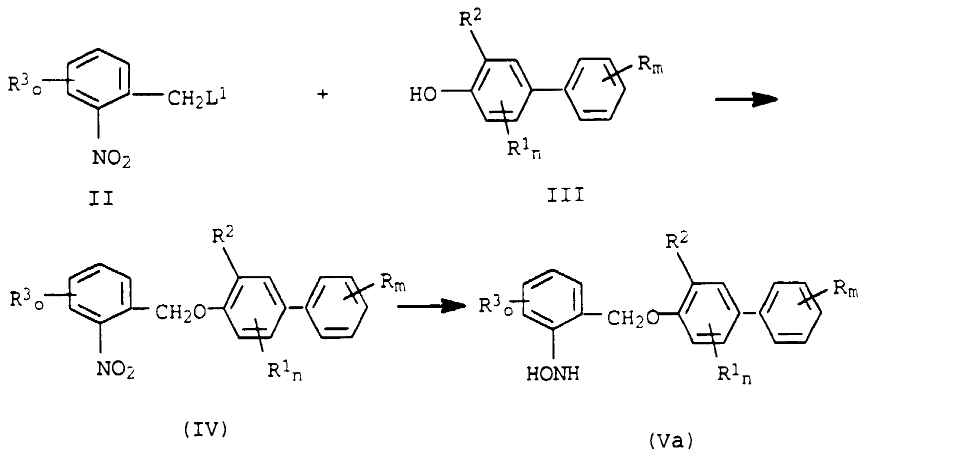

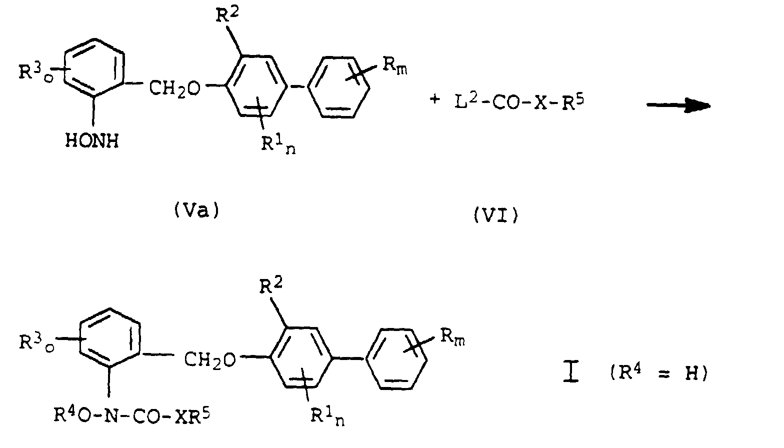

L¹ in der Formel II und L² in der Formel VI bedeuten jeweils eine nucleophil austauschbare Gruppe, beispielsweise Halogen (z.B. Chlor, Brom und Iod), oder ein Alkyl- oder Arylsulfonat (z.B. Methylsulfonat, Trifluormethylsulfonat, Phenylsulfonat und 4-Methylphenylsulfonat).

Die Veretherung der Verbindungen II und III wird üblicherweise bei Temperaturen von 0°C bis 80°C, vorzugsweise 20°C bis 60°C, durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Dimethylsulfoxid, Dimethylformamid, Dimethylacetamid, 1,3-Dimethylimidazolidin-2-on und 1,2-Dimethyltetrahydro-2(1H)-pyrimidin, vorzugsweise Methylenchlorid, Aceton und Dimethylformamid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall- und Erdalkalimetalloxide (z.B. Lithiumoxid, Natriumoxid, Calziumoxid und Magnesiumoxid), Alkalimetall- und Erdalkalimetallhydride (z.B. Lithiumhydrid, Natriumhydrid, Kaliumhydrid und Calziumhydrid), Alkalimetallamide (z.B. Lithiumamid, Natriumamid und Kaliumamid), Alkalimetall- und Erdalkalimetallcarbonate (z.B. Lithiumcarbonat und Calziumcarbonat) sowie Alkalimetallhydrogencarbonate (z.B. Natriumhydrogencarbonat), metallorganische Verbindungen, insbesondere Alkalimetallalkyle (z.B. wie Methyllithium, Butyllithium und Phenyllithium), Alkylmagnesiumhalogenide (z.B. Methylmagnesiumchlorid) sowie Alkalimetall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte pyridine wie Collidin, Lutidin und 4-Dimethylaminopyridin sowie bicyclische Amine in Betracht. Besonders bevorzugt werden Natriumhydroxid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Terabutylammoniumtetrafluoroborat) sowie Phosphoniumhalogenide (z.B. Tetrabutylphosphoniumchlorid und Tetraphenylphosphoniumbromid) in Betracht.

Es kann für die Umsetzung vorteilhaft sein, zunächst das 4-Hydroxybiphenyl III mit der Base in das entsprechende Hydroxylat umzusetzen, welches dann mit dem Benzylderivat umgesetzt wird.

Die für die Herstellung der Verbindungen I benötigten Ausgangsstoffe II sind aus EP-A 513 580 und WO-A 93/15,046 bekannt oder können nach den dort beschriebenen Methoden hergestellt werden.

4-Biphenole der Formel III sind ebenfalls bekannt oder können nach bekannten Verfahren erhalten werden [vgl. Synthesis 1993, 735; Synthesis 1992, 803; Synthesis 1992, 413; Pure and Appl. Chem. 66, 213 (1994); J. Org. Chem. 58, 2201 (1993); Tetrahedron 44, 9577 (1992); Chem. Rev. 93, 2117 (1993); Acc. Chem. Res. 15, 340 (1982); Pure and Appl. Chem. 52, 669 (1980)]

Die Reduktion der Nitroverbindungen IV zu den entsprechenden N-Hydroxyanilinen Va erfolgt analog zu literaturbekannten Methoden beispielsweise mit Metallen wie Zink [vgl. Ann. Chem. 316, 278 (1901)] oder mit Wasserstoff (vgl. EP-A 085 890).

Die Umsetzung der N-Hydroxyaniline Va mit den Carbonylverbindungen VI erfolgt unter alkalischen Bedingungen gemäß den vorstehend für die Umsetzung der Verbindungen II mit den 4-Biphenolen III beschriebenen Bedingungen. Insbesondere bevorzugt wird die Umsetzung bei Temperaturen von -10°C bis 30°C durchgeführt. Die bevorzugten Lösungsmittel sind Methylenchlorid, Toluol, tert.-Butylmethylether oder Essigsäureethylester. Die bevorzugten Basen sind Natriumhydrogencarbonat, Kaliumcarbonat oder wäßrige Natriumhydroxid Lösung.

Außerdem erhält man die Verbindungen der Formel I, in denen X für eine direkte Bindung oder Sauerstoff steht, beispielsweise dadurch, daß man ein Benzylderivat der Formel IIa zunächst zum entsprechenden N-Hydroxyanilin der Formel Vb reduziert, Vb mit einer Carbonylverbindung der Formel VI in das entsprechende Anilid der Formel VII überführt, VII anschließend mit einer Verbindung VIII in das Amid der Formel IX überführt, XI anschließend in das Benzylhalogenid X überführt und X in Gegenwart einer Base mit einem 4-Biphenol der Formel III in I umwandelt.

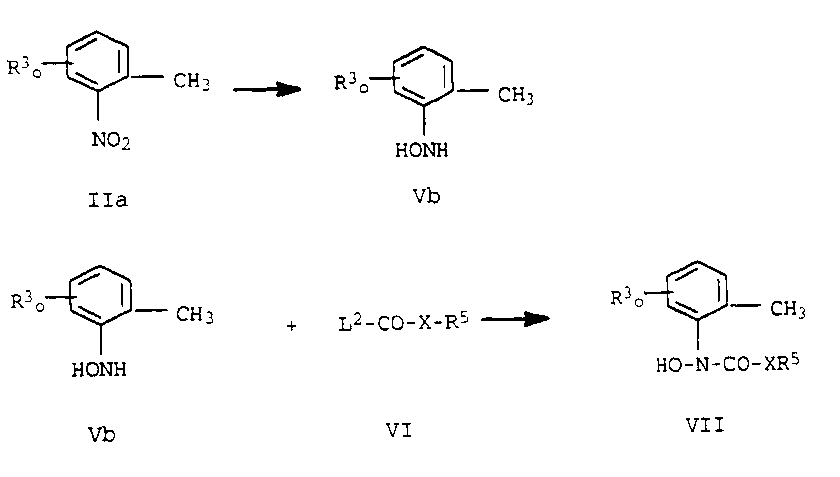

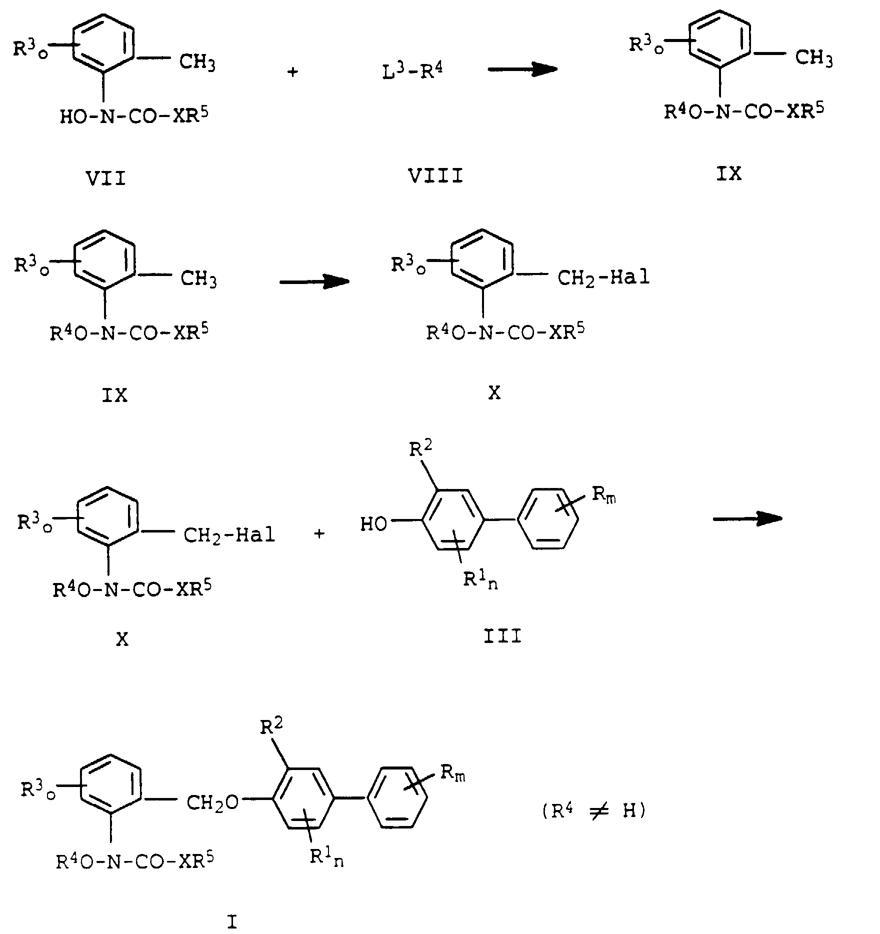

In der Formel X bedeutet Hal ein Halogenatom, insbesondere Chlor oder Brom.

L³ in der Formel VIII bedeutet eine nucleophil austauschbare Gruppe, beispielsweise Halogen (z.B: Chlor, Brom und Iod), oder ein Alkyl- oder Arylsulfonat (z.B. Methylsulfonat, Trifluormethylsulfonat, Phenylsulfonat und 4-Methylsulfonat) und R⁴ steht nicht für Wasserstoff.

Die Umsetzungen erfolgen analog den vorstehend ausgeführten Verfahren.

Die Halogenierung der Verbindungen IX erfolgt radikalisch, wobei als Halogenierungsmittel beispielsweise N-chlor- oder N-Bromsuccinimid, elementare Halogene (z.B. Chlor oder Brom) oder Thionylchlorid, Phosphortri- oder Phosphorpentachlorid und ähnliche Verbindungen eingesetzt werden können. Üblicherweise verwendet man zusätzlich einen Radikalstarter (z.B. Azobisisobutyronitril) oder man führt die Umsetzung unter Bestrahlung (mit UV-Licht) durch. Die Halogenierung erfolgt in an sich bekannter Weise in einem üblichen organischen Verdünnungsmittel.

Die Verbindungen I, in denen R⁴ nicht Wasserstoff bedeutet, erhält man außerdem dadurch, daß man eine entsprechende Verbindung der Formel I, in der R⁴ Wasserstof bedeutet, mit einer Verbindung der Formel VIII umsetzt.

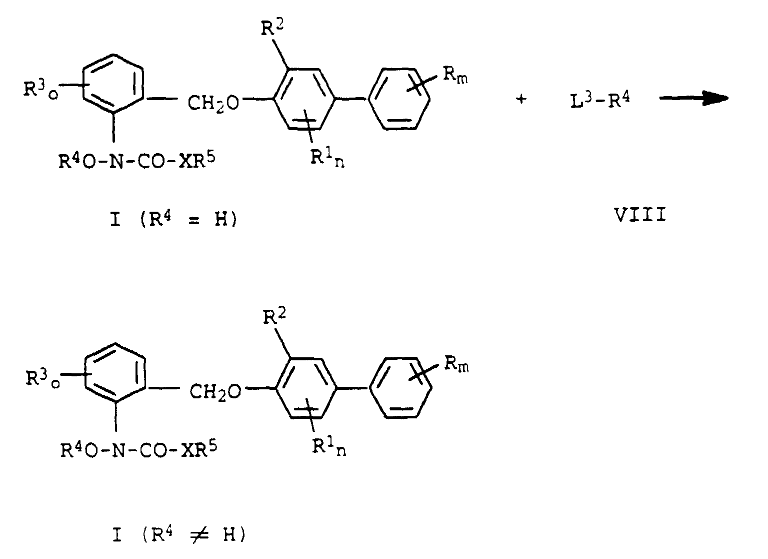

Die Umsetzung erfolgt in an sich bekannter Weise in einem inerten organischen Lösungsmittel in Gegenwart einer Base bei Temperaturen von 0°C bis 50°C.

Als Basen dienen insbesondere Natriumhydrogencarbonat, Kaliumcarbonat, Natriumhydroxid und wäßrige Natriumhydroxid Lösungen.

Als Lösungsmittel finden insbesondere Aceton, Dimethylformamid, Toluol, tert.-Butylmethylether, Essigsäureethylester und Methanol Verwendung.

Die Verbindungen der Formel I, in denen X für NR^{c} steht, erhält man vorteilhaft dadurch, daß man ein Benzylanilid der Formel IXa in das entsprechende Benzylhalogenid der Formel Xa überführt, Xa in Gegenwart einer Base mit einem 4-Biphenol der Formel III in eine Verbindung der Formel I.A überführt und I.A anschließend mit einem Amin der Formel XIa bzw. XIb zu I umsetzt.

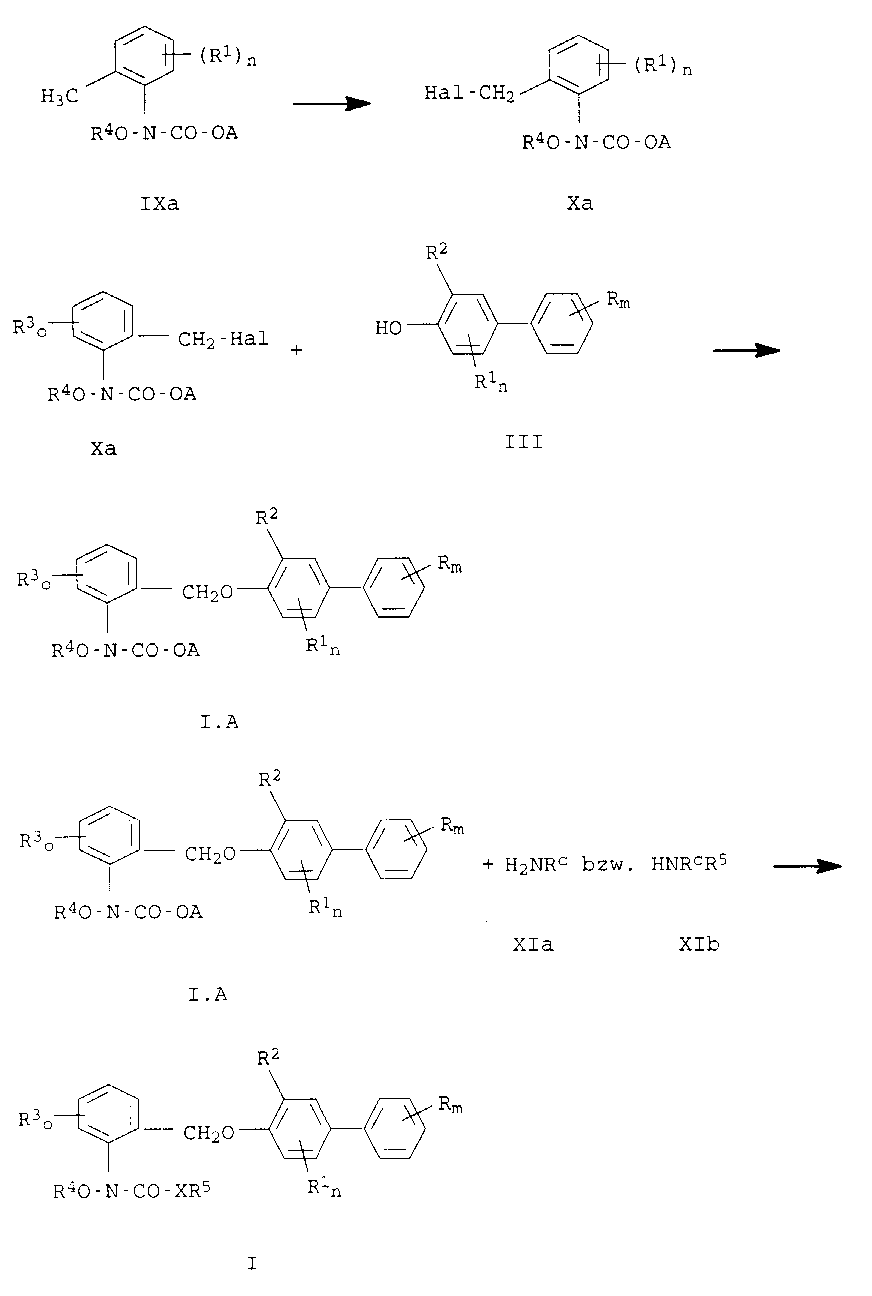

A in der Formel IXa steht für Alkyl (insbesondere C₁-C₆-Alkyl) oder Phenyl; Hal in der Formel Xa steht für Halogen (insbesondere Chlor und Brom).

Die Umsetzungen von IXa nach Xa und von Xa nach I.A erfolgen im allgemeinen und im besonderen unter den vorstehend beschriebenen Bedingungen.

Die Umsetzung der Verbindungen I.A mit den primären oder sekundären Aminen der Formel XIa bzw. XIb erfolgt bei Temperaturen von 0°C bis 100°C in einem inerten Lösungsmittel oder in einem Lösungsmittelgemisch.

Als Lösungsmittel eignen sich insbesondere Wasser, tert.-Butylmethylether und Toluol oder deren Gemische. Es kann vorteilhaft sein, zur Verbesserung der Löslichkeit der Edukte zusätzlich eines der folgenden Lösungsmittel (als Lösungsvermittler) zuzusetzen: Tetrahydrofuran, Methanol, Dimethylformamid und Ethylenglycolether.

Die Amine XIa bzw. XIb werden üblicherweise in einem Überschuß bis zu 100% bezogen auf die Verbindungen X eingesetzt oder können als Lösungsmittel verwendet werden. Es kann im Hinblick auf die Ausbeute vorteilhaft sein, die Umsetzung unter Druck durchzuführen.

Amide der Formel I.A' (A=Phenyl) erhält man durch Umsetzung der Hydroxyaniline V.A mit einem Acylierungsmittel VI.A (A=Phenyl).

Die Verbindung I.A' kann mit einem Amin der Formel XIa bzw. XIb zur Verbindung I (R⁴=H, X=NR^{c}) umgesetzt werden. Durch Reaktion der Verbindung I (R⁴=H, X=NR^{c}) mit einem Elektrophil VIII erhält man die Verbindungen I (R⁴≠ H, X=NR^{c}).

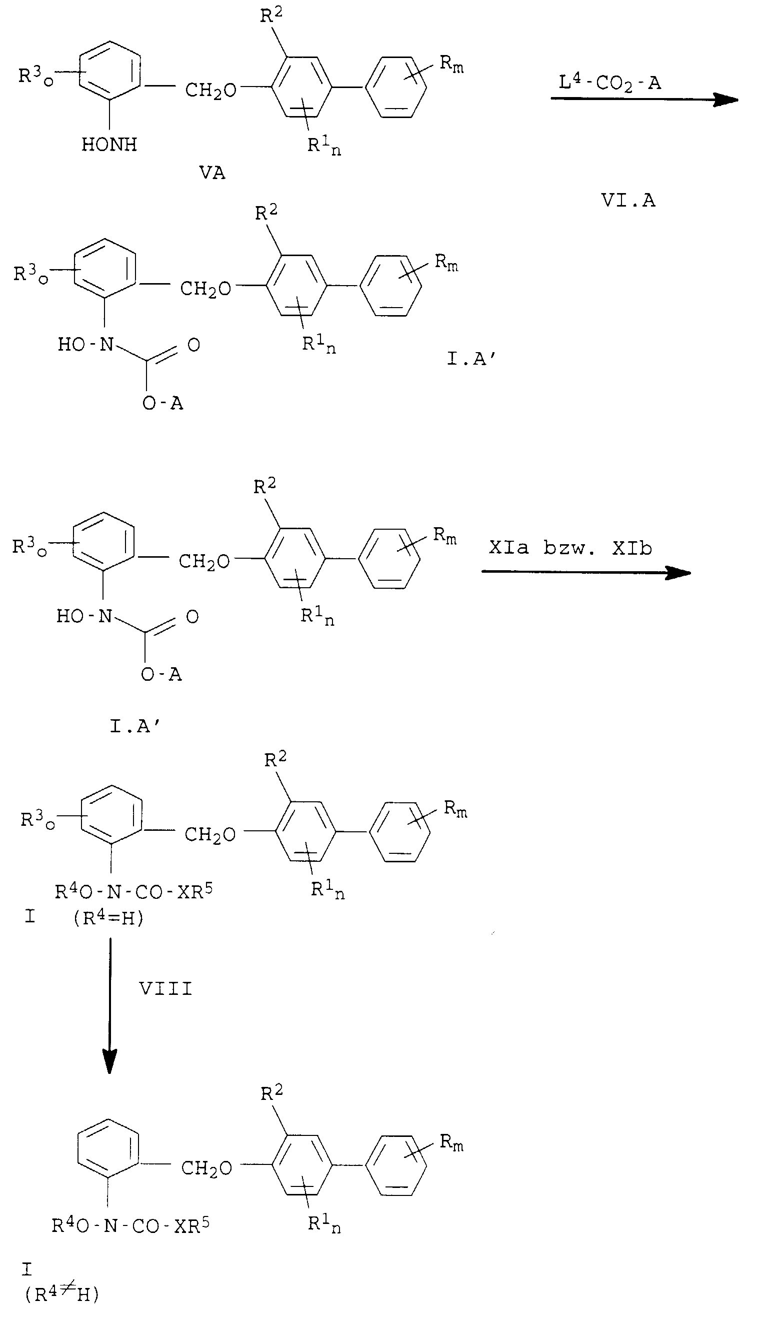

Daneben erhält man die Verbindungen I beispielsweise auch dadurch, daß man eine Verbindung XIV in Gegenwart eines Palladium-Katalysators mit einer metallorganischen Verbindung XV umsetzt.

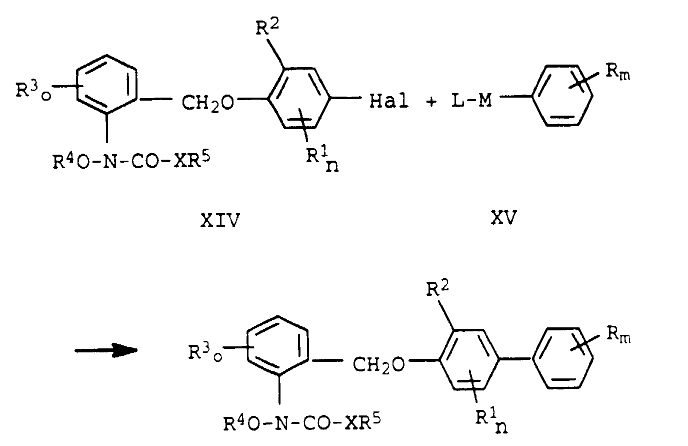

Hal in der Formel XIV bedeutet ein Halogenatom (beispielsweise Chlor, Brom und Iod).

L-M in der Formel XV steht für einen metallorganischen Rest wie Magnesiumhalogenide, oder B(OH)₂ oder tri-Alkyl-Sn.

Die Reaktionsbedingungen der Biphenylsynthese entsprechen den der literaturmäßig bekannten Biphenylsynthesen [vgl. Synthesis 1993, 735; Synthesis 1992, 803; Synthesis 1992, 413; Pure and Appl. Chem. 66, 213 (1994); J. Org. Chem. 58, 2201 (1993); Tetrahedron 44, 9577 (1992); Chem. Rev. 93 2117 (1993); Acc. Chem. Res. 15, 340 (1982); Pure and Appl. Chem. 52, 669 (1980)].

Die Herstellung der Verbindungen I erfolgt analog zu den in WO-A 93/15,046 beschriebenen Methoden über zwischenprodukte der Formel XII 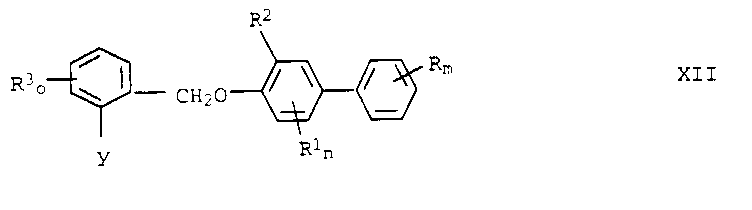 in der Y für NO₂, NHOH, NHOR⁴, N(OH) -CO₂C₆H₅ oder N(OR⁴)-CO₂C₆H₅ steht und die Indices m, n und o und die Substituenten R, R¹, R², R³ und R⁴ die in Anspruch 1 gegebene Bedeutung haben.

Außerdem kann die Herstellung der Verbindungen I über Zwischenprodukte der Formel XIIA 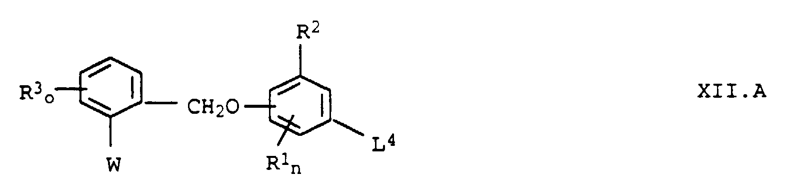 nach den Methoden der Biarylsynthese erfolgen (Literaturstelle siehe vorne),
wobei W NO₂, NHOH, NHOR⁴, N(OR⁴)COXR⁵ oder N(OR⁴)CO₂ Phenyl bedeutet,
L⁴ Halogen (Cl, Br, J) oder O-SO₂-CF₃ oder einer metallorganischen Gruppe entsprechend den Methoden der vorne genannten Biarylsynthesen entspricht und
R¹ₙ, R², R³ₒ, R⁴, R⁵ und X die in Anspruch 1 genannte Bedeutung besitzen.

Die Verbindungen I können saure oder basische zentren enthalten und dementsprechend Säureadditionsprodukte oder Basenadditionsprodukte oder Salze bilden.

Säuren für Säureadditionsprodukte sind u.a. Mineralsäuren (z.B. Halogenwasserstoffsäuren wie Chlorwasserstoff- und Bromwasserstoffsäure, Phosphorsäure, Schweferlsäure, Salpetersäure), organsiche Säuren (z.B. Ameisensäure, Essigsäure, Oxalsäure, Malonsäure, Milchsäure, Äpfelsäure, Bernseinsäure, Weinsäure, Zitronensäure, Salizylsäure, p-Toluolsulfonsäure, Dodecylbenzolsulfonsäure) oder andere protonenacide Verbindugnen (z.B. Saccharin). Basen für Basenadditionsprodukte sind u.a. Oxide, Hydroxide, Carbonate oder Hydrogencarbonate von Alkalimetallen oder Erdalkaimetallen (z.B. Kalium- oder Natriumhydroxyd oder -carbonat) oder Ammoniumverbindungen (z.B. Ammoniumhydroxyd).

Bei den in den vorstehenden Formeln angegebenen Definitionen der Symbole wurden z.T. Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4 oder 10 Kohlenstoffatomen, z.B. Methyl, Ethyl, Propyl, 1-Methylethyl, Butyl, 1-Methyl-propyl, 2-Methylpropyl und 1,1-Dimethylethyl;
Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-chlor-2-fluorethyl, 2-Chlor-2,2-difluorethyl, 2,2-Dichlor-2-fluorethyl, 2,2,2-Trichlorethyl und Pentafluorethyl;
Alkylcarbonyl: geradkettige oder verzweigte Alkylgruppen, insbesondere mit 1 bis 10 Kohlenstoffatomen (wie vorstehend genannt), welche über eine Carbonylgruppe (-CO-) an das Gerüst gebunden sind;
Alkoxy: geradkettige oder verzweigte Alkylgruppen mit 1 bis 4 oder 10 Kohlenstoffatomen (wie vorstehend genannt), welche über ein Sauerstoffatom (-O-) an das Gerüst gebunden sind;
Alkoxycarbonyl: geradkettige oder verzweigte Alkoxygruppen mit 1 bis 4 Kohlenstoffatomen (wie vorstehend genannt), welche über eine Carbonylgruppe (-CO-) an das Gerüst gebunden sind;
Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind;
ggf. subst. Alkyl: gesättigte, geradkettige oder verzweigte Kohlenwasserstoffreste, insbesondere mit 1 bis 10 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
ggf. subst. Alkenyl: ungesättigte, geradkettige oder verzweigte Kohlenwasserstoffreste, insbesondere mit 2 bis 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl,
1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
ggf. subst. Alkenyloxy: geradkettige oder verzweigte Alkenylgruppen mit 3 bis 10 Kohlenstoffatomen (wie vorstehend genannt), welche über ein Sauerstoffatom (-O-) an das Gerüst gebunden sind;
Alkinyl: geradkettige oder verzweigte Kohlenwasserstoffgruppen, insbesondere mit 2 bis 20 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl- 1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
ggf. subst. Alkinyloxy: geradkettige oder verzweigte Alkinylgruppen mit 3 bis 10 Kohlenstoffatomen (wie vorstehend genannt), welche über ein Sauerstoffatom (-O-) an das Gerüst gebunden sind;
ggf. subst. Cycloalkyl: mono- oder bicyclische Kohlenwaserstoffreste mit 3 bis 10 Kohlenstoffatomen, z.B. C₃-C₁₀-(Bi)cycloalkyl wie Cyclopropyl, Cyclobutyl, Cyclopentyl, Cyclohexyl, Cycloheptyl, Bornanyl, Norbornanyl, Dicyclohexyl, Bicyclo[3,3,0]octyl, Bicyclo[3,2,1]octyl, Bicyclo[2,2,2]octyl oder Bicyclo[3,3,1]nonyl;
ggf. subst. Cycloalkenyl: mono- oder bicyclische Kohlenwaserstoffreste mit 5 bis 10 Kohlenstoffatomen und einer Doppelbindung in einer beliebigen Ringposition, z.B. C₅-C₁₀-(Bi)cycloalkenyl wie Cyclopentenyl, Cyclohexenyl, Cycloheptenyl, Bornenyl, Norbornenyl, Dicyclohexenyl und Bicyclo[3,3,0]octenyl;
eine an zwei benachbarte Ringatome gebundene ggf. subst. Brücke, welche drei bis vier Glieder aus der Gruppe 3 oder 4 Kohlenstoffatome oder 1 bis 3 Kohlenstoffatome und 1 oder 2 Stickstoff-, Sauerstoff- und/oder Schwefelatome besitzt, wobei diese Brücke gemeinsam mit dem Ring an den sie gebunden ist einen partiell ungesättigten oder aromatischen Rest bilden kann: Brücken, die mit dem Ring, an den sie gebunden sind beispielsweise eines der folgenden Systeme bilden: Chinolinyl, Benzofuranyl und Naphthyl;
ein ggf. subst. gesättigter oder ein- oder zweifach ungesättigter Ring, welcher neben Kohlenstoffatomen ein bis drei der folgenden Heteroatome als Ringglieder enthalten kann: Sauerstoff, Schwefel und Stickstoff, beispielsweise Carbocyclen wie Cyclopropyl, Cyclopentyl, Cyclohexyl, Cyclopent-2-enyl, Cyclohex-2-enyl, 5- bis 6-gliedrige, gesättigte oder ungesättigte Heterocyclen, enthaltend ein bis drei Stickstoffatome und/oder ein Sauerstoff- oder Schwefelatom wie 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl,1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, vorzugsweise 2-Tetrahydrofuranyl, 2-Tetrahydrothienyl, 2-Pyrrolidinyl, 3-Isoxazolidinyl, 3-Isothiazolidinyl, 1,3,4-Oxazolidin-2-yl, 2,3-Dihydrothien-2-yl, 4,5-Isoxazolin-3-yl, 3-Piperidinyl, 1,3-Dioxan-5-yl, 4-Piperidinyl, 2-Tetrahydropyranyl, 4-Tetrahydropyranyl und Morpholinyl;
oder ein ggf. subst. ein- oder zweikerniges aromatisches Ringsystem, welches neben Kohlenstoffatomen ein bis vier Stickstoffatome, oder ein oder zwei Stickstoffatome und ein Sauerstoffoder Schwefelatom, oder ein Sauerstoff- oder Schwefelatom als Ringglieder enthalten kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-Pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
Sechsring Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz "ggf. subst" in Bezug auf Alkyl-, Alkenyl- und Alkinylgruppen soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein können und/oder einen bis drei, insbesondere einen, der folgenden Reste tragen können:
C₁-C₆-Alkoxy, C₁-C₆-Halogenalkoxy, C₁-C₆-Alkylthio, C₁-C₆Halogenalkylthio, C₁-C₆-Alkylamino, Di-C₁-C₆-alkylamino, C2-C₆-Alkenyloxy, C₂-C₆-Halogenalkenyloxy, C₂-C₆-Alkinyloxy, C₂-C₆-Halogenalkinyloxy, C₃-C₆-Cycloalkyl, C₃-C₆-Cycloalkyloxy, C₃-C₆-Cycloalkenyl, C3-C6-Cycloalkenyloxy,
oder ein ggf. subst. ein- oder zweikerniges aromatisches Ringsystem, welches neben Kohlenstoffatomen ein bis vier Stickstoffatome oder ein oder zwei Stickstoffatome und ein Sauerstoff- oder Schwefelatom oder ein Sauerstoff- oder Schwefelatom als Ringglieder enthalten kann (wie vorstehend genannt), welches direkt oder über ein Sauerstoffatom (-O-), ein Schwefelatom (-S-) oder eine Aminogruppe (-NR^{a}-) an den Substituenten gebunden sein kann, d.h. Arylreste wie Phenyl und Naphthyl, vorzugsweise Phenyl oder 1- oder 2-Naphthyl, und Hetarylreste, beispielsweise 5-Ring Heteroaromaten enthaltend ein bis drei Stickstoffatome und/oder ein Sauerstoff- oder Schwefelatom wie 2-Furyl, 3-Furyl, 2-Thienyl, 3-Thienyl, 1-pyrrolyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 1-Pyrazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 1-Imidazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,5-Triazol-3-yl, 1,2,3-Triazol-4-yl, 1,2,3-Triazol-5-yl, 1,2,3-Triazol-4-yl, 5-Tetrazolyl, 1,2,3,4-Thiatriazol-5-yl und 1,2,3,4-Oxatriazol-5-yl, insbesondere 3-Isoxazolyl, 5-Isoxazolyl, 4-Oxazolyl, 4-Thiazolyl, 1,3,4-Oxadiazol-2-yl und 1,3,4-Thiadiazol-2-yl;
Sechsring Heteroaromaten enthaltend ein bis vier Stickstoffatome als Heteroatome wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl, insbesondere 2-Pyridinyl, 3-Pyridinyl, 4-Pyridinyl, 2-Pyrimidinyl, 4-Pyrimidinyl, 2-Pyrazinyl und 4-Pyridazinyl.

Der Zusatz "ggf. subst" in Bezug auf die cyclischen (gesättigten, ungesättigtern oder aromatischen) Gruppen soll zum Ausdruck bringen, daß diese Gruppen partiell oder vollständig halogeniert sein können (d.h. die Wasserstoffatome dieser Gruppen können teilweise oder vollständig durch gleiche oder verschiedene Halogenatome wie vorstehend genannt (vorzugsweise Fluor, Chlor und Brom, insbesondere Fluor und Chlor) ersetzt sein können und/oder einen bis drei, der folgenden Reste tragen können:
Nitro;
Cyano, Thiocyanato;
Alkyl, besonders C₁-C₆-Alkyl wie vorstehend genannt, vorzugsweise Methyl, Ethyl, 1-Methylethyl, 1,1-Dimethylethyl, Butyl, Hexyl, insbesondere Methyl und 1-Methylethyl;
C₁-C₄-Halogenalkyl, wie vorstehend genannt, vorzugsweise Trichlormethyl, Difluormethyl, Trifluormethyl, 2,2-Difluorethyl, 2,2,2-Trifluorethyl und Pentafluorethyl;
C₁-C₄-Alkoxy, vorzugsweise Methoxy, Ethoxy, 1-Methylethoxy und 1,1-Dimethylethoxy, insbesondere Methoxy ;
C₁-C₄-Halogenalkoxy, besonders C₁-C₂-Halogenalkoxy, vorzugsweise Difluormethyloxy, Trifluormethyloxy und 2,2,2-Trifluorethyloxy, insbesondere Difluormethyloxy;
C₁-C₄-Alkylthio, vorzugsweise Methylthio und 1-Methylethylthio, insbesondere Methylthio;
C₁-C₄-Alkylamino wie Methylamino, Ethylamino, Propylamino, 1-Methylethylamino, Butylamino, 1-Methylpropylamino, 2-Methylpropylamino und 1,1-Dimethylethylamino, vorzugsweise Methylamino und 1,1-Dimethylethylamino, insbesondere Methylamino,
Di-C₁-C₄-alkylamino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl) amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl) amino, N-Butyl-N-(2-methylpropyl) amino, N-Butyl-N-(1,1-dimethylethyl)amino, N-(1-Methylpropyl)N-(2-methylpropyl)amino, N-(1,1-Dimethylethyl)-N-(1-methylpropyl)amino und N-(1,1-Dimethylethyl)-N-(2-methylpropyl)amino, vorzugsweise N,N-Dimethylamino und N,N-Diethylamino, insbesondere N,N-Dimethylamino;
C₁-C₆-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Methylcarbonyl, Ethylcarbonyl und 1,1-Dimethylcarbonyl, insbesondere Ethylcarbonyl;
C₁-C₆-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethylpropoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise Methoxycarbonyl, Ethoxycarbonyl und 1,1-Dimethylethoxycarbonyl, insbesondere Ethoxycarbonyl;
C₁-C₆-Alkylaminocarbonyl wie Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl, 1,1-Dimethylethylaminocarbonyl, Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl und 1-Ethyl-2-methylpropylaminocarbonyl, vorzugsweise Methylamincarbonyl und Ethylamincarbonyl, insbesondere Methylaminocarbonyl;
Di-C₁-C₆-alkylaminocarbonyl, besonders Di-C₁-C₄-alkylaminocarbonyl wie N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl) aminocarbonyl, N,N-Di-(2-methylpropyl)aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Di-methylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methyl-propyl)aminocarbonyl, N-(1,1-Dimethylethyl)-N-(1-methylpropyl)aminocarbonyl und N-(1,1-Dimethylethyl)-N-(2-methylpropyl)aminocarbonyl, vorzugsweise N,N-Dimethylaminocarbonyl und N,N-Diethylamincarbonyl, insbesondere N,N-Dimethylaminocarbonyl;
C₁-C₆-Alkylcarboxyl wie Methylcarboxyl, Ethylcarboxyl, Propylcarboxyl, 1-Methylethyl-carboxyl, Butylcarboxyl, 1-Methylpropylcarboxyl, 2-Methylpropylcarboxyl, 1,1-Dimethylethylcarboxyl, Pentylcarboxyl, 1-Methylbutylcarboxyl, 2-Methylbutylcarboxyl, 3-Methylbutylcarboxyl, 1,1-Dimethylpropylcarboxyl, 1,2-Dimethylpropylcarboxyl, 2,2-Dimethylpropylcarboxyl, 1-Ethylpropylcarboxyl, Hexylcarboxyl, 1-Methylpentylcarboxyl, 2-Methylpentylcarboxyl, 3-Methylpentylcarboxyl, 4-Methylpentylcarboxyl, 1,1-Dimethylbutylcarboxyl, 1,2-Dimethylbutylcarboxyl, 1,3-Dimethylbutylcarboxyl, 2,2-Dimethylbutylcarboxyl, 2,3-Dimethylbutylcarboxyl, 3,3-Dimethylbutylcarboxyl, 1-Ethylbutylcarboxyl, 2-Ethylbutylcarboxyl, 1,1,2-Trimethylpropylcarboxyl, 1,2,2-Trimethylpropylcarboxyl, 1-Ethyl-1-methylpropylcarboxyl und 1-Ethyl-2-methylpropylcarboxyl, vorzugsweise Methylcarboxyl, Ethylcarboxyl und 1,1-Dimethylethylcarbonyl, insbesondere Methylcarboxyl und 1,1-Dimethylethylcarboxyl;
C₁-C₆-Alkylcarbonylamino wie Methylcarbonylamino, Ethylcarbonylamino, Propylcarbonylamino, 1-Methylethylcarbonylamino, Butylcarbonylamino, 1-Methylpropylcarbonylamino, 2-Methylpropylcarbonylamino, 1,1-Dimethylethylcarbonylamino, Pentylcarbonylamino, 1-Methylbutylcarbonylamino, 2-Methylbutylcarbonylamino, 3-Methylbutylcarbonylamino, 2,2-Dimethylpropylcarbonylamino, 1-Ethylpropylcarbonylamino, Hexylcarbonylamino, 1,1-Dimethylpropylcarbonylamino, 1,2-Dimethylpropylcarbonylamino, 1-Methylpentylcarbonylamino, 2-Methylpentylcarbonylamino,3-Methylpentylcarbonylamino, 4-Methylpentylcarbonylamino, 1,1-Dimethylbutylcarbonylamino, 1,2-Dimethylbutylcarbonylamino, 1,3-Dimethylbutylcarbonylamino, 2,2-Dimethylbutylcarbonylamino, 2,3-Dimethylbutylcarbonylamino, 3,3-Dimethylbutylcarbonylamino, 1-Ethylbutylcarbonylamino, 2-Ethylbutylcarbonylamino, 1,1,2-Trimethylpropylcarbonylamino, 1,2,2-Trimethylpropylcarbonylamino, 1-Ethyl-1-methylpropylcarbonylamino und 1-Ethyl-2-methylpropylcarbonylamino, vorzugsweise Methylcarbonylamino und Ethylcarbonylamino, insbesondere Ethylcarbonylamino;
C₃-C₇-Cycloalkyl wie Cyclopropyl, Cyclobutyl, Cyclopentyl, Cyclohexyl und Cycloheptyl, vorzugsweise Cyclopropyl, Cyclopentyl und Cyclohexyl, insbesondere Cyclopropyl;
C₃-C₇-Cycloalkoxy wie Cyclopropyloxy, Cyclobutyloxy, Cyclopentyloxy, Cyclohexyloxy und Cycloheptyloxy, vorzugsweise Cyclopentyloxy und Cyclohexyloxy, insbesondere Cyclohexyloxy;
C₃-C₇-Cycloalkylthio wie Cyclopropylthio, Cyclobutylthio, Cyclopentylthio, Cyclohexylthio und Cycloheptylthio, vorzugsweise Cyclohexylthio;
C₃-C₇-Cycloalkylamino wie Cyclopropylamino, Cyclobutylamino, Cyclopentylamino, Cyclohexylamino und Cycloheptylamino, vorzugsweise Cyclopropylamino und Cyclohexylamino, insbesondere Cyclopropylamino;

Die ein- oder zweikernigen aromatischen oder heteroaromatischen Systeme können neben den vorstehend genannten Substituenten auch einen Rest -CR'=NOR" tragen, wobei die Reste R' und R" für die folgenden Gruppen stehen:
- R': Wasserstoff, Cyano, Alkyl (vorzugsweise C₁-C₆-Alkyl, insbesondere C₁-C₄-Alkyl), Haloalkyl (vorzugsweise C₁-C₄-Haloalkyl, insbesondere C₁-C₂-Haloalkyl), Alkenyl (vorzugsweise C₂-C₆-Alkenyl, insbesondere C₂-C₄-Alkenyl), Haloalkenyl (vorzugsweise C₂-C₆-Haloalkenyl, insbesondere C₂-C₄-Haloalkenyl), Alkinyl (vorzugsweise C₂-C₆-Alkinyl, insbesondere C₂-C₄-Alkinyl), Haloalkinyl (vorzugsweise C₂-C₆-Haloalkinyl, insbesondere C₂-C₄-Haloalkinyl) und Cycloalkyl (vorzugsweise C₃-C₈-Cycloalkyl, insbesondere C₃-C₆-Cycloalkyl);
- R": Alkyl (vorzugsweise C₁-C₆-Alkyl, insbesondere C₁-C₄-Alkyl), Haloalkyl (vorzugsweise C₁-C₄-Haloalkyl, insbesondere C₁-C₂-Haloalkyl), Alkenyl (vorzugsweise C₂-C₆-Alkenyl, insbesondere C₂-C₄-Alkenyl), Haloalkenyl (vorzugsweise C₂-C₆-Haloalkenyl, insbesondere C₂-C₄-Haloalkenyl), Alkinyl (vorzugsweise C₂-C₆-Alkinyl, insbesondere C₂-C₄-Alkinyl), Haloalkinyl (vorzugsweise C₂-C₆-Haloalkinyl, insbesondere C₂-C₄-Haloalkinyl) und Cycloalkyl (vorzugsweise C₃-C₈-Cycloalkyl, insbesondere C₃-C₆-Cycloalkyl).

Im Hinblick auf ihre biologische Wirkung sind Verbindungen I bevorzugt, in denen R für C₁-C₄-Alkyl steht.

Desweiteren sind Verbindungen I bevorzugt, in denen R für Halogen, insbesondere Fluor, Chlor und Brom steht.

Gleichermaßen sind Verbindungen I bevorzugt, in denen R für C₁-C₄-Alkoxy steht.

Außerdem werden Verbindungen I bevorzugt, in denen R für Cyano oder Nitro steht.

Gleichermaßen sind Verbindungen I bevorzugt, in denen R für C₁-C₄-Halogenalkyl (insbesondere Trifluormethyl) oder C₁-C₄-Halogenalkoxy (insbesondere Trifluormethoxy) steht.

Außerdem sind Verbindungen I bevorzugt, in denen R für eine Gruppe C(R^{a})=NOR^{b} steht, wobei R^{a} und R^{b} insbesondere C₁-C₄-Alkylgruppen repräsentieren.

Daneben sind Verbindungen I bevorzugt, in denen m für 1, 2 oder 3, insbesondere 1 oder 2 steht.

Daneben sind Verbindungen I bevorzugt, in denen R¹ für C₁-C₄-Alkyl oder C₁-C₄-Halogenalkyl steht.

Desweiteren sind Verbindungen I bevorzugt, in denen R¹ für Halogen (insbesondere Fluor, Chlor oder Brom) steht.

Gleichermaßen sind Verbindungen I bevorzugt, in denen R¹ für Cyano oder Nitro steht.

Außerdem werden Verbindungen I bevorzugt, in denen R¹ für C₁-C₄-Alkoxy oder C₁-C₄-Halogenalkoxy steht.

Daneben sind Verbindungen I bevorzugt, in denen n für 0, 1 oder 2 (insbesondere 0 oder 1) steht.

Daneben sind Verbindungen I bevorzugt, in denen R² für C₁-C₄-Alkyl oder C₁-C₄-Halogenalkyl steht.

Desweiteren sind Verbindungen I bevorzugt, in denen R² für Halogen (insbesondere Fluor, Chlor und Brom) steht.

Gleichermaßen sind Verbindungen I bevorzugt, in denen R² für Cyano oder Nitro steht.

Außerdem werden Verbindungen I bevorzugt, in denen R² für C₁-C₄-Alkoxy oder C₁-C₄-Halogenalkoxy steht.

Daneben sind Verbindungen I bevorzugt, in denen R³ für Cyano, Halogen, C₁-C₄-Alkyl, C₁-C₂-Halogenalkyl, C₁-C₄-Alkoxy oder C₁-C₂-Halogenalkoxy steht.

Gleichermaßen werden Verbindungen I bevorzugt, in denen o für 0 oder 1, insbesondere für 0, steht.

Für den Fall, daß o nicht 0 bedeutet sind Verbindungen I bevorzugt, in denen ein Rest R³ in 3- oder 6-Position steht.

Daneben werden Verbindungen I bevorzugt, in denen R⁴ für Wasserstoff, C₁-C₄-Alkyl oder C₁-C₂-Halogenalkyl steht.

Außerdem werden Verbindungen I bevorzugt, in denen R⁵X für Methyl, Ethyl, Methoxy oder Methylamino steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet (unabhängig von der Kombination, in der sie genannt sind) eine besonders bevorzugte Ausgestaltung des betreffenden Substituenten dar.

### Tabelle 1

Verbindungen der allgemeinen Formel I (o = 0), in denen R⁴ für Methyl steht, R⁵X für Wasserstoff steht und die Kombination der Substituenten Rₘ, R¹ₙ und R² für eine Verbindung jeweils einer Zeile der Tabelle A entspricht

### Tabelle 2

Verbindungen der allgemeinen Formel I (o = 0), in denen R⁴ für Methyl steht, R⁵X für Methyl steht und die Kombination der Substituenten Rₘ, R¹ₙ und R² für eine Verbindung jeweils einer Zeile der Tabelle A entspricht

### Tabelle 3

Verbindungen der allgemeinen Formel I (o = 0), in denen R⁴ für Methyl steht, R⁵X für Ethyl steht und die Kombination der Substituenten Rₘ, R¹ₙ und R² für eine Verbindung jeweils einer Zeile der Tabelle A entspricht

### Tabelle 4

Verbindungen der allgemeinen Formel I (o = 0), in denen R⁴ für Methyl steht, R⁵X für Methyloxy steht und die Kombination der Substituenten Rₘ, R¹ₙ und R² für eine Verbindung jeweils einer Zeile der Tabelle A entspricht

### Tabelle 5

Verbindungen der allgemeinen Formel I (o = 0), in denen R⁴ für Methyl steht, R⁵X für Methylamino steht und die Kombination der Substituenten Rₘ, R¹ₙ und R² für eine Verbindung jeweils einer Zeile der Tabelle A entspricht

### Tabelle 6

Verbindungen der allgemeinen Formel I (o = 0), in denen R⁴ für Ethyl steht, R⁵X für Wasserstoff steht und die Kombination der Substituenten Rₘ, R¹ₙ und R² für eine Verbindung jeweils einer Zeile der Tabelle A entspricht

### Tabelle 7

Verbindungen der allgemeinen Formel I (o = 0), in denen R⁴ für Ethyl steht, R⁵X für Methyl steht und die Kombination der Substituenten Rₘ, R¹ₙ und R² für eine Verbindung jeweils einer Zeile der Tabelle A entspricht

### Tabelle 8

Verbindungen der allgemeinen Formel I (o = 0), in denen R⁴ für Ethyl steht, R⁵X für Ethyl steht und die Kombination der Substituenten Rₘ, R¹ₙ und R² für eine Verbindung jeweils einer Zeile der Tabelle A entspricht

### Tabelle 9

Verbindungen der allgemeinen Formel I (o = 0), in denen R⁴ für Ethyl steht, R⁵X für Methyloxy steht und die Kombination der Substituenten Rₘ, R¹ₙ und R² für eine Verbindung jeweils einer Zeile der Tabelle A entspricht

### Tabelle 10

Verbindungen der allgemeinen Formel I (o = 0), in denen R⁴ für Ethyl steht, R⁵X für Methylamino steht und die Kombination der Substituenten Rₘ, R¹ₙ und R² für eine Verbindung jeweils einer Zeile der Tabelle A entspricht

### Tabelle 11

Verbindungen der allgemeinen Formel I (o = 1), in denen R³ 3-Fluor bedeutet, R⁴ für Methyl steht, R⁵X für Wasserstoff steht und die Kombination der Substituenten Rₘ, R¹ₙ und R² für eine Verbindung jeweils einer Zeile der Tabelle A entspricht

### Tabelle 12

Verbindungen der allgemeinen Formel I (o = 1), in denen R³ 3-Fluor bedeutet, R⁴ für Methyl steht, R⁵X für Methyl steht und die Kombination der Substituenten Rₘ, R¹ₙ und R² für eine Verbindung jeweils einer Zeile der Tabelle A entspricht

### Tabelle 13

Verbindungen der allgemeinen Formel I (o = 1), in denen R³ 3-Fluor bedeutet, R⁴ für Methyl steht, R⁵X für Ethyl steht und die Kombination der Substituenten Rₘ, R¹ₙ und R² für eine Verbindung jeweils einer Zeile der Tabelle A entspricht

### Tabelle 14

Verbindungen der allgemeinen Formel I (o = 1), in denen R³ 3-Fluor bedeutet, R⁴ für Methyl steht, R⁵X für Methyloxy steht und die Kombination der Substituenten Rₘ, R¹ₙ und R² für eine Verbindung jeweils einer Zeile der Tabelle A entspricht

### Tabelle 15

Verbindungen der allgemeinen Formel I (o = 1), in denen R³ 3-Fluor bedeutet, R⁴ für Methyl steht, R⁵X für Methylamino steht und die Kombination der Substituenten Rₘ, R¹ₙ und R² für eine Verbindung jeweils einer Zeile der Tabelle A entspricht

### Tabelle 16

Verbindungen der allgemeinen Formel I (o = 1), in denen R³ 3-Chlor bedeutet, R⁴ für Methyl steht, R⁵X für Wasserstoff steht und die Kombination der Substituenten Rₘ, R¹ₙ und R² für eine Verbindung jeweils einer Zeile der Tabelle A entspricht

### Tabelle 17

Verbindungen der allgemeinen Formel I (o = 1), in denen R³ 3-Chlor bedeutet, R⁴ für Methyl steht, R⁵X für Methyl steht und die Kombination der Substituenten Rₘ, R¹ₙ und R² für eine Verbindung jeweils einer Zeile der Tabelle A entspricht

### Tabelle 18

Verbindungen der allgemeinen Formel I (o = 1), in denen R³ 3-Chlor bedeutet, R⁴ für Methyl steht, R⁵X für Ethyl steht und die Kombination der Substituenten Rₘ, R¹ₙ und R² für eine Verbindung jeweils einer Zeile der Tabelle A entspricht

### Tabelle 19

Verbindungen der allgemeinen Formel I (o = 1), in denen R³ 3-Chlor bedeutet, R⁴ für Methyl steht, R⁵X für Methyloxy steht und die Kombination der Substituenten Rₘ, R¹ₙ und R² für eine Verbindung jeweils einer Zeile der Tabelle A entspricht

### Tabelle 20

Verbindungen der allgemeinen Formel I (o = 1), in denen R³ 3-Chlor bedeutet, R⁴ für Methyl steht, R⁵X für Methylamino steht und die Kombination der Substituenten Rₘ, R¹ₙ und R² für eine Verbindung jeweils einer Zeile der Tabelle A entspricht

### Tabelle 21

Verbindungen der allgemeinen Formel I (o = 1), in denen R³ 6-Chlor bedeutet, R⁴ für Methyl steht, R⁵X für Wasserstoff steht und die Kombination der Substituenten Rₘ, R¹ₙ und R² für eine Verbindung jeweils einer zeile der Tabelle A entspricht

### Tabelle 22

Verbindungen der allgemeinen Formel I (o = 1), in denen R³ 6-Chlor bedeutet, R⁴ für Methyl steht, R⁵X für Methyl steht und die Kombination der Substituenten Rₘ, R¹ₙ und R² für eine Verbindung jeweils einer Zeile der Tabelle A entspricht

### Tabelle 23

Verbindungen der allgemeinen Formel I (o = 1), in denen R³ 6-Chlor bedeutet, R⁴ für Methyl steht, R⁵X für Ethyl steht und die Kombination der Substituenten Rₘ, R¹ₙ und R² für eine Verbindung jeweils einer Zeile der Tabelle A entspricht

### Tabelle 24

Verbindungen der allgemeinen Formel I (o = 1), in denen R³ 6-Chlor bedeutet, R⁴ für Methyl steht, R⁵X für Methyloxy steht und die Kombination der Substituenten Rₘ, R¹ₙ und R² für eine Verbindung jeweils einer Zeile der Tabelle A entspricht

### Tabelle 25

Verbindungen der allgemeinen Formel I (o = 1), in denen R³ 6-Chlor bedeutet, R⁴ für Methyl steht, R⁵X für Methylamino steht und die Kombination der Substituenten Rₘ, R¹ₙ und R² für eine Verbindung jeweils einer Zeile der Tabelle A entspricht

### Tabelle 26

Verbindungen der allgemeinen Formel I (o = 0), in denen R⁴ für Wasserstoff steht, R⁵X für Wasserstoff steht und die Kombination der Substituenten Rₘ, R¹ₙ und R² für eine Verbindung jeweils einer Zeile der Tabelle A entspricht

### Tabelle 27

Verbindungen der allgemeinen Formel I (o = 0), in denen R⁴ für Wasserstoff steht, R⁵X für Methyl steht und die Kombination der Substituenten Rₘ, R¹ₙ und R² für eine Verbindung jeweils einer Zeile der Tabelle A entspricht

### Tabelle 28

Verbindungen der allgemeinen Formel I (o = 0), in denen R⁴ für Wasserstoff steht, R⁵X für Ethyl steht und die Kombination der Substituenten Rₘ, R¹ₙ und R² für eine Verbindung jeweils einer Zeile der Tabelle A entspricht

### Tabelle 29

Verbindungen der allgemeinen Formel I (o = 0), in denen R⁴ für Wasserstoff steht, R⁵X für Methyloxy steht und die Kombination der Substituenten Rₘ, R¹ₙ und R² für eine Verbindung jeweils einer Zeile der Tabelle A entspricht

### Tabelle 30

Verbindungen der allgemeinen Formel I (o = 0), in denen R⁴ für Wasserstoff steht, R⁵X für Methylamino steht und die Kombination der Substituenten Rₘ, R¹ₙ und R² für eine Verbindung jeweils einer Zeile der Tabelle A entspricht

### Tabelle 31

Verbindungen der allgemeinen Formel I (o = 1), in denen R³ 6-Methyl bedeutet, R⁴ für Methyl steht, R⁵X für Wasserstoff steht und die Kombination der Substituenten Rₘ, R¹ₙ und R² für eine Verbindung jeweils einer Zeile der Tabelle A entspricht

### Tabelle 32

Verbindungen der allgemeinen Formel I (o = 1), in denen R³ 6-Methyl bedeutet, R⁴ für Methyl steht, R⁵X für Methyl steht und die Kombination der Substituenten Rₘ, R¹ₙ und R² für eine Verbindung jeweils einer Zeile der Tabelle A entspricht

### Tabelle 33

Verbindungen der allgemeinen Formel I (o = 1), in denen R³ 6-Methyl bedeutet, R⁴ für Methyl steht, R⁵X für Ethyl steht und die Kombination der Substituenten Rₘ, R¹ₙ und R² für eine Verbindung jeweils einer Zeile der Tabelle A entspricht

### Tabelle 34

Verbindungen der allgemeinen Formel I (o = 1), in denen R³ 6-Methyl bedeutet, R⁴ für Methyl steht, R⁵X für Methyloxy steht und die Kombination der Substituenten Rₘ, R¹ₙ und R² für eine Verbindung jeweils einer Zeile der Tabelle A entspricht

### Tabelle 35

Verbindungen der allgemeinen Formel I (o = 1), in denen R³ 6-Methyl bedeutet, R⁴ für Methyl steht, R⁵X für Methylamino steht und die Kombination der Substituenten Rₘ, R¹ₙ und R² für eine Verbindung jeweils einer Zeile der Tabelle A entspricht

### Tabelle 36

Verbindungen der allgemeinen Formel I (o = 1), in denen R³ 6-Methyl bedeutet, R⁴ für Wasserstoff steht, R⁵X für Wasserstoff steht und die Kombination der Substituenten Rₘ, R¹ₙ und R² für eine Verbindung jeweils einer Zeile der Tabelle A entspricht

### Tabelle 37

Verbindungen der allgemeinen Formel I (o = 1), in denen R³ 6-Methyl bedeutet, R⁴ für Wasserstoff steht, R⁵X für Methyl steht und die Kombination der Substituenten Rₘ, R¹ₙ und R² für eine Verbindung jeweils einer Zeile der Tabelle A entspricht

### Tabelle 38

Verbindungen der allgemeinen Formel I (o = 1), in denen R³ 6-Methyl bedeutet, R⁴ für Wasserstoff steht, R⁵X für Ethyl steht und die Kombination der Substituenten Rₘ, R¹ₙ und R² für eine Verbindung jeweils einer Zeile der Tabelle A entspricht

### Tabelle 39

Verbindungen der allgemeinen Formel I (o = 1), in denen R³ 6-Methyl bedeutet, R⁴ für Wasserstoff steht, R⁵X für Methyloxy steht und die Kombination der Substituenten Rₘ, R¹ₙ und R² für eine Verbindung jeweils einer Zeile der Tabelle A entspricht

### Tabelle 40

Verbindungen der allgemeinen Formel I (o = 1), in denen R³ 6-Methyl bedeutet, R⁴ für Wasserstoff steht, R⁵X für Methylamino steht und die Kombination der Substituenten Rₘ, R¹ₙ und R² für eine Verbindung jeweils einer Zeile der Tabelle A entspricht

### Tabelle 41

Verbindungen der allgemeinen Formel I (o = 1), in denen R³ 6-Chlor bedeutet, R⁴ für Wasserstoff steht, R⁵X für Wasserstoff steht und die Kombination der Substituenten Rₘ, R¹ₙ und R² für eine Verbindung jeweils einer Zeile der Tabelle A entspricht

### Tabelle 42

Verbindungen der allgemeinen Formel I (o = 1), in denen R³ 6-Chlor bedeutet, R⁴ für Wasserstoff steht, R⁵X für Methyl steht und die Kombination der Substituenten Rₘ, R¹ₙ und R² für eine Verbindung jeweils einer Zeile der Tabelle A entspricht

### Tabelle 43

Verbindungen der allgemeinen Formel I (o = 1), in denen R³ 6-Chlor bedeutet, R⁴ für Wasserstoff steht, R⁵X für Ethyl steht und die Kombination der Substituenten Rₘ, R¹ₙ und R² für eine Verbindung jeweils einer Zeile der Tabelle A entspricht

### Tabelle 44

Verbindungen der allgemeinen Formel I (o = 1), in denen R³ 6-Chlor bedeutet, R⁴ für Wasserstoff steht, R⁵X für Methyloxy steht und die Kombination der Substituenten Rₘ, R¹ₙ und R² für eine Verbindung jeweils einer Zeile der Tabelle A entspricht

### Tabelle 45

Verbindungen der allgemeinen Formel I (o = 1), in denen R³ 6-Chlor bedeutet, R⁴ für Wasserstoff steht, R⁵X für Methylamino steht und die Kombination der Substituenten Rₘ, R¹ₙ und R² für eine Verbindung jeweils einer Zeile der Tabelle A entspricht

### Tabelle 46

Verbindungen der allgemeinen Formel I (o = 1), in denen R³ 3-Chlor bedeutet, R⁴ für Wasserstoff steht, R⁵X für Wasserstoff steht und die Kombination der Substituenten Rₘ, R¹ₙ und R² für eine Verbindung jeweils einer Zeile der Tabelle A entspricht

### Tabelle 47

### Verbindungen der allgemeinen Formel I (o = 1), in denen R³ 3-Chlor bedeutet, R⁴ für Wasserstoff steht, R⁵X für Methyl steht und die Kombination der Substituenten Rₘ, R¹ₙ und R² für eine Verbindung jeweils einer Zeile der Tabelle A entspricht

### Tabelle 48

Verbindungen der allgemeinen Formel I (o = 1), in denen R³ 3-Chlor bedeutet, R⁴ für Wasserstoff steht, R⁵X für Ethyl steht und die Kombination der Substituenten Rₘ, R¹ₙ und R² für eine Verbindung jeweils einer Zeile der Tabelle A entspricht

### Tabelle 49

Verbindungen der allgemeinen Formel I (o = 1), in denen R³ 3-Chlor bedeutet, R⁴ für Wasserstoff steht, R⁵X für Methyloxy steht und die Kombination der Substituenten Rₘ, R¹ₙ und R² für eine Verbindung jeweils einer Zeile der Tabelle A entspricht

### Tabelle 50

Verbindungen der allgemeinen Formel I (o = 1), in denen R³ 3-Chlor bedeutet, R⁴ für Wasserstoff steht, R⁵X für Methylamino steht und die Kombination der Substituenten Rₘ, R¹ₙ und R² für eine Verbindung jeweils einer Zeile der Tabelle A entspricht

### Tabelle 51

Verbindungen der allgemeinen Formel I (o = 1), in denen R³ 3-Fluor bedeutet, R⁴ für Wasserstoff steht, R⁵X für Wasserstoff steht und die Kombination der Substituenten Rₘ, R¹ₙ und R² für eine Verbindung jeweils einer Zeile der Tabelle A entspricht

### Tabelle 52

Verbindungen der allgemeinen Formel I (o = 1), in denen R³ 3-Fluor bedeutet, R⁴ für Wasserstoff steht, R⁵X für Methyl steht und die Kombination der Substituenten Rₘ, R¹ₙ und R² für eine Verbindung jeweils einer Zeile der Tabelle A entspricht

### Tabelle 53

Verbindungen der allgemeinen Formel I (o = 1), in denen R³ 3-Flour bedeutet, R⁴ für Wasserstoff steht, R⁵X für Ethyl steht und die Kombination der Substituenten Rₘ, R¹ₙ und R² für eine Verbindung jeweils einer Zeile der Tabelle A entspricht

### Tabelle 54

Verbindungen der allgemeinen Formel I (o = 1), in denen R³ 3-Fluor bedeutet, R⁴ für Wasserstoff steht, R⁵X für Methyloxy steht und die Kombination der Substituenten Rₘ, R¹ₙ und R² für eine Verbindung jeweils einer Zeile der Tabelle A entspricht

### Tabelle 55

Verbindungen der allgemeinen Formel I (o = 1), in denen R³ 3-Fluor bedeutet, R⁴ für Wasserstoff steht, R⁵X für Methylamino steht und die Kombination der Substituenten Rₘ, R¹ₙ und R² für eine Verbindung jeweils einer Zeile der Tabelle A entspricht

**Tabelle A**

| Nummer | Rₘ | R² | R¹ₙ |
|---|---|---|---|
| 12 | 2-F | CH₃ | H |
| 13 | 3-F | CH₃ | H |
| 14 | 4-F | CH₃ | H |
| 15 | 2,4-F₂ | CH₃ | H |
| 16 | 2,4,6-F₃ | CH₃ | H |
| 17 | 2,3,4,5,6-F₅ | CH₃ | H |
| 18 | 2,3-F₂ | CH₃ | H |
| 19 | 2-Cl | CH₃ | H |
| 20 | 3-Cl | CH₃ | H |
| 21 | 4-Cl | CH₃ | H |
| 22 | 2,3-Cl₂ | CH₃ | H |
| 23 | 2,4-Cl₂ | CH₃ | H |
| 24 | 2,5-Cl₂ | CH₃ | H |
| 25 | 2,6-Cl₂ | CH₃ | H |
| 26 | 3,4-Cl₂ | CH₃ | H |
| 27 | 3,5-Cl₂ | CH₃ | H |
| 28 | 2,3,4-Cl₃ | CH₃ | H |
| 29 | 2,3,5-Cl₃ | CH₃ | H |
| 30 | 2,3,6-Cl₃ | CH₃ | H |
| 31 | 2,4,5-Cl₃ | CH₃ | H |
| 32 | 2,4,6-Cl₃ | CH₃ | H |
| 33 | 3,4,5-Cl₃ | CH₃ | H |
| 34 | 2,3,4,6-Cl₄ | CH₃ | H |
| 35 | 2,3,5,6-Cl₄ | CH₃ | H |
| 36 | 2,3,4,5,6-Cl₅ | CH₃ | H |
| 37 | 2 -Br | CH₃ | H |
| 38 | 3 -Br | CH₃ | H |
| 3 9 | 4 -Br | CH₃ | H |
| 40 | 2,4-Br₂ | CH₃ | H |
| 41 | 2,5-Br₂ | CH₃ | H |
| 42 | 2,6-Br₂ | CH₃ | H |
| 43 | 2,4,6-Br₃ | CH₃ | H |
| 44 | 2,3,4,5,6-Br₅ | CH₃ | H |
| 45 | 2-J | CH₃ | H |
| 46 | 3-J | CH₃ | H |
| 47 | 4-J | CH₃ | H |
| 48 | 2,4-J₂ | CH₃ | H |
| 49 | 2-Cl, 3-F | CH₃ | H |
| 50 | 2-Cl, 4-F | CH₃ | H |
| 51 | 2-Cl, 5-F | CH₃ | H |
| 52 | 2-Cl, 6-F | CH₃ | H |
| 53 | 2-Cl, 3-Br | CH₃ | H |
| 54 | 2-Cl, 4-Br | CH₃ | H |
| 55 | 2-Cl, 5-Br | CH₃ | H |
| 56 | 2-Cl, 6-Br | CH₃ | H |
| 57 | 2-Br, 3-Cl | CH₃ | H |
| 58 | 2-Br, 4-Cl | CH₃ | H |
| 59 | 2-Br, 5-Cl | CH₃ | H |
| 60 | 2-Br, 3-F | CH₃ | H |
| 61 | 2-Br, 4-F | CH₃ | H |
| 62 | 2-Br, 5-F | CH₃ | H |
| 63 | 2-Br, 6-F | CH₃ | H |
| 64 | 2-F, 3-Cl | CH₃ | H |
| 65 | 2-F, 4-Cl | CH₃ | H |
| 66 | 2-F, 5-Cl | CH₃ | H |
| 67 | 3-Cl, 4-F | CH₃ | H |
| 68 | 3-Cl, 5-F | CH₃ | H |
| 69 | 3-Cl, 4-Br | CH₃ | H |
| 70 | 3-Cl, 5-Br | CH₃ | H |
| 71 | 3-F, 4-Cl | CH₃ | H |
| 72 | 3-F, 4-Br | CH₃ | H |
| 73 | 3-Br, 4-Cl | CH₃ | H |
| 74 | 3-Br, 4-F | CH₃ | H |
| 75 | 2,6-Cl₂, 4-Br | CH₃ | H |
| 76 | 2-CH₃ | CH₃ | H |
| 77 | 3-CH₃ | CH₃ | H |
| 78 | 4-CH₃ | CH₃ | H |
| 79 | 2,3-(CH₃)₂ | CH₃ | H |
| 80 | 2,4-(CH₃)₂ | CH₃ | H |
| 81 | 2,5-(CH₃)₂ | CH₃ | H |
| 82 | 2,6-(CH₃)₂ | CH₃ | H |
| 83 | 3,4-(CH₃)₂ | CH₃ | H |
| 84 | 3,5-(CH₃)₂ | CH₃ | H |
| 85 | 2,3,5-(CH₃)₃ | CH₃ | H |
| 86 | 2,3,4-(CH₃)₃ | CH₃ | H |
| 87 | 2,3,6-(CH₃)₃ | CH₃ | H |
| 88 | 2,4,5-(CH₃)₃ | CH₃ | H |
| 89 | 2,4,6-(CH₃)₃ | CH₃ | H |
| 90 | 3,4,5-(CH₃)₃ | CH₃ | H |
| 91 | 2,3,4,6-(CH₃)₄ | CH₃ | H |
| 92 | 2,3,5,6-(CH₃)₄ | CH₃ | H |
| 93 | 2,3,4,5,6-(CH₃)₅ | CH₃ | H |
| 94 | 2-C₂H₅ | CH₃ | H |
| 95 | 3-C₂H₅ | CH₃ | H |
| 96 | 4-C₂H₅ | CH₃ | H |
| 97 | 2,4-(C₂H₅)₅ | CH₃ | H |
| 98 | 2,6-(C₂H₅)₂ | CH₃ | H |
| 99 | 3,5-(C₂H₅)₂ | CH₃ | H |
| 100 | 2,4,6-(C₂H₅)₃ | CH₃ | H |
| 101 | 2-n-C₃H₇ | CH₃ | H |
| 102 | 3-n-C₃H₇ | CH₃ | H |
| 103 | 4-n-C₃H₇ | CH₃ | H |
| 104 | 2-i-C₃H₇ | CH₃ | H |
| 105 | 3-i-C₃H₇ | CH₃ | H |
| 106 | 4-i-C₃H₇ | CH₃ | H |
| 107 | 2,4-(i-C₃H₇)₂ | CH₃ | H |
| 108 | 2,6-(i-C₃H₇)₂ | CH₃ | H |
| 109 | 3,5-(i-C₃H₇)₂ | CH₃ | H |
| 110 | 2-s-C₄H₉ | CH₃ | H |
| 111 | 3-s-C₄H₉ | CH₃ | H |
| 112 | 4-s-C₄H₉ | CH₃ | H |
| 113 | 2-t-C₄H₉ | CH₃ | H |
| 114 | 3-t-C₄H₉ | CH₃ | H |
| 115 | 4-t-C₄H₉ | CH₃ | H |
| 116 | 4-n-C₉H₁₉ | CH₃ | H |
| 117 | 2-CH₃, 4-t-C₄H₉ | CH₃ | H |
| 118 | 2-CH₃, 6-t-C₄H₉ | CH₃ | H |
| 119 | 2-CH₃, 4-i-C₃H₇ | CH₃ | H |
| 120 | 2-CH₃, 5-i-C₃H₇ | CH₃ | H |
| 121 | 3-CH₃ , 4-i-C₃H₇ | CH₃ | H |
| 122 | 2-cyclo-C₆H₁₁ | CH₃ | H |
| 123 | 3-cyclo-C₆H₁₁ | CH₃ | H |
| 124 | 4-cyclo-C₆H₁₁ | CH₃ | H |
| 125 | 2-Cl, 4-C₆H₅ | CH₃ | H |
| 126 | 2-Br, 4-C₆H₅ | CH₃ | H |
| 127 | 2-OCH₃ | CH₃ | H |
| 128 | 3-OCH₃ | CH₃ | H |
| 129 | 4-OCH₃ | CH₃ | H |
| 130 | 2-OC₂H₅ | CH₃ | H |
| 131 | 3-O-C₂H₅ | CH₃ | H |
| 132 | 4-O-C₂H₅ | CH₃ | H |
| 133 | 2-O-n-C₃H₇ | CH₃ | H |
| 134 | 3-O-n-C₃H₇ | CH₃ | H |
| 135 | 4-O-n-C₃H₇ | CH₃ | H |
| 136 | 2-O-i-C₃H₇ | CH₃ | H |
| 137 | 3-O-i-C₃H₇ | CH₃ | H |
| 138 | 4-O-i-C₃H₇ | CH₃ | H |
| 139 | 2-O-n-C₆H₁₃ | CH₃ | H |
| 140 | 3-O-n-C₆H₁₃ | CH₃ | H |
| 141 | 4-O-n-C₆H₁₃ | CH₃ | H |
| 142 | 2-O-CH₂C₆H₅ | CH₃ | H |
| 143 | 3-O-CH₂C₆H₅ | CH₃ | H |
| 144 | 4-O-CH₂C₆H₅ | CH₃ | H |
| 145 | 2-O-(CH₂)₃C₆H₅ | CH₃ | H |
| 146 | 4-O-(CH₂)₃C₆H₅ | CH₃ | H |
| 147 | 2,3-(OCH₃)₂ | CH₃ | H |
| 148 | 2,4-(OCH₃)₂ | CH₃ | H |
| 149 | 2,5-(OCH₃)₂ | CH₃ | H |
| 150 | 2,6-(OCH₃)₂ | CH₃ | H |
| 151 | 3,4-(OCH₃)₂ | CH₃ | H |
| 152 | 3,5-(OCH₃)₂ | CH₃ | H |
| 153 | 2-O-t-C₄H₉ | CH₃ | H |
| 154 | 3-O-t-C₄H₉ | CH₃ | H |
| 155 | 4-O-t-C₄H₉ | CH₃ | H |
| 156 | 3-(3'-Cl-C₆H₄) | CH₃ | H |
| 157 | 4-(4'-CH₃-C₆H₄) | CH₃ | H |
| 158 | 2-O-C₆H₅ | CH₃ | H |
| 159 | 3-O-C₆H₅ | CH₃ | H |
| 160 | 4-O-C₆H₅ | CH₃ | H |
| 161 | 2-O-(2'-F-C₆H₄) | CH₃ | H |
| 162 | 3-O-(3'-Cl-C₆H₄) | CH₃ | H |
| 163 | 4-O-(4'-CH₃-C₆H₄) | CH₃ | H |
| 164 | 2,3,6-(CH₃)₃, 4-F | CH₃ | H |
| 165 | 2,3,6-(CH₃)₃, 4-Cl | CH₃ | H |
| 166 | 2,3,6-(CH₃)₃,4-Br | CH₃ | H |
| 167 | 2,4-(CH₃)₂, 6-F | CH₃ | H |
| 168 | 2,4-(CH₃)₂, 6-Cl | CH₃ | H |
| 169 | 2,4-(CH₃)₂, 6-Br | CH₃ | H |
| 170 | 2-i-C₃H₇, 4-Cl, 5-CH₃ | CH₃ | H |
| 171 | 2-Cl, 4-NO₂ | CH₃ | H |
| 172 | 2-NO₂, 4-Cl | CH₃ | H |
| 173 | 2-OCH₃, 5-NO₂ | CH₃ | H |
| 174 | 2,4-Cl₂, 5-NO₂ | CH₃ | H |
| 175 | 2,4-Cl₂,6-NO₂ | CH₃ | H |
| 176 | 2,6-Cl₂, 4-NO₂ | CH₃ | H |
| 177 | 2,6-Br₂,4-NO₂ | CH₃ | H |
| 178 | 2,6-J₂,4-NO₂ | CH₃ | H |
| 179 | 2-CH₃, 5-i-C₃H₇ , 4-Cl | CH₃ | H |
| 180 | 2-CO₂CH₃ | CH₃ | H |
| 181 | 3-CO₂CH₃ | CH₃ | H |
| 182 | 4-CO₂CH₃ | CH₃ | H |
| 183 | 2-CH₂-OCH₃ | CH₃ | H |
| 184 | 3-CH₂-OCH₃ | CH₃ | H |
| 185 | 4-CH₂-OCH₃ | CH₃ | H |
| 186 | 2-Me-4-CH₃-CH(CH₃)-CO | CH₃ | H |
| 187 | 2-CH₃-4-(CH₃-C=NOCH₃) | CH₃ | H |
| 188 | 2-CH₃-4-(CH₃-C=NOC₂H₅) | CH₃ | H |
| 189 | 2-CH₃-4-(CH₃-C=NO-n-C₃H₇) | CH₃ | H |
| 190 | 2-CH₃-4-(CH₃-C=NO-i-C₃H₇) | CH₃ | H |
| 191 | 2,5-(CH₃)₂-4-(CH₃-C=NOCH₃) | CH₃ | H |
| 192 | 2,5-(CH₃)₂-4-(CH₃-C=NOC₂H₅) | CH₃ | H |
| 193 | 2,5-(CH₃-4-(CH₃-C=NO-n-C₃H₇) | CH₃ | H |
| 194 | 2,5-(CH₃)₂-4-(CH₃-C=NO-i-C₃H₇) | CH₃ | H |
| 195 | 2-C₆H₅ | CH₃ | H |
| 196 | 3-C₆H₅ | CH₃ | H |
| 197 | 4-C₆H₅ | CH₃ | H |
| 198 | 2-(2'-F-C₆H₄) | CH₃ | H |
| 199 | 2-CH₃, 5-Br | CH₃ | H |
| 200 | 2-CH₃, 6-Br | CH₃ | H |
| 201 | 2-Cl, 3-CH₃ | CH₃ | H |
| 202 | 2-Cl, 4-CH₃ | CH₃ | H |
| 203 | 2-Cl, 5-CH₃ | CH₃ | H |
| 204 | 2-F, 3-CH₃ | CH₃ | H |
| 205 | 2-F, 4-CH₃ | CH₃ | H |
| 206 | 2-F, 5-CH₃ | CH₃ | H |
| 207 | 2-Br, 3-CH₃ | CH₃ | H |
| 208 | 2-Br, 4-CH₃ | CH₃ | H |
| 209 | 2-Br, 5-CH₃ | CH₃ | H |
| 210 | 3-CH₃, 4-Cl | CH₃ | H |
| 211 | 3-CH₃, 5-Cl | CH₃ | H |
| 212 | 3-CH₃, 4-F | CH₃ | H |
| 213 | 3-CH₃, 5-F | CH₃ | H |
| 214 | 3-CH₃, 4-Br | CH₃ | H |
| 215 | 3-CH₃, 5-Br | CH₃ | H |
| 216 | 3-F, 4-CH₃ | CH₃ | H |
| 217 | 3-Cl, 4-CH₃ | CH₃ | H |
| 218 | 3-Br, 4-CH₃ | CH₃ | H |
| 219 | 2-Cl, 4,5-(CH₃)₂ | CH₃ | H |
| 220 | 2-Br, 4,5-(CH₃)₂ | CH₃ | H |
| 221 | 2-Cl, 3,5-(CH₃)₂ | CH₃ | H |
| 222 | 2-Br, 3,5-(CH₃)₂ | CH₃ | H |
| 223 | 2,6-Cl₂ , 4-CH₃ | CH₃ | H |
| 224 | 2,6-F₂,4-CH₃ | CH₃ | H |
| 225 | 2,6-Br₂,4-CH₃ | CH₃ | H |
| 226 | 2,4-Br₂,6-CH₃ | CH₃ | H |
| 227 | 2,4-F₂, 6-CH₃ | CH₃ | H |
| 228 | 2,4-Br₂,6-CH₃ | CH₃ | H |
| 229 | 2,6-(CH₃)₂, 4-F | CH₃ | H |
| 230 | 2,6-(CH₃)₂, 4-Cl | CH₃ | H |
| 231 | 2,6-(CH₃)₂,4-Br | CH₃ | H |
| 232 | 3,5-(CH₃)₂, 4-F | CH₃ | H |
| 233 | 3,5-(CH₃)₂, 4-Cl | CH₃ | H |
| 234 | 3,5-(CH₃)₂,4-Br | CH₃ | H |
| 235 | 2-CF₃ | CH₃ | H |
| 236 | 3-CF₃ | CH₃ | H |
| 237 | 4-CF₃ | CH₃ | H |
| 238 | 2-OCF₃ | CH₃ | H |
| 239 | 3-OCF₃ | CH₃ | H |
| 240 | 4-OCF₃ | CH₃ | H |
| 241 | 3-OCH₂CHF₂ | CH₃ | H |
| 242 | 2-NO₂ | CH₃ | H |
| 243 | 3-NO₂ | CH₃ | H |
| 244 | 4-NO₂ | CH₃ | H |
| 245 | 2-CN | CH₃ | H |
| 246 | 3-CN | CH₃ | H |
| 247 | 4-CN | CH₃ | H |
| 248 | 2-CH₃, 3-Cl | CH₃ | H |
| 249 | 2-CH₃, 4-Cl | CH₃ | H |
| 250 | 2-CH₃, 5-Cl | CH₃ | H |
| 251 | 2-CH₃, 6-Cl | CH₃ | H |
| 252 | 2-CH₃, 3-F | CH₃ | H |
| 253 | 2-CH₃, 4-F | CH₃ | H |
| 254 | 2-CH₃, 5-F | CH₃ | H |
| 255 | 2-CH₃, 6-F | CH₃ | H |
| 256 | 2-CH₃, 3-Br | CH₃ | H |
| 257 | 2-CH₃, 4-Br | CH₃ | H |
| 258 | 2-Pyridyl-2' | CH₃ | H |
| 259 | 3-Pyridyl-3' | CH₃ | H |
| 260 | 4-Pyridyl-4' | CH₃ | H |
| 261 | 2-CO-CH₃ | CH₃ | H |
| 262 | 3-CO-CH₃ | CH₃ | H |
| 263 | 4-CO-CH₃ | CH₃ | H |
| 264 | 2-C(=N-OCH₃)-CH₃ | CH₃ | H |
| 265 | 3-C(=N-OCH₃)-CH₃ | CH₃ | H |
| 266 | 4-C(=N-OCH₃)-CH₃ | CH₃ | H |
| 267 | 2-F | F | H |
| 268 | 3-F | F | H |
| 269 | 4-F | F | H |
| 270 | 2,4-F₂ | F | H |
| 271 | 2,4,6-F₃ | F | H |
| 272 | 2,3,4,5,6-F₅ | F | H |
| 273 | 2,3-F₂ | F | H |
| 274 | 2-Cl | F | H |
| 275 | 3-Cl | F | H |
| 276 | 4-Cl | F | H |
| 277 | 2,3-Cl₂ | F | H |
| 278 | 2,4-Cl₂ | F | H |
| 279 | 2,5-Cl₂ | F | H |
| 280 | 2,6-Cl₂ | F | H |
| 281 | 3,4-Cl₂ | F | H |
| 282 | 3,5-Cl₂ | F | H |
| 283 | 2,3,4-Cl₃ | F | H |
| 284 | 2,3,5-Cl₃ | F | H |
| 285 | 2,3,6-Cl₃ | F | H |
| 286 | 2,4,5-Cl₃ | F | H |
| 287 | 2,4,6-Cl₃ | F | H |
| 288 | 3,4,5-Cl₃ | F | H |
| 289 | 2,3,4,6-Cl₄ | F | H |
| 290 | 2,3,5,6-Cl₄ | F | H |
| 291 | 2,3,4,5,6-Cl₅ | F | H |
| 292 | 2-Br | F | H |
| 293 | 3-Br | F | H |
| 294 | 4-Br | F | H |
| 295 | 2,4-Br₂ | F | H |
| 296 | 2,5-Br₂ | F | H |
| 297 | 2,6-Br₂ | F | H |
| 298 | 2,4,6-Br₃ | F | H |
| 299 | 2,3,4,5,6-Br₅ | F | H |
| 300 | 2-J | F | H |
| 301 | 3-J | F | H |
| 302 | 4-J | F | H |
| 303 | 2,4-J₂ | F | H |
| 304 | 2-Cl, 3-F | F | H |
| 305 | 2-Cl, 4-F | F | H |
| 306 | 2-Cl, 5-F | F | H |
| 307 | 2-Cl, 6-F | F | H |
| 308 | 2-Cl, 3-Br | F | H |
| 309 | 2-Cl, 4-Br | F | H |
| 310 | 2-Cl, 5-Br | F | H |
| 311 | 2-Cl, 6-Br | F | H |
| 312 | 2-Br, 3-Cl | F | H |
| 313 | 2-Br, 4-Cl | F | H |
| 314 | 2-Br, 5-Cl | F | H |
| 315 | 2-Br, 3-F | F | H |
| 316 | 2-Br, 4-F | F | H |
| 317 | 2-Br, 5-F | F | H |
| 318 | 2-Br, 6-F | F | H |
| 319 | 2-F, 3-Cl | F | H |
| 320 | 2-F, 4-Cl | F | H |
| 321 | 2-F 5-Cl | F | H |
| 322 | 3-Cl, 4-F | F | H |
| 323 | 3-Cl, 5-F | F | H |
| 324 | 3-Cl, 4-Br | F | H |
| 325 | 3-Cl, 5-Br | F | H |
| 326 | 3-F, 4-Cl | F | H |
| 327 | 3-F, 4-Br | F | H |
| 328 | 3-Br, 4-Cl | F | H |
| 329 | 3-Br, 4-F | F | H |
| 330 | 2,6-Cl₂, 4-Br | F | H |
| 331 | 2-CH₃ | F | H |
| 332 | 3-CH₃ | F | H |
| 333 | 4-CH₃ | F | H |
| 334 | 2,3-(CH₃)₂ | F | H |
| 335 | 2,4-(CH₃)₂ | F | H |
| 336 | 2,5-(CH₃)₂ | F | H |
| 337 | 2,6-(CH₃)₂ | F | H |
| 338 | 3,4-(CH₃)₂ | F | H |
| 339 | 3,5-(CH₃)₂ | F | H |
| 340 | 2,3,5-(CH₃)₃ | F | H |
| 341 | 2,3,4-(CH₃)₃ | F | H |
| 342 | 2,3,6-(CH₃)₃ | F | H |
| 343 | 2,4,5-(CH₃)₃ | F | H |
| 344 | 2,4,6-(CH₃)₃ | F | H |
| 345 | 3,4,5-(CH₃)₃ | F | H |
| 346 | 2,3,4,6-(CH₃)₄ | F | H |
| 347 | 2,3,5,6-(CH₃)₄ | F | H |
| 348 | 2,3,4,5,6-(CH₃)₅ | F | H |
| 349 | 2-C₂H₅ | F | H |
| 350 | 3-C₂H₅ | F | H |
| 351 | 4-C₂H₅ | F | H |
| 352 | 2,4-(C₂H₅)₅ | F | H |
| 353 | 2,6-(C₂H₅)₂ | F | H |
| 354 | 3,5-(C₂H₅)₂ | F | H |
| 355 | 2,4,6-(C₂H₅)₃ | F | H |
| 356 | 2-n-C₃H₇ | F | H |
| 357 | 3-n-C₃H₇ | F | H |
| 358 | 4-n-C₃H₇ | F | H |
| 359 | 2-i-C₃H₇ | F | H |
| 360 | 3-i-C₃H₇ | F | H |
| 361 | 4-i-C₃H₇ | F | H |
| 362 | 2,4-(i-C₃H₇)₂ | F | H |
| 363 | 2,6-(i-C₃H₇)₂ | F | H |
| 364 | 3,5-(i-C₃H₇)₂ | F | H |
| 365 | 2-s-C₄H₉ | F | H |
| 366 | 3-s-C₄H₉ | F | H |
| 367 | 4-s-C₄H₉ | F | H |
| 368 | 2-t-C₄H₉ | F | H |
| 369 | 3-t-C₄H₉ | F | H |
| 370 | 4-t-C₄H₉ | F | H |
| 371 | 4-n-C₉H₁₉ | F | H |
| 372 | 2-CH₃, 4-t-C₄H₉ | F | H |
| 373 | 2-CH₃, 6-t-C₄H₉ | F | H |
| 374 | 2-CH₃ , 4-i-C₃H₇ | F | H |
| 375 | 2-CH₃ , 5-i-C₃H₇ | F | H |
| 376 | 3-CH₃, 4-i-C₃H₇ | F | H |
| 377 | 2-cyclo-C₆H₁₁ | F | H |
| 378 | 3-cyclo-C₆H₁₁ | F | H |
| 379 | 4-cyclo-C₆H₁₁ | F | H |
| 380 | 2-Cl, 4-C₆H₅ | F | H |
| 381 | 2-Br, 4-C₆H₅ | F | H |
| 382 | 2-OCH₃ | F | H |
| 383 | 3-OCH₃ | F | H |
| 384 | 4-OCH₃ | F | H |
| 385 | 2-OC₂H₅ | F | H |
| 386 | 3-O-C₂H₅ | F | H |
| 387 | 4-O-C₂H₅ | F | H |
| 388 | 2-O-n-C₃H₇ | F | H |
| 389 | 3-O-n-C₃H₇ | F | H |
| 390 | 4-O-n-C₃H₇ | F | H |
| 391 | 2-O-i-C₃H₇ | F | H |
| 392 | 3-O-i-C₃H₇ | F | H |
| 393 | 4-O-i-C₃H₇ | F | H |
| 394 | 2-O-n-C₆H₁₃ | F | H |
| 391 | 3-O-n-C₆H₁₃ | F | H |
| 396 | 4-O-n-C₆H₁₃ | F | H |
| 397 | 2-O-CH₂C₆H₅ | F | H |
| 398 | 3-O-CH₂C₆H₅ | F | H |
| 399 | 4-O-CH₂C₆H₅ | F | H |
| 400 | 2-O-(CH₂)₃C₆H₅ | F | H |
| 401 | 4-O-(CH₂)₃C₆H₅ | F | H |
| 402 | 2,3-(OCH₃)₂ | F | H |
| 403 | 2,4-(OCH₃)₂ | F | H |
| 404 | 2,5-(OCH₃)₂ | F | H |
| 405 | 2,6-(OCH₃)₂ | F | H |
| 406 | 3,4-(OCH₃)₂ | F | H |
| 407 | 3,5-(OCH₃)₂ | F | H |
| 408 | 2-O-t-C₄H₉ | F | H |
| 409 | 3-O-t-C₄H₉ | F | H |
| 410 | 4-O-t-C₄H₉ | F | H |
| 411 | 3-(3'-Cl-C₆H₄) | F | H |
| 412 | 4-(4'-CH₃-C₆H₄) | F | H |
| 413 | 2-O-C₆H₅ | F | H |
| 414 | 3-O-C₆H₅ | F | H |
| 415 | 4-O-C₆H₅ | F | H |
| 416 | 2-O-(2'-F-C₆H₄) | F | H |
| 417 | 3-O-(3'-Cl-C₆H₄) | F | H |
| 418 | 4-O-(4'-CH₃-C₆H₄) | F | H |
| 419 | 2,3,6-(CH₃)₃, 4-F | F | H |
| 420 | 2,3,6-(CH₃)₃, 4-Cl | F | H |
| 421 | 2,3,6-(CH₃)₃, 4-Br | F | H |
| 422 | 2,4-(CH₃)₂, 6-F | F | H |
| 423 | 2,4-(CH₃)₂, 6-Cl | F | H |
| 424 | 2,4-(CH₃)₂, 6-Br | F | H |
| 425 | 2-i-C₃H₇, 4-Cl, 5-CH₃ | F | H |
| 426 | 2-Cl, 4-NO₂ | F | H |
| 427 | 2-NO₂, 4-Cl | F | H |
| 428 | 2-OCH₃, 5-NO₂ | F | H |
| 429 | 2,4-Cl₂,5-NO₂ | F | H |
| 430 | 2,4-Cl₂, 6-NO₂ | F | H |
| 431 | 2,6-Cl₂, 4-NO₂ | F | H |
| 432 | 2,6-Br₂, 4-NO₂ | F | H |
| 433 | 2,6-J₂,4-NO₂ | F | H |
| 434 | 2-CH₃, 5-i-C₃H₇, 4-Cl | F | H |
| 435 | 2-CO₂CH₃ | F | H |
| 436 | 3-CO₂CH₃ | F | H |
| 437 | 4-CO₂CH₃ | F | H |
| 438 | 2-CH₂-OCH₃ | F | H |
| 439 | 3-CH₂-OCH₃ | F | H |
| 440 | 4-CH₂-OCH₃ | F | H |
| 441 | 2-Me-4-CH₃-CH(CH₃)-CO | F | H |
| 442 | 2-CH₃-4-(CH₃-C=NOCH₃) | F | H |
| 443 | 2-CH₃-4-(CH₃-C=NOC₂H₅) | F | H |
| 444 | 2-CH₃-4-(CH₃-C=NO-n-C₃H₇) | F | H |
| 445 | 2-CH₃-4-(CH₃-C=NO-i-C₃H₇) | F | H |
| 446 | 2,5-(CH₃)₂-4-(CH₃-C=NOCH₃) | F | H |
| 447 | 2,5-(CH₃)₂-4-(CH₃-C=NOC₂H₅) | F | H |
| 448 | 2,5-(CH₃-4-(CH₃-C=NO-n-C₃H₇) | F | H |
| 449 | 2,5-(CH₃)2-4-(CH₃-C=NO-i-C₃H₇) | F | H |
| 450 | 2-C₆H₅ | F | H |
| 451 | 3-C₆H₅ | F | H |
| 452 | 4-C₆H₅ | F | H |
| 453 | 2-(2'-F-C₆H₄) | F | H |
| 454 | 2-CH₃, 5-Br | F | H |
| 455 | 2-CH₃, 6-Br | F | H |
| 456 | 2-Cl, 3-CH₃ | F | H |
| 457 | 2-Cl, 4-CH₃ | F | H |
| 458 | 2-Cl, 5-CH₃ | F | H |
| 459 | 2-F, 3-CH₃ | F | H |
| 460 | 2-F, 4-CH₃ | F | H |
| 461 | 2-F, 5-CH₃ | F | H |
| 462 | 2-Br, 3-CH₃ | F | H |
| 463 | 2-Br, 4-CH₃ | F | H |
| 464 | 2-Br, 5-CH₃ | F | H |
| 465 | 3-CH₃, 4-Cl | F | H |
| 466 | 3-CH₃, 5-Cl | F | H |
| 467 | 3-CH₃, 4-F | F | H |
| 468 | 3-CH₃, 5-F | F | H |
| 469 | 3-CH₃, 4-Br | F | H |
| 470 | 3-CH₃, 5-Br | F | H |
| 471 | 3-F, 4-CH₃ | F | H |
| 472 | 3-Cl, 4-CH₃ | F | H |
| 473 | 3-Br, 4-CH₃ | F | H |
| 474 | 2-Cl, 4,5-(CH₃)₂ | F | H |
| 475 | 2-Br, 4,5-(CH₃)₂ | F | H |
| 476 | 2-Cl, 3,5-(CH₃)₂ | F | H |
| 477 | 2-Br, 3,5-(CH₃)₂ | F | H |
| 478 | 2,6-Cl₂, 4-CH₃ | F | H |
| 479 | 2,6-F₂, 4-CH₃ | F | H |
| 480 | 2,6-Br₂, 4-CH₃ | F | H |
| 481 | 2,4-Br₂, 6-CH₃ | F | H |
| 482 | 2,4-F₂, 6-CH₃ | F | H |
| 483 | 2,4-Br₂,6-CH₃ | F | H |
| 484 | 2,6-(CH₃)₂, 4-F | F | H |
| 485 | 2,6-(CH₃)₂, 4-Cl | F | H |
| 486 | 2,6-(CH₃)₂, 4-Br | F | H |
| 487 | 3,5-(CH₃)₂, 4-F | F | H |
| 488 | 3,5-(CH₃)₂, 4-Cl | F | H |
| 489 | 3,5-(CH₃)₂, 4-Br | F | H |
| 490 | 2-CF₃ | F | H |
| 491 | 3-CF₃ | F | H |
| 492 | 4-CF₃ | F | H |
| 493 | 2-OCF₃ | F | H |
| 494 | 3-OCF₃ | F | H |
| 495 | 4-OCF₃ | F | H |
| 496 | 3-OCH₂CHF₂ | F | H |
| 497 | 2-NO₂ | F | H |
| 498 | 3-NO₂ | F | H |
| 499 | 4-NO₂ | F | H |
| 500 | 2-CN | F | H |
| 501 | 3-CN | F | H |
| 502 | 4-CN | F | H |
| 503 | 2-CH₃, 3-Cl | F | H |
| 504 | 2-CH₃, 4-Cl | F | H |
| 505 | 2-CH₃, 5-Cl | F | H |
| 506 | 2-CH₃, 6-Cl | F | H |
| 507 | 2-CH₃, 3-F | F | H |
| 508 | 2-CH₃, 4-F | F | H |
| 509 | 2-CH₃, 5-F | F | H |
| 510 | 2-CH₃, 6-F | F | H |
| 511 | 2-CH₃, 3-Br | F | H |
| 512 | 2-CH₃, 4-Br | F | H |
| 513 | 2-Pyridyl-2' | F | H |
| 514 | 3-Pyridyl-3' | F | H |
| 515 | 4-Pyridyl-4' | F | H |
| 516 | 2-CO-CH₃ | F | H |
| 517 | 3-CO-CH₃ | F | H |
| 518 | 4-CO-CH₃ | F | H |
| 519 | 2-C(=N-OCH₃)-CH₃ | F | H |
| 520 | 3-C(=N-OCH₃)-CH₃ | F | H |
| 521 | 4-C(=N-OCH₃)-CH₃ | F | H |
| 522 | 2-F | Cl | H |
| 523 | 3-F | Cl | H |
| 524 | 4-F | Cl | H |
| 525 | 2,4-F₂ | Cl | H |
| 526 | 2,4,6-F₃ | Cl | H |
| 527 | 2,3,4,5,6-F₅ | Cl | H |
| 528 | 2,3-F₂ | Cl | H |
| 529 | 2-Cl | Cl | H |
| 530 | 3-Cl | Cl | H |
| 531 | 4-Cl | Cl | H |
| 532 | 2,3-Cl₂ | Cl | H |
| 533 | 2,4-Cl₂ | Cl | H |
| 534 | 2,5-Cl₂ | Cl | H |
| 535 | 2,6-Cl₂ | Cl | H |
| 536 | 3,4-Cl₂ | Cl | H |
| 537 | 3,5-Cl₂ | Cl | H |
| 538 | 2,3,4-Cl₃ | Cl | H |
| 539 | 2,3,5-Cl₃ | Cl | H |
| 540 | 2,3,6-Cl₃ | Cl | H |
| 541 | 2,4,5-Cl₃ | Cl | H |
| 542 | 2,4,6-C13 | Cl | H |
| 543 | 3,4,5-Cl₃ | Cl | H |
| 544 | 2,3,4,6-Cl₄ | Cl | H |
| 545 | 2,3,5,6-Cl₄ | Cl | H |
| 546 | 2,3,4,5,6-Cl₅ | Cl | H |
| 547 | 2-Br | Cl | H |
| 548 | 3-Br | Cl | H |
| 549 | 4-Br | Cl | H |
| 550 | 2,4-Br₂ | Cl | H |
| 551 | 2,5-Br₂ | Cl | H |
| 552 | 2,6-Br₂ | Cl | H |
| 553 | 2,4,6-Br₃ | Cl | H |
| 554 | 2,3,4,5,6-Br₅ | Cl | H |
| 555 | 2-J | Cl | H |
| 556 | 3-J | Cl | H |
| 557 | 4-J | Cl | H |
| 558 | 2,4-J₂ | Cl | H |
| 559 | 2-Cl, 3-F | Cl | H |
| 560 | 2-Cl, 4-F | Cl | H |
| 561 | 2-Cl, 5-F | Cl | H |
| 562 | 2-Cl, 6-F | Cl | H |
| 563 | 2-Cl, 3-Br | Cl | H |
| 564 | 2-Cl, 4-Br | Cl | H |
| 565 | 2-Cl, 5-Br | Cl | H |
| 566 | 2-Cl, 6-Br | Cl | H |
| 567 | 2-Br, 3-Cl | Cl | H |
| 568 | 2-Br, 4-Cl | Cl | H |
| 569 | 2-Br, 5-Cl | Cl | H |
| 570 | 2-Br, 3-F | Cl | H |
| 571 | 2-Br, 4-F | Cl | H |
| 572 | 2-Br, 5-F | Cl | H |
| 573 | 2-Br, 6-F | Cl | H |
| 574 | 2-F, 3-Cl | Cl | H |
| 575 | 2-F, 4-Cl | Cl | H |
| 576 | 2-F, 5-Cl | Cl | H |
| 577 | 3-Cl, 4-F | Cl | H |
| 578 | 3-Cl, 5-F | Cl | H |
| 579 | 3-Cl, 4-Br | Cl | H |
| 580 | 3-Cl, 5-Br | Cl | H |
| 581 | 3-F, 4-Cl | Cl | H |
| 582 | 3-F, 4-Br | Cl | H |
| 583 | 3-Br, 4-Cl | Cl | H |
| 584 | 3-Br, 4-F | Cl | H |
| 585 | 2,6-Cl₂, 4-Br | Cl | H |
| 586 | 2-CH₃ | Cl | H |
| 587 | 3-CH₃ | Cl | H |
| 588 | 4-CH₃ | Cl | H |
| 589 | 2,3-(CH₃)₂ | Cl | H |
| 590 | 2,4-(CH₃)₂ | Cl | H |
| 591 | 2,5-(CH₃)₂ | Cl | H |
| 592 | 2,6-(CH₃)₂ | Cl | H |
| 593 | 3,4-(CH₃)₂ | Cl | H |
| 594 | 3,5-(CH₃)₂ | Cl | H |
| 595 | 2,3,5-(CH₃)₃ | Cl | H |
| 596 | 2,3,4-(CH₃)₃ | Cl | H |
| 597 | 2,3,6-(CH₃)₃ | Cl | H |
| 598 | 2,4,5-(CH₃)₃ | Cl | H |
| 599 | 2,4,6-(CH₃)₃ | Cl | H |
| 600 | 3,4,5-(CH₃)₃ | Cl | H |
| 601 | 2,3,4,6-(CH₃)₄ | Cl | H |
| 602 | 2,3,5,6-(CH₃)₄ | Cl | H |
| 603 | 2,3,4,5,6-(CH₃)₅ | Cl | H |
| 604 | 2-C₂H₅ | Cl | H |
| 605 | 3-C₂H₅ | Cl | H |
| 606 | 4-C₂H₅ | Cl | H |
| 607 | 2,4-(C₂H₅)₅ | Cl | H |
| 608 | 2,6-(C₂H₅)₂ | Cl | H |
| 609 | 3,5-(C₂H₅)₂ | Cl | H |
| 610 | 2,4,6-(C₂H₅)₃ | Cl | H |
| 611 | 2-n-C₃H₇ | Cl | H |
| 612 | 3-n-C₃H₇ | Cl | H |
| 613 | 4-n-C₃H₇ | Cl | H |
| 614 | 2-i-C₃H₇ | Cl | H |
| 615 | 3-i-C₃H₇ | Cl | H |
| 616 | 4-i-C₃H₇ | Cl | H |
| 617 | 2,4-(i-C₃H₇)₂ | Cl | H |
| 618 | 2,6-(i-C₃H₇)₂ | Cl | H |
| 619 | 3,5-(i-C₃H₇)₂ | Cl | H |
| 620 | 2-s-C₄H₉ | Cl | H |
| 621 | 3-s-C₄H₉ | Cl | H |
| 622 | 4-s-C₄H₉ | Cl | H |
| 623 | 2-t-C₄H₉ | Cl | H |
| 624 | 3-t-C₄H₉ | Cl | H |
| 625 | 4-t-C₄H₉ | Cl | H |
| 626 | 4-n-C₉H₁₉ | Cl | H |
| 627 | 2-CH₃, 4-t-C₄H₉ | Cl | H |
| 628 | 2-CH₃, 6-t-C₄H₉ | Cl | H |
| 629 | 2-CH₃, 4-i-C₃H₇ | Cl | H |
| 630 | 2-CH₃, 5-i-C₃H₇ | Cl | H |
| 631 | 3-CH₃, 4-i-C₃H₇ | Cl | H |
| 632 | 2-cyclo-C₆H₁₁ | Cl | H |
| 633 | 3-cyclo-C₆H₁₁ | Cl | H |
| 634 | 4-cyclo-C₆H₁₁ | Cl | H |
| 635 | 2-Cl, 4-C₆H₅ | Cl | H |
| 636 | 2-Br, 4-C₆H₅ | Cl | H |
| 637 | 2-OCH₃ | Cl | H |
| 638 | 3-OCH₃ | Cl | H |
| 639 | 4-OCH₃ | Cl | H |
| 640 | 2-OC₂H₅ | Cl | H |
| 641 | 3-O-C₂H₅ | Cl | H |
| 642 | 4-O-C₂H₅ | Cl | H |
| 643 | 2-O-n-C₃H₇ | Cl | H |
| 644 | 3-O-n-C₃H₇ | Cl | H |
| 645 | 4-O-n-C₃H₇ | Cl | H |
| 646 | 2-O-i-C₃H₇ | Cl | H |
| 647 | 3-O-i-C₃H₇ | Cl | H |
| 648 | 4-O-i-C₃H₇ | Cl | H |
| 649 | 2-O-n-C₆H₁₃ | Cl | H |
| 650 | 3-O-n-C₆H₁₃ | Cl | H |
| 651 | 4-O-n-C₆H₁₃ | Cl | H |
| 652 | 2-O-CH₂C₆H₅ | Cl | H |
| 653 | 3-O-CH₂C₆H₅ | Cl | H |
| 654 | 4-O-CH₂C₆H₅ | Cl | H |
| 655 | 2-O-(CH₂)₃C₆H₅ | Cl | H |
| 656 | 4-O-(CH₂)₃C₆H₅ | Cl | H |
| 657 | 2,3-(OCH₃)₂ | Cl | H |
| 658 | 2,4-(OCH₃)₂ | Cl | H |
| 659 | 2,5-(OCH₃)₂ | Cl | H |
| 660 | 2,6-(OCH₃)₂ | Cl | H |
| 661 | 3,4-(OCH₃)₂ | Cl | H |
| 662 | 3,5-(OCH₃)₂ | Cl | H |
| 663 | 2-O-t-C₄H₉ | Cl | H |
| 664 | 3-O-t-C₄H₉ | Cl | H |
| 665 | 4-O-t-C₄H₉ | Cl | H |
| 666 | 3-(3'-Cl-C₆H₄) | Cl | H |
| 667 | 4-(4'-CH₃-C₆H₄) | Cl | H |
| 668 | 2-O-C₆H₅ | Cl | H |
| 669 | 3-O-C₆H₅ | Cl | H |
| 670 | 4-O-C₆H₅ | Cl | H |
| 671 | 2-O-(2'-F-C₆H₄) | Cl | H |
| 672 | 3-O-(3'-Cl-C₆H₄) | Cl | H |
| 673 | 4-O-(4'-CH₃-C₆H₄) | Cl | H |
| 674 | 2,3,6-(CH₃)₃, 4-F | Cl | H |
| 675 | 2,3,6-(CH₃)₃, 4-Cl | Cl | H |
| 676 | 2,3,6-(CH₃)₃, 4-Br | Cl | H |
| 677 | 2,4-(CH₃)₂, 6-F | Cl | H |
| 678 | 2,4-(CH₃)₂, 6-Cl | Cl | H |
| 679 | 2,4-(CH₃)₂, 6-Br | Cl | H |
| 680 | 2-i-C₃H₇, 4-Cl, 5-CH₃ | Cl | H |
| 681 | 2-Cl, 4-NO₂ | Cl | H |
| 682 | 2-NO₂, 4-Cl | Cl | H |
| 683 | 2-OCH₃, 5-NO₂ | Cl | H |
| 684 | 2,4-Cl₂,5-NO₂ | Cl | H |
| 685 | 2,4-Cl₂, 6-NO₂ | Cl | H |
| 686 | 2,6-Cl₂, 4-NO₂ | Cl | H |
| 687 | 2,6-Br₂, 4-NO₂ | Cl | H |
| 688 | 2,6-J₂, 4-NO₂ | Cl | H |
| 689 | 2-CH₃, 5-i-C₃H₇, 4-Cl | Cl | H |
| 690 | 2-CO₂CH₃ | Cl | H |
| 691 | 3-CO₂CH₃ | Cl | H |
| 692 | 4-CO₂CH₃ | Cl | H |
| 693 | 2-CH₂-OCH₃ | Cl | H |
| 694 | 3-CH₂-OCH₃ | Cl | H |
| 695 | 4-CH₂-OCH₃ | Cl | H |
| 696 | 2-Me-4-CH₃-CH(CH₃)-CO | Cl | H |
| 697 | 2-CH₃-4-(CH₃-C=NOCH₃) | Cl | H |
| 698 | 2-CH₃-4-(CH₃-C=NOC₂H₅) | Cl | H |
| 699 | 2-CH₃-4-(CH₃-C=NO-n-C₃H₇) | Cl | H |
| 700 | 2-CH₃-4-(CH₃-C=NO-i-C₃H₇) | Cl | H |
| 701 | 2,5-(CH₃)₂-4-(CH₃-C=NOCH₃) | Cl | H |
| 702 | 2,5-(CH₃)₂-4-(CH₃-C=NOC₂H₅) | Cl | H |
| 703 | 2,5-(CH₃-4-(CH₃-C=NO-n-C₃H₇) | Cl | H |
| 704 | 2,5-(CH₃)₂-4-(CH₃-C=NO-i-C₃H₇) | Cl | H |
| 705 | 2-C₆H₅ | Cl | H |
| 706 | 3-C₆H₅ | Cl | H |
| 707 | 4-C₆H₅ | Cl | H |
| 708 | 2-(2'-F-C₆H₄) | Cl | H |
| 709 | 2-CH₃, 5-Br | Cl | H |
| 710 | 2-CH₃, 6-Br | Cl | H |
| 711 | 2-Cl, 3-CH₃ | Cl | H |
| 712 | 2-Cl, 4-CH₃ | Cl | H |
| 713 | 2-Cl, 5-CH₃ | Cl | H |
| 714 | 2-F, 3-CH₃ | Cl | H |
| 715 | 2-F, 4-CH₃ | Cl | H |
| 716 | 2-F, 5-CH₃ | Cl | H |
| 717 | 2-Br, 3-CH₃ | Cl | H |
| 718 | 2-Br, 4-CH₃ | Cl | H |
| 719 | 2-Br, 5-CH₃ | Cl | H |
| 720 | 3-CH₃, 4-Cl | Cl | H |
| 721 | 3-CH₃, 5-Cl | Cl | H |
| 722 | 3-CH₃, 4-F | Cl | H |
| 723 | 3-CH₃, 5-F | Cl | H |
| 724 | 3-CH₃, 4-Br | Cl | H |
| 725 | 3-CH₃, 5-Br | Cl | H |
| 726 | 3-F, 4-CH₃ | Cl | H |
| 727 | 3-Cl, 4-CH₃ | Cl | H |
| 728 | 3-Br, 4-CH₃ | Cl | H |
| 729 | 2-Cl, 4,5-(CH₃)₂ | Cl | H |
| 730 | 2-Br, 4,5-(CH₃)₂ | Cl | H |
| 731 | 2-Cl, 3,5-(CH₃)₂ | Cl | H |
| 732 | 2-Br, 3,5-(CH₃)₂ | Cl | H |
| 733 | 2,6-Cl₂, 4-CH₃ | Cl | H |
| 734 | 2,6-F₂, 4-CH₃ | Cl | H |
| 735 | 2,6-Br₂, 4-CH₃ | Cl | H |
| 736 | 2,4-Br₂,6-CH₃ | Cl | H |
| 737 | 2,4-F₂, 6-CH₃ | Cl | H |
| 738 | 2,4-Br₂, 6-CH₃ | Cl | H |
| 739 | 2,6-(CH₃)₂, 4-F | Cl | H |
| 740 | 2,6-(CH₃)₂, 4-Cl | Cl | H |
| 741 | 2,6-(CH₃)₂, 4-Br | Cl | H |
| 742 | 3,5-(CH₃)₂, 4-F | Cl | H |
| 743 | 3,5-(CH₃)₂, 4-Cl | Cl | H |
| 744 | 3,5-(CH₃)₂, 4-Br | Cl | H |
| 745 | 2-CF₃ | Cl | H |
| 746 | 3-CF₃ | Cl | H |
| 747 | 4-CF₃ | Cl | H |
| 748 | 2-OCF₃ | Cl | H |
| 749 | 3-OCF₃ | Cl | H |
| 750 | 4-OCF₃ | Cl | H |
| 751 | 3-OCH₂CHF₂ | Cl | H |
| 752 | 2-NO₂ | Cl | H |
| 753 | 3-NO₂ | Cl | H |
| 754 | 4-NO₂ | Cl | H |
| 755 | 2-CN | Cl | H |
| 756 | 3-CN | Cl | H |
| 757 | 4-CN | Cl | H |
| 758 | 2-CH₃, 3-Cl | Cl | H |
| 759 | 2-CH₃, 4-Cl | Cl | H |
| 760 | 2-CH₃, 5-Cl | Cl | H |
| 761 | 2-CH₃, 6-Cl | Cl | H |
| 762 | 2-CH₃, 3-F | Cl | H |
| 763 | 2-CH₃, 4-F | Cl | H |
| 764 | 2-CH₃, 5-F | Cl | H |
| 765 | 2-CH₃, 6-F | Cl | H |
| 766 | 2-CH₃, 3-Br | Cl | H |
| 767 | 2-CH₃, 4-Br | Cl | H |
| 768 | 2-Pyridyl-2' | Cl | H |
| 769 | 3-Pyridyl-3' | Cl | H |
| 770 | 4-Pyridyl-4' | Cl | H |
| 771 | 2-CO-CH₃ | Cl | H |
| 772 | 3-CO-CH₃ | Cl | H |
| 773 | 4-CO-CH₃ | Cl | H |
| 774 | 2-C(=N-OCH₃)-CH₃ | Cl | H |
| 775 | 3-C(=N-OCH₃)-CH₃ | Cl | H |
| 776 | 4-C(=N-OCH₃)-CH₃ | Cl | H |
| 777 | 2-F | CN | H |
| 778 | 3-F | CN | H |
| 779 | 4-F | CN | H |
| 780 | 2,4-F₂ | CN | H |
| 781 | 2,4,6-F₃ | CN | H |
| 782 | 2,3,4,5,6-F₅ | CN | H |
| 783 | 2,3-F₂ | CN | H |
| 784 | 2-Cl | CN | H |
| 785 | 3-Cl | CN | H |
| 786 | 4-Cl | CN | H |
| 787 | 2,3-Cl₂ | CN | H |
| 788 | 2,4-Cl₂ | CN | H |
| 789 | 2,5-Cl₂ | CN | H |
| 790 | 2,6-Cl₂ | CN | H |
| 791 | 3,4-Cl₂ | CN | H |
| 792 | 3,5-Cl₂ | CN | H |
| 793 | 2,3,4-Cl₃ | CN | H |
| 794 | 2,3,5-Cl₃ | CN | H |
| 795 | 2,3,6-Cl₃ | CN | H |
| 796 | 2,4,5-Cl₃ | CN | H |
| 797 | 2,4,6-Cl₃ | CN | H |
| 798 | 3,4,5-Cl₃ | CN | H |
| 799 | 2,3,4,6-Cl₄ | CN | H |
| 800 | 2,3,5,6-Cl₄ | CN | H |
| 801 | 2,3,4,5,6-Cl₅ | CN | H |
| 802 | 2-Br | CN | H |
| 803 | 3-Br | CN | H |
| 804 | 4-Br | CN | H |
| 805 | 2,4-Br₂ | CN | H |
| 806 | 2,5-Br₂ | CN | H |
| 807 | 2,6-Br₂ | CN | H |
| 808 | 2,4,6-Br₃ | CN | H |
| 809 | 2,3,4,5,6-Br₅ | CN | H |
| 810 | 2-J | CN | H |
| 811 | 3-J | CN | H |
| 812 | 4-J | CN | H |
| 813 | 2,4-J₂ | CN | H |
| 814 | 2-Cl, 3-F | CN | H |
| 815 | 2-Cl, 4-F | CN | H |
| 816 | 2-Cl, 5-F | CN | H |
| 817 | 2-Cl, 6-F | CN | H |
| 818 | 2-Cl, 3-Br | CN | H |
| 819 | 2-Cl, 4-Br | CN | H |
| 820 | 2-Cl, 5-Br | CN | H |
| 821 | 2-Cl, 6-Br | CN | H |
| 822 | 2-Br, 3-Cl | CN | H |
| 823 | 2-Br, 4-Cl | CN | H |
| 824 | 2-Br, 5-Cl | CN | H |
| 825 | 2-Br, 3-F | CN | H |
| 826 | 2-Br, 4-F | CN | H |
| 827 | 2-Br, 5-F | CN | H |
| 828 | 2-Br, 6-F | CN | H |
| 829 | 2-F, 3-Cl | CN | H |
| 830 | 2-F, 4-Cl | CN | H |
| 831 | 2-F, 5-Cl | CN | H |
| 832 | 3-Cl, 4-F | CN | H |
| 833 | 3-Cl, 5-F | CN | H |
| 834 | 3-Cl, 4-Br | CN | H |
| 835 | 3-Cl, 5-Br | CN | H |
| 836 | 3-F, 4-Cl | CN | H |
| 837 | 3-F, 4-Br | CN | H |
| 838 | 3-Br, 4-Cl | CN | H |
| 839 | 3-Br, 4-F | CN | H |
| 840 | 2,6-Cl₂, 4-Br | CN | H |
| 841 | 2-CH₃ | CN | H |
| 842 | 3-CH₃ | CN | H |
| 843 | 4-CH₃ | CN | H |
| 844 | 2,3-(CH₃)₂ | CN | H |
| 845 | 2,4-(CH₃)₂ | CN | H |
| 846 | 2,5-(CH₃)₂ | CN | H |
| 847 | 2,6-(CH₃)₂ | CN | H |
| 848 | 3,4-(CH₃)₂ | CN | H |
| 849 | 3,5-(CH₃)₂ | CN | H |
| 850 | 2,3,5-(CH₃)₃ | CN | H |
| 851 | 2,3,4-(CH₃)₃ | CN | H |
| 852 | 2,3,6-(CH₃)₃ | CN | H |
| 853 | 2,4,5-(CH₃)₃ | CN | H |
| 854 | 2,4,6-(CH₃)₃ | CN | H |
| 855 | 3,4,5-(CH₃)₃ | CN | H |
| 856 | 2,3,4,6-(CH₃)₄ | CN | H |
| 857 | 2,3,5,6-(CH₃)₄ | CN | H |
| 858 | 2,3,4,5,6-(CH₃)₅ | CN | H |
| 859 | 2-C₂H₅ | CN | H |
| 860 | 3-C₂H₅ | CN | H |
| 861 | 4-C₂H₅ | CN | H |
| 862 | 2,4-(C₂H₅)₅ | CN | H |
| 863 | 2,6-(C₂H₅)₂ | CN | H |
| 864 | 3,5-(C₂H₅)₂ | CN | H |
| 865 | 2,4,6-(C₂H₅)₃ | CN | H |
| 866 | 2-n-C₃H₇ | CN | H |
| 867 | 3-n-C₃H₇ | CN | H |
| 868 | 4-n-C₃H₇ | CN | H |
| 869 | 2-i-C₃H₇ | CN | H |
| 870 | 3-i-C₃H₇ | CN | H |
| 871 | 4-i-C₃H₇ | CN | H |
| 872 | 2,4-(i-C₃H₇)₂ | CN | H |
| 873 | 2,6-(i-C₃H₇)₂ | CN | H |
| 874 | 3,5-(i-C₃H₇)₂ | CN | H |
| 875 | 2-s-C₄H₉ | CN | H |
| 876 | 3-s-C₄H₉ | CN | H |
| 877 | 4-s-C₄H₉ | CN | H |
| 878 | 2-t-C₄H₉ | CN | H |
| 879 | 3-t-C₄H₉ | CN | H |
| 880 | 4-t-C₄H₉ | CN | H |
| 881 | 4-n-C₉H₁₉ | CN | H |
| 882 | 2-CH₃, 4-t-C₄H₉ | CN | H |
| 883 | 2-CH₃, 6-t-C₄H₉ | CN | H |
| 884 | 2-CH₃, 4-i-C₃H₇ | CN | H |
| 885 | 2-CH₃, 5-i-C₃H₇ | CN | H |
| 886 | 3-CH₃, 4-i-C₃H₇ | CN | H |
| 887 | 2-cyclo-C₆H₁₁ | CN | H |
| 888 | 3-cyclo-C₆H₁₁ | CN | H |
| 889 | 4-cyclo-C₆H₁₁ | CN | H |
| 890 | 2-Cl, 4-C₆H₅ | CN | H |
| 891 | 2-Br, 4-C₆H₅ | CN | H |
| 892 | 2-OCH₃ | CN | H |
| 893 | 3-OCH₃ | CN | H |
| 894 | 4-OCH₃ | CN | H |
| 895 | 2-OC₂H₅ | CN | H |
| 896 | 3-O-C₂H₅ | CN | H |
| 897 | 4-O-C₂H₅ | CN | H |
| 898 | 2-O-n-C₃H₇ | CN | H |
| 899 | 3-O-n-C₃H₇ | CN | H |
| 900 | 4-O-n-C₃H₇ | CN | H |
| 901 | 2-O-i-C₃H₇ | CN | H |
| 902 | 3-O-i-C₃H₇ | CN | H |
| 903 | 4-O-i-C₃H₇ | CN | H |
| 904 | 2-O-n-C₆H₁₃ | CN | H |
| 905 | 3-O-n-C₆H₁₃ | CN | H |
| 906 | 4-O-n-C₆H₁₃ | CN | H |
| 907 | 2-O-CH₂C₆H₅ | CN | H |
| 908 | 3-O-CH₂C₆H₅ | CN | H |
| 909 | 4-O-CH₂C₆H₅ | CN | H |
| 910 | 2-O-(CH₂)₃C₆H₅ | CN | H |
| 911 | 4-O-(CH₂)₃C₆H₅ | CN | H |
| 912 | 2,3-(OCH₃)₂ | CN | H |
| 913 | 2,4-(OCH₃)₂ | CN | H |
| 914 | 2,5-(OCH₃)₂ | CN | H |
| 915 | 2,6-(OCH₃)₂ | CN | H |
| 916 | 3,4-(OCH₃)₂ | CN | H |
| 917 | 3,5-(OCH₃)₂ | CN | H |
| 918 | 2-O-t-C₄H₉ | CN | H |
| 919 | 3-O-t-C₄H₉ | CN | H |
| 920 | 4-O-t-C₄H₉ | CN | H |
| 921 | 3-(3'-Cl-C₆H₄) | CN | H |
| 922 | 4-(4'-CH₃-C₆H₄) | CN | H |
| 923 | 2-O-C₆H₅ | CN | H |
| 924 | 3-O-C₆H₅ | CN | H |
| 925 | 4-O-C₆H₅ | CN | H |
| 926 | 2-O-(2'-F-C₆H₄) | CN | H |
| 927 | 3-O-(3'-Cl-C₆H₄) | CN | H |
| 928 | 4-O-(4'-CH₃-C₆H₄) | CN | H |
| 929 | 2,3,6-(CH₃)₃, 4-F | CN | H |
| 930 | 2,3,6-(CH₃)₃, 4-Cl | CN | H |
| 931 | 2,3,6-(CH₃)₃, 4-Br | CN | H |
| 932 | 2,4-(CH₃)₂, 6-F | CN | H |
| 933 | 2,4-(CH₃)₂, 6-Cl | CN | H |
| 934 | 2,4-(CH₃)₂, 6-Br | CN | H |
| 935 | 2-i-C₃H₇, 4-Cl, 5-CH₃ | CN | H |
| 936 | 2-Cl, 4-NO₂ | CN | H |
| 937 | 2-NO₂, 4-Cl | CN | H |
| 938 | 2-OCH₃, 5-NO₂ | CN | H |
| 939 | 2,4-Cl₂, 5-NO₂ | CN | H |
| 940 | 2,4-Cl₂, 6-NO₂ | CN | H |
| 941 | 2,6-Cl₂, 4-NO₂ | CN | H |
| 942 | 2,6-Br₂, 4-NO₂ | CN | H |
| 943 | 2,6-J₂,4-NO₂ | CN | H |
| 944 | 2-CH₃, 5-i-C₃H₇, 4-Cl | CN | H |
| 945 | 2-CO₂CH₃ | CN | H |
| 946 | 3-CO₂CH₃ | CN | H |
| 947 | 4-CO₂CH₃ | CN | H |
| 948 | 2-CH₂-OCH₃ | CN | H |
| 949 | 3-CH₂-OCH₃ | CN | H |
| 950 | 4-CH₂-OCH₃ | CN | H |
| 951 | 2-Me-4-CH₃-CH(CH₃)-CO | CN | H |
| 952 | 2-CH₃-4-(CH₃-C=NOCH₃) | CN | H |
| 953 | 2-CH₃-4-(CH₃-C=NOC₂H₅) | CN | H |
| 954 | 2-CH₃-4-(CH₃-C=NO-n-C₃H₇) | CN | H |
| 955 | 2-CH₃-4-(CH₃-C=NO-i-C₃H₇) | CN | H |
| 956 | 2,5-(CH₃)₂-4-(CH₃-C=NOCH₃) | CN | H |
| 957 | 2,5-(CH₃)₂-4-(CH₃-C=NOC₂H₅) | CN | H |
| 958 | 2,5-(CH₃-4-(CH₃-C=NO-n-C₃H₇) | CN | H |
| 959 | 2,5-(CH₃)₂-4-(CH₃-C=NO-i-C₃H₇) | CN | H |
| 960 | 2-C₆H₅ | CN | H |
| 961 | 3-C₆H₅ | CN | H |
| 962 | 4-C₆H₅ | CN | H |
| 963 | 2-(2'-F-C₆H₄) | CN | H |
| 964 | 2-CH₃, 5-Br | CN | H |
| 965 | 2-CH₃, 6-Br | CN | H |
| 966 | 2-Cl, 3-CH₃ | CN | H |
| 967 | 2-Cl, 4-CH₃ | CN | H |
| 968 | 2-Cl, 5-CH₃ | CN | H |
| 969 | 2-F, 3-CH₃ | CN | H |
| 970 | 2-F, 4-CH₃ | CN | H |
| 971 | 2-F, 5-CH₃ | CN | H |
| 972 | 2-Br, 3-CH₃ | CN | H |
| 973 | 2-Br, 4-CH₃ | CN | H |
| 974 | 2-Br, 5-CH₃ | CN | H |
| 975 | 3-CH₃, 4-Cl | CN | H |
| 976 | 3-CH₃, 5-Cl | CN | H |
| 977 | 3-CH₃, 4-F | CN | H |
| 978 | 3-CH₃, 5-F | CN | H |
| 979 | 3-CH₃, 4-Br | CN | H |
| 980 | 3-CH₃, 5-Br | CN | H |
| 981 | 3-F, 4-CH₃ | CN | H |
| 982 | 3-Cl, 4-CH₃ | CN | H |
| 983 | 3-Br, 4-CH₃ | CN | H |
| 984 | 2-Cl, 4,5-(CH₃)₂ | CN | H |
| 985 | 2-Br, 4,5-(CH₃)₂ | CN | H |
| 986 | 2-Cl, 3,5-(CH₃)₂ | CN | H |
| 987 | 2-Br, 3,5-(CH₃)₂ | CN | H |
| 988 | 2,6-C12, 4-CH₃ | CN | H |
| 989 | 2,6-F₂, 4-CH₃ | CN | H |
| 990 | 2,6-Br₂, 4-CH₃ | CN | H |
| 991 | 2,4-Br₂, 6-CH₃ | CN | H |
| 992 | 2,4-F₂, 6-CH₃ | CN | H |
| 993 | 2,4-Br₂, 6-CH₃ | CN | H |
| 994 | 2,6-(CH₃)₂, 4-F | CN | H |
| 995 | 2,6-(CH₃)₂, 4-Cl | CN | H |
| 996 | 2,6-(CH₃)₂, 4-Br | CN | H |
| 997 | 3,5-(CH₃)₂, 4-F | CN | H |
| 998 | 3,5-(CH₃)₂, 4-Cl | CN | H |
| 999 | 3,5-(CH₃)₂, 4-Br | CN | H |
| 1000 | 2-CF₃ | CN | H |
| 1001 | 3-CF₃ | CN | H |
| 1002 | 4-CF₃ | CN | H |
| 1003 | 2-OCF₃ | CN | H |
| 1004 | 3-OCF₃ | CN | H |
| 1005 | 4-OCF₃ | CN | H |
| 1006 | 3-OCH₂CHF₂ | CN | H |
| 1007 | 2-NO₂ | CN | H |
| 1008 | 3-NO₂ | CN | H |
| 1009 | 4-NO₂ | CN | H |
| 1010 | 2-CN | CN | H |
| 1011 | 3-CN | CN | H |
| 1012 | 4-CN | CN | H |
| 1013 | 2-CH₃, 3-Cl | CN | H |
| 1014 | 2-CH₃, 4-Cl | CN | H |
| 1015 | 2-CH₃, 5-Cl | CN | H |
| 1016 | 2-CH₃, 6-Cl | CN | H |
| 1017 | 2-CH₃, 3-F | CN | H |
| 1018 | 2-CH₃, 4-F | CN | H |
| 1019 | 2-CH₃, 5-F | CN | H |
| 1020 | 2-CH₃, 6-F | CN | H |
| 1021 | 2-CH₃, 3-Br | CN | H |
| 1022 | 2-CH₃, 4-Br | CN | H |
| 1023 | 2-Pyridyl-2' | CN | H |
| 1024 | 3-Pyridyl-3' | CN | H |
| 1025 | 4-Pyridyl-4' | CN | H |
| 1026 | 2-CO-CH₃ | CN | H |
| 1027 | 3-CO-CH₃ | CN | H |
| 1028 | 4-CO-CH₃ | CN | H |
| 1029 | 2-C(=N-OCH₃)-CH₃ | CN | H |
| 1030 | 3-C(=N-OCH₃)-CH₃ | CN | H |
| 1031 | 4-C(=N-OCH₃)-CH₃ | CN | H |
| 1032 | 2-F | OCH₃ | H |
| 1033 | 3-F | OCH₃ | H |
| 1034 | 4-F | OCH₃ | H |
| 1035 | 2,4-F₂ | OCH₃ | H |
| 1036 | 2,4,6-F₃ | OCH₃ | H |
| 1037 | 2,3,4,5,6-F₅ | OCH₃ | H |
| 1038 | 2,3-F₂ | OCH₃ | H |
| 1039 | 2-Cl | OCH₃ | H |
| 1040 | 3-Cl | OCH₃ | H |
| 1041 | 4-Cl | OCH₃ | H |
| 1042 | 2,3-Cl₂ | OCH₃ | H |
| 1043 | 2,4-Cl₂ | OCH₃ | H |
| 1044 | 2,5-Cl₂ | OCH₃ | H |
| 1045 | 2,6-Cl₂ | OCH₃ | H |
| 1046 | 3,4-Cl₂ | OCH₃ | H |
| 1047 | 3,5-Cl₂ | OCH₃ | H |
| 1048 | 2,3,4-Cl₃ | OCH₃ | H |
| 1049 | 2,3,5-Cl₃ | OCH₃ | H |
| 1050 | 2,3,6-Cl₃ | OCH₃ | H |
| 1051 | 2,4,5-Cl₃ | OCH₃ | H |
| 1052 | 2,4,6-Cl₃ | OCH₃ | H |
| 1053 | 3,4,5-Cl₃ | OCH₃ | H |
| 1054 | 2,3,4,6-Cl₄ | OCH₃ | H |
| 1055 | 2,3,5,6-Cl₄ | OCH₃ | H |
| 1056 | 2,3,4,5,6-Cl₅ | OCH₃ | H |
| 1057 | 2-Br | OCH₃ | H |
| 1058 | 3-Br | OCH₃ | H |
| 1059 | 4-Br | OCH₃ | H |
| 1060 | 2,4-Br₂ | OCH₃ | H |
| 1061 | 2,5-Br₂ | OCH₃ | H |
| 1062 | 2,6-Br₂ | OCH₃ | H |
| 1063 | 2,4,6-Br₃ | OCH₃ | H |
| 1064 | 2,3,4,5,6-Br₅ | OCH₃ | H |
| 1065 | 2-J | OCH₃ | H |
| 1066 | 3-J | OCH₃ | H |
| 1067 | 4-J | OCH₃ | H |
| 106 | 2,4-J₂ | OCH₃ | H |
| 1069 | 2-Cl, 3-F | OCH₃ | H |
| 1070 | 2-Cl, 4-F | OCH₃ | H |
| 1071 | 2-Cl, 5-F | OCH₃ | H |
| 1072 | 2-Cl, 6-F | OCH₃ | H |
| 1073 | 2-Cl, 3-Br | OCH₃ | H |
| 1074 | 2-Cl, 4-Br | OCH₃ | H |
| 1075 | 2-Cl, 5-Br | OCH₃ | H |
| 1076 | 2-Cl, 6-Br | OCH₃ | H |
| 1077 | 2-Br, 3-Cl | OCH₃ | H |
| 1078 | 2-Br, 4-Cl | OCH₃ | H |
| 1079 | 2-Br, 5-Cl | OCH₃ | H |
| 1080 | 2-Br, 3-F | OCH₃ | H |
| 1081 | 2-Br, 4-F | OCH₃ | H |
| 1082 | 2-Br, 5-F | OCH₃ | H |
| 1083 | 2-Br, 6-F | OCH₃ | H |
| 1084 | 2-F, 3-Cl | OCH₃ | H |
| 1085 | 2-F, 4-Cl | OCH₃ | H |
| 1086 | 2-F, 5-Cl | OCH₃ | H |
| 1087 | 3-Cl, 4-F | OCH₃ | H |
| 1088 | 3-Cl, 5-F | OCH₃ | H |
| 1089 | 3-Cl, 4-Br | OCH₃ | H |
| 1090 | 3-Cl, 5-Br | OCH₃ | H |
| 1091 | 3-F, 4-Cl | OCH₃ | H |
| 1092 | 3-F, 4-Br | OCH₃ | H |
| 1093 | 3-Br, 4-Cl | OCH₃ | H |
| 1094 | 3-Br, 4-F | OCH₃ | H |
| 1095 | 2,6-Cl₂, 4-Br | OCH₃ | H |
| 1096 | 2-CH₃ | OCH₃ | H |
| 1097 | 3-CH₃ | OCH₃ | H |
| 1098 | 4-CH₃ | OCH₃ | H |
| 1099 | 2,3-(CH₃)₂ | OCH₃ | H |
| 1100 | 2,4-(CH₃)₂ | OCH₃ | H |
| 1101 | 2,5-(CH₃)₂ | OCH₃ | H |
| 1102 | 2,6-(CH₃)₂ | OCH₃ | H |
| 1103 | 3,4-(CH₃)₂ | OCH₃ | H |
| 1104 | 3,5-(CH₃)₂ | OCH₃ | H |
| 1105 | 2,3,5-(CH₃)₃ | OCH₃ | H |
| 1106 | 2,3,4-(CH₃)₃ | OCH₃ | H |
| 1107 | 2,3,6-(CH₃)₃ | OCH₃ | H |
| 1108 | 2,4,5-(CH₃)₃ | OCH₃ | H |
| 1109 | 2,4,6-(CH₃)₃ | OCH₃ | H |
| 1110 | 3,4,5-(CH₃)₃ | OCH₃ | H |
| 1111 | 2,3,4,6-(CH₃)₄ | OCH₃ | H |
| 1112 | 2,3,5,6-(CH₃)₄ | OCH₃ | H |
| 1113 | 2,3,4,5,6-(CH₃)₅ | OCH₃ | H |
| 1114 | 2-C₂H₅ | OCH₃ | H |
| 1115 | 3-C₂H₅ | OCH₃ | H |
| 1116 | 4-C₂H₅ | OCH₃ | H |
| 1117 | 2,4-(C₂H₅)₅ | OCH₃ | H |
| 1118 | 2,6-(C₂H₅)₂ | OCH₃ | H |
| 1119 | 3,5-(C₂H₅)₂ | OCH₃ | H |
| 1120 | 2,4,6-(C₂H₅)₃ | OCH₃ | H |
| 1121 | 2-n-C₃H₇ | OCH₃ | H |
| 1122 | 3-n-C₃H₇ | OCH₃ | H |
| 1123 | 4-n-C₃H₇ | OCH₃ | H |
| 1124 | 2-i-C₃H₇ | OCH₃ | H |
| 1125 | 3-i-C₃H₇ | OCH₃ | H |
| 1126 | 4-i-C₃H₇ | OCH₃ | H |
| 1127 | 2,4-(i-C₃H₇)₂ | OCH₃ | H |
| 1128 | 2,6-(i-C₃H₇)₂ | OCH₃ | H |
| 1129 | 3,5-(i-C₃H₇)₂ | OCH₃ | H |
| 1130 | 2-s-C₄H₉ | OCH₃ | H |
| 1131 | 3-s-C₄H₉ | OCH₃ | H |
| 1132 | 4-s-C₄H₉ | OCH₃ | H |
| 1133 | 2-t-C₄H₉ | OCH₃ | H |
| 1134 | 3-t-C₄H₉ | OCH₃ | H |
| 1135 | 4-t-C₄H₉ | OCH₃ | H |
| 1136 | 4-n-C₉H₁₉ | OCH₃ | H |
| 1137 | 2-CH₃, 4-t-C₄H₉ | OCH₃ | H |
| 1138 | 2-CH₃, 6-t-C₄H₉ | OCH₃ | H |
| 1139 | 2-CH₃, 4-i-C₃H₇ | OCH₃ | H |
| 1140 | 2-CH₃, 5-i-C₃H₇ | OCH₃ | H |
| 1141 | 3-CH₃, 4-i-C₃H₇ | OCH₃ | H |
| 1142 | 2-cyclo-C₆H₁₁ | OCH₃ | H |
| 1143 | 3-cyclo-C₆H₁₁ | OCH₃ | H |
| 1144 | 4-cyclo-C₆H₁₁ | OCH₃ | H |
| 1145 | 2-Cl, 4-C₆H₅ | OCH₃ | H |
| 1146 | 2-Br, 4-C₆H₅ | OCH₃ | H |
| 1147 | 2-OCH₃ | OCH₃ | H |
| 1148 | 3-OCH₃ | OCH₃ | H |
| 1149 | 4-OCH₃ | OCH₃ | H |
| 1150 | 2-OC₂H₅ | OCH₃ | H |
| 1151 | 3-O-C₂H₅ | OCH₃ | H |
| 1152 | 4-O-C₂H₅ | OCH₃ | H |
| 1153 | 2-O-n-C₃H₇ | OCH₃ | H |
| 1154 | 3-O-n-C₃H₇ | OCH₃ | H |
| 1155 | 4-O-n-C₃H₇ | OCH₃ | H |
| 1156 | 2-O-i-C₃H₇ | OCH₃ | H |
| 1157 | 3-O-i-C₃H₇ | OCH₃ | H |
| 1158 | 4-O-i-C₃H₇ | OCH₃ | H |
| 1159 | 2-O-n-C₆H₁₃ | OCH₃ | H |
| 1160 | 3-O-n-C₆H₁₃ | OCH₃ | H |
| 1161 | 4-O-n-C₆H₁₃ | OCH₃ | H |
| 1162 | 2-O-CH₂C₆H₅ | OCH₃ | H |
| 1163 | 3-O-CH₂C₆H₅ | OCH₃ | H |
| 1164 | 4-O-CH₂C₆H₅ | OCH₃ | H |
| 1165 | 2-O-(CH₂)₃C₆H₅ | OCH₃ | H |
| 1166 | 4-O-(CH₂)₃C₆H₅ | OCH₃ | H |
| 1167 | 2,3-(OCH₃)₂ | OCH₃ | H |
| 1168 | 2,4-(OCH₃)₂ | OCH₃ | H |
| 1169 | 2,5-(OCH₃)₂ | OCH₃ | H |
| 1170 | 2,6-(OCH₃)₂ | OCH₃ | H |
| 1171 | 3,4-(OCH₃)₂ | OCH₃ | H |
| 1172 | 3,5-(OCH₃)₂ | OCH₃ | H |
| 1173 | 2-O-t-C₄H₉ | OCH₃ | H |
| 1174 | 3-O-t-C₄H₉ | OCH₃ | H |
| 1175 | 4-O-t-C₄H₉ | OCH₃ | H |
| 1176 | 3-(3'-Cl-C₆H₄) | OCH₃ | H |
| 1177 | 4-(4'-CH₃-C₆H₄) | OCH₃ | H |
| 1178 | 2-O-C₆H₅ | OCH₃ | H |
| 1179 | 3-O-C₆H₅ | OCH₃ | H |
| 1180 | 4-O-C₆H₅ | OCH₃ | H |
| 1181 | 2-O-(2'-F-C₆H₄) | OCH₃ | H |
| 1182 | 3-O-(3'-Cl-C₆H₄) | OCH₃ | H |
| 1183 | 4-O-(4'-CH₃-C₆H₄) | OCH₃ | H |
| 1184 | 2,3,6-(CH₃)₃, 4-F | OCH₃ | H |
| 1185 | 2,3,6-(CH₃)₃, 4-Cl | OCH₃ | H |
| 1186 | 2,3,6-(CH₃)₃, 4-Br | OCH₃ | H |
| 1187 | 2,4-(CH₃)₂, 6-F | OCH₃ | H |
| 1188 | 2,4-(CH₃)₂, 6-Cl | OCH₃ | H |
| 1189 | 2,4-(CH₃)₂, 6-Br | OCH₃ | H |
| 1190 | 2-i-C₃H₇, 4-Cl, 5-CH₃ | OCH₃ | H |
| 1191 | 2-Cl, 4-NO₂ | OCH₃ | H |
| 1192 | 2-NO₂, 4-Cl | OCH₃ | H |
| 1193 | 2-OCH₃, 5-NO₂ | OCH₃ | H |
| 1194 | 2,4-Cl₂, 5-NO₂ | OCH₃ | H |
| 1195 | 2,4-Cl₂, 6-NO₂ | OCH₃ | H |
| 1196 | 2,6-Cl₂, 4-NO₂ | OCH₃ | H |
| 1197 | 2,6-Br₂, 4-NO₂ | OCH₃ | H |
| 1198 | 2,6-J₂,4-NO₂ | OCH₃ | H |
| 1199 | 2-CH₃, 5-i-C₃H₇, 4-Cl | OCH₃ | H |
| 1200 | 2-CO₂CH₃ | OCH₃ | H |
| 1201 | 3-CO₂CH₃ | OCH₃ | H |
| 1202 | 4-CO₂CH₃ | OCH₃ | H |
| 1203 | 2-CH₂-OCH₃ | OCH₃ | H |
| 1204 | 3-CH₂-OCH₃ | OCH₃ | H |
| 1205 | 4-CH₂-OCH₃ | OCH₃ | H |
| 1206 | 2-Me-4-CH₃-CH(CH₃)-CO | OCH₃ | H |
| 1207 | 2-CH₃-4-(CH₃-C=NOCH₃) | OCH₃ | H |
| 1208 | 2-CH₃-4-(CH₃-C=NOC₂H₅) | OCH₃ | H |
| 1209 | 2-CH₃-4-(CH₃-C=NO-n-C₃H₇) | OCH₃ | H |
| 1210 | 2-CH₃-4-(CH₃-C=NO-i-C₃H₇) | OCH₃ | H |
| 1211 | 2,5-(CH₃)₂-4-(CH₃-C=NOCH₃) | OCH₃ | H |
| 1212 | 2,5-(CH₃)₂-4-(CH₃-C=NOC₂H₅) | OCH₃ | H |
| 1213 | 2,5-(CH₃-4-(CH₃-C=NO-n-C₃H₇) | OCH₃ | H |
| 1214 | 2,5-(CH₃)₂-4-(CH₃-C=NO-i-C₃H₇) | OCH₃ | H |
| 1215 | 2-C₆H₅ | OCH₃ | H |
| 1216 | 3-C₆H₅ | OCH₃ | H |
| 1217 | 4-C₆H₅ | OCH₃ | H |
| 1218 | 2-(2'-F-C₆H₄) | OCH₃ | H |
| 1219 | 2-CH₃, 5-Br | OCH₃ | H |
| 1220 | 2-CH₃, 6-Br | OCH₃ | H |
| 1221 | 2-Cl, 3-CH₃ | OCH₃ | H |
| 1222 | 2-Cl, 4-CH₃ | OCH₃ | H |
| 1223 | 2-Cl, 5-CH₃ | OCH₃ | H |
| 1224 | 2-F, 3-CH₃ | OCH₃ | H |
| 1225 | 2-F, 4-CH₃ | OCH₃ | H |
| 1226 | 2-F, 5-CH₃ | OCH₃ | H |
| 1227 | 2-Br, 3-CH₃ | OCH₃ | H |
| 1228 | 2-Br, 4-CH₃ | OCH₃ | H |
| 1229 | 2-Br, 5-CH₃ | OCH₃ | H |
| 1230 | 3-CH₃, 4-Cl | OCH₃ | H |
| 1231 | 3-CH₃, 5-Cl | OCH₃ | H |
| 1232 | 3-CH₃, 4-F | OCH₃ | H |
| 1233 | 3-CH₃, 5-F | OCH₃ | H |
| 1234 | 3-CH₃, 4-Br | OCH₃ | H |
| 1235 | 3-CH₃, 5-Br | OCH₃ | H |
| 1236 | 3-F, 4-CH₃ | OCH₃ | H |
| 1237 | 3-Cl, 4-CH₃ | OCH₃ | H |
| 1238 | 3-Br, 4-CH₃ | OCH₃ | H |
| 1239 | 2-Cl, 4,5-(CH₃)₂ | OCH₃ | H |
| 1240 | 2-Br, 4,5-(CH₃)₂ | OCH₃ | H |
| 1241 | 2-Cl, 3,5-(CH₃)₂ | OCH₃ | H |
| 1242 | 2-Br, 3,5-(CH₃)₂ | OCH₃ | H |
| 1243 | 2,6-Cl₂, 4-CH₃ | OCH₃ | H |
| 1244 | 2,6-F₂, 4-CH₃ | OCH₃ | H |
| 1245 | 2,6-Br₂, 4-CH₃ | OCH₃ | H |
| 1246 | 2,4-Br₂, 6-CH₃ | OCH₃ | H |
| 1247 | 2,4-F₂, 6-CH₃ | OCH₃ | H |
| 1248 | 2,4-Br₂,6-CH₃ | OCH₃ | H |
| 1249 | 2,6-(CH₃)₂, 4-F | OCH₃ | H |
| 1250 | 2,6-(CH₃)₂, 4-Cl | OCH₃ | H |
| 1251 | 2,6-(CH₃)₂, 4-Br | OCH₃ | H |
| 1252 | 3,5-(CH₃)₂, 4-F | OCH₃ | H |
| 1253 | 3,5-(CH₃)₂, 4-Cl | OCH₃ | H |
| 1254 | 3,5-(CH₃)₂, 4-Br | OCH₃ | H |
| 1255 | 2-CF₃ | OCH₃ | H |
| 1256 | 3-CF₃ | OCH₃ | H |
| 1257 | 4-CF₃ | OCH₃ | H |
| 1258 | 2-OCF₃ | OCH₃ | H |
| 1259 | 3-OCF₃ | OCH₃ | H |
| 1260 | 4-OCF₃ | OCH₃ | H |
| 1261 | 3-OCH₂CHF₂ | OCH₃ | H |
| 1262 | 2-NO₂ | OCH₃ | H |
| 1263 | 3-NO₂ | OCH₃ | H |
| 1264 | 4-NO₂ | OCH₃ | H |
| 1265 | 2-CN | OCH₃ | H |
| 1266 | 3-CN | OCH₃ | H |
| 1267 | 4-CN | OCH₃ | H |
| 1268 | 2-CH₃, 3-Cl | OCH₃ | H |
| 1269 | 2-CH₃, 4-Cl | OCH₃ | H |
| 1270 | 2-CH₃, 5-Cl | OCH₃ | H |
| 1271 | 2-CH₃, 6-Cl | OCH₃ | H |
| 1272 | 2-CH₃, 3-F | OCH₃ | H |
| 1273 | 2-CH₃, 4-F | OCH₃ | H |
| 1274 | 2-CH₃, 5-F | OCH₃ | H |
| 1275 | 2-CH₃, 6-F | OCH₃ | H |
| 1276 | 2-CH₃, 3-Br | OCH₃ | H |
| 1277 | 2-CH₃, 4-Br | OCH₃ | H |
| 1278 | 2-Pyridyl-2' | OCH₃ | H |
| 1279 | 3-Pyridyl-3' | OCH₃ | H |
| 1280 | 4-Pyridyl-4' | OCH₃ | H |
| 1281 | 2-CO-CH₃ | OCH₃ | H |
| 1282 | 3-CO-CH₃ | OCH₃ | H |
| 1283 | 4-CO-CH₃ | OCH₃ | H |
| 1284 | 2-C(=N-OCH₃)-CH₃ | OCH₃ | H |
| 1285 | 3-C(=N-OCH₃)-CH₃ | OCH₃ | H |
| 1286 | 4-C(=N-OCH₃)-CH₃ | OCH₃ | H |
| 1287 | 2-F | CH₃ | 5-CH₃ |
| 1288 | 3-F | CH₃ | 5-CH₃ |
| 1289 | 4-F | CH₃ | 5-CH₃ |
| 1290 | 2,4-F₂ | CH₃ | 5-CH₃ |
| 1291 | 2,4,6-F₃ | CH₃ | 5-CH₃ |
| 1292 | 2,3,4,5,6-F₅ | CH₃ | 5-CH₃ |
| 1293 | 2,3-F₂ | CH₃ | 5-CH₃ |
| 1294 | 2-Cl | CH₃ | 5-CH₃ |
| 1295 | 3-Cl | CH₃ | 5-CH₃ |
| 1296 | 4-Cl | CH₃ | 5-CH₃ |
| 1297 | 2,3-Cl₂ | CH₃ | 5-CH₃ |
| 1298 | 2,4-Cl₂ | CH₃ | 5-CH₃ |
| 1299 | 2,5-Cl₂ | CH₃ | 5-CH₃ |
| 1300 | 2,6-Cl₂ | CH₃ | 5-CH₃ |
| 1301 | 3,4-Cl₂ | CH₃ | 5-CH₃ |
| 1302 | 3,5-Cl₂ | CH₃ | 5-CH₃ |
| 1303 | 2,3,4-Cl₃ | CH₃ | 5-CH₃ |
| 1304 | 2,3,5-Cl₃ | CH₃ | 5-CH₃ |
| 1305 | 2,3,6-Cl₃ | CH₃ | 5-CH₃ |
| 1306 | 2,4,5-Cl₃ | CH₃ | 5-CH₃ |
| 1307 | 2,4,6-Cl₃ | CH₃ | 5-CH₃ |
| 1308 | 3,4,5-Cl₃ | CH₃ | 5-CH₃ |
| 1309 | 2,3,4,6-Cl₄ | CH₃ | 5-CH₃ |
| 1310 | 2,3,5,6-Cl₄ | CH₃ | 5-CH₃ |
| 1311 | 2,3,4,5,6-Cl₅ | CH₃ | 5-CH₃ |
| 1312 | 2-Br | CH₃ | 5-CH₃ |
| 1313 | 3-Br | CH₃ | 5-CH₃ |
| 1314 | 4-Br | CH₃ | 5-CH₃ |
| 1315 | 2,4-Br₂ | CH₃ | 5-CH₃ |
| 1316 | 2,5-Br₂ | CH₃ | 5-CH₃ |
| 1317 | 2,6-Br₂ | CH₃ | 5-CH₃ |
| 1318 | 2,4,6-Br₃ | CH₃ | 5-CH₃ |
| 1319 | 2,3,4,5,6-Br₅ | CH₃ | 5-CH₃ |
| 1320 | 2-J | CH₃ | 5-CH₃ |
| 1321 | 3-J | CH₃ | 5-CH₃ |
| 1322 | 4-J | CH₃ | 5-CH₃ |
| 1323 | 2,4-J₂ | CH₃ | 5-CH₃ |
| 1324 | 2-Cl, 3-F | CH₃ | 5-CH₃ |
| 1325 | 2-Cl, 4-F | CH₃ | 5-CH₃ |
| 1326 | 2-Cl, 5-F | CH₃ | 5-CH₃ |
| 1327 | 2-Cl, 6-F | CH₃ | 5-CH₃ |
| 1328 | 2-Cl, 3-Br | CH₃ | 5-CH₃ |
| 1329 | 2-Cl, 4-Br | CH₃ | 5-CH₃ |
| 1330 | 2-Cl, 5-Br | CH₃ | 5-CH₃ |
| 1331 | 2-Cl, 6-Br | CH₃ | 5-CH₃ |
| 1332 | 2-Br, 3-Cl | CH₃ | 5-CH₃ |
| 1333 | 2-Br, 4-Cl | CH₃ | 5-CH₃ |
| 1334 | 2-Br, 5-Cl | CH₃ | 5-CH₃ |
| 1335 | 2-Br, 3-F | CH₃ | 5-CH₃ |
| 1336 | 2-Br, 4-F | CH₃ | 5-CH₃ |
| 1337 | 2-Br, 5-F | CH₃ | 5-CH₃ |
| 1338 | 2-Br, 6-F | CH₃ | 5-CH₃ |
| 1339 | 2-F, 3-Cl | CH₃ | 5-CH₃ |
| 1340 | 2-F, 4-Cl | CH₃ | 5-CH₃ |
| 1341 | 2-F, 5-Cl | CH₃ | 5-CH₃ |
| 1342 | 3-Cl, 4-F | CH₃ | 5-CH₃ |
| 1343 | 3-Cl, 5-F | CH₃ | 5-CH₃ |
| 1344 | 3-Cl, 4-Br | CH₃ | 5-CH₃ |
| 1345 | 3-Cl, 5-Br | CH₃ | 5-CH₃ |
| 1346 | 3-F, 4-Cl | CH₃ | 5-CH₃ |
| 1347 | 3-F, 4-Br | CH₃ | 5-CH₃ |
| 1348 | 3-Br, 4-Cl | CH₃ | 5-CH₃ |
| 1349 | 3-Br, 4-F | CH₃ | 5-CH₃ |
| 1350 | 2,6-Cl₂, 4-Br | CH₃ | 5-CH₃ |
| 1351 | 2-CH₃ | CH₃ | 5-CH₃ |
| 1352 | 3-CH₃ | CH₃ | 5-CH₃ |
| 1353 | 4-CH₃ | CH₃ | 5-CH₃ |
| 1354 | 2,3-(CH₃)₂ | CH₃ | 5-CH₃ |
| 1355 | 2,4-(CH₃)₂ | CH₃ | 5-CH₃ |
| 1356 | 2,5-(CH₃)₂ | CH₃ | 5-CH₃ |
| 1357 | 2,6-(CH₃)₂ | CH₃ | 5-CH₃ |
| 1358 | 3,4-(CH₃)₂ | CH₃ | 5-CH₃ |
| 1359 | 3,5-(CH₃)₂ | CH₃ | 5-CH₃ |
| 1360 | 2,3,5-(CH₃)₃ | CH₃ | 5-CH₃ |
| 1361 | 2,3,4-(CH₃)₃ | CH₃ | 5-CH₃ |
| 1362 | 2,3,6-(CH₃)₃ | CH₃ | 5-CH₃ |
| 1363 | 2,4,5-(CH₃)₃ | CH₃ | 5-CH₃ |
| 1364 | 2,4,6-(CH₃)₃ | CH₃ | 5-CH₃ |
| 1365 | 3,4,5-(CH₃)₃ | CH₃ | 5-CH₃ |
| 1366 | 2,3,4,6-(CH₃)₄ | CH₃ | 5-CH₃ |
| 1367 | 2,3,5,6-(CH₃)₄ | CH₃ | 5-CH₃ |
| 1368 | 2,3,4,5,6-(CH₃)₅ | CH₃ | 5-CH₃ |
| 1369 | 2-C₂H₅ | CH₃ | 5-CH₃ |
| 1370 | 3-C₂H₅ | CH₃ | 5-CH₃ |
| 1371 | 4-C₂H₅ | CH₃ | 5-CH₃ |
| 1372 | 2,4-(C₂H₅)₅ | CH₃ | 5-CH₃ |
| 1373 | 2,6-(C₂H₅)₂ | CH₃ | 5-CH₃ |
| 1374 | 3,5-(C₂H₅)₂ | CH₃ | 5-CH |
| 1375 | 2,4,6-(C₂H₅)₃ | CH₃ | 5-CH₃ |
| 1376 | 2-n-C₃H₇ | CH₃ | 5-CH₃ |
| 1377 | 3-n-C₃H₇ | CH₃ | 5-CH₃ |
| 1378 | 4-n-C₃H₇ | CH₃ | 5-CH₃ |
| 1379 | 2-i-C₃H₇ | CH₃ | 5-CH₃ |
| 1380 | 3-i-C₃H₇ | CH₃ | 5-CH₃ |
| 1381 | 4-i-C₃H₇ | CH₃ | 5-CH₃ |
| 1382 | 2,4-(i-C₃H₇)₂ | CH₃ | 5-CH₃ |
| 1383 | 2,6-(i-C₃H₇)₂ | CH₃ | 5-CH₃ |
| 1384 | 3,5-(i-C₃H₇)₂ | CH₃ | 5-CH₃ |
| 1385 | 2-s-C₄H₉ | CH₃ | 5-CH₃ |
| 1386 | 3-s-C₄H₉ | CH₃ | 5-CH₃ |
| 1387 | 4-s-C₄H₉ | CH₃ | 5-CH₃ |
| 1388 | 2-t-C₄H₉ | CH₃ | 5-CH₃ |
| 1389 | 3-t-C₄H₉ | CH₃ | 5-CH₃ |
| 1390 | 4-t-C₄H₉ | CH₃ | 5-CH₃ |
| 1391 | 4-n-C₉H₁₉ | CH₃ | 5-CH₃ |
| 1392 | 2-CH₃, 4-t-C₄H₉ | CH₃ | 5-CH₃ |
| 1393 | 2-CH₃, 6-t-C₄H₉ | CH₃ | 5-CH₃ |
| 1394 | 2-CH₃, 4-i-C₃H₇ | CH₃ | 5-CH₃ |
| 1395 | 2-CH₃, 5-i-C₃H₇ | CH₃ | 5-CH₃ |
| 1396 | 3-CH₃, 4-i-C₃H₇ | CH₃ | 5-CH₃ |
| 1397 | 2-cyclo-C₆H₁₁ | CH₃ | 5-CH₃ |
| 1398 | 3-cyclo-C₆H₁₁ | CH₃ | 5-CH₃ |
| 1399 | 4-cyclo-C₆H₁₁ | CH₃ | 5-CH₃ |
| 1400 | 2-Cl, 4-C₆H₅ | CH₃ | 5-CH₃ |
| 1401 | 2-Br, 4-C₆H₅ | CH₃ | 5-CH₃ |
| 1402 | 2-OCH₃ | CH₃ | 5-CH₃ |
| 1403 | 3-OCH₃ | CH₃ | 5-CH₃ |
| 1404 | 4-OCH₃ | CH₃ | 5-CH₃ |
| 1405 | 2-OC₂H₅ | CH₃ | 5-CH₃ |
| 1406 | 3-O-C₂H₅ | CH₃ | 5-CH₃ |
| 1407 | 4-O-C₂H₅ | CH₃ | 5-CH₃ |
| 1408 | 2-O-n-C₃H₇ | CH₃ | 5-CH₃ |
| 1409 | 3-O-n-C₃H₇ | CH₃ | 5-CH₃ |
| 1410 | 4-O-n-C₃H₇ | CH₃ | 5-CH₃ |
| 1411 | 2-O-i-C₃H₇ | CH₃ | 5-CH₃ |
| 1412 | 3-O-i-C₃H₇ | CH₃ | 5-CH₃ |
| 1413 | 4-O-i-C₃H₇ | CH₃ | 5-CH₃ |
| 1414 | 2-O-n-C₆H₁₃ | CH₃ | 5-CH₃ |
| 1415 | 3-O-n-C₆H₁₃ | CH₃ | 5-CH₃ |
| 1416 | 4-O-n-C₆H₁₃ | CH₃ | 5-CH₃ |
| 1417 | 2-O-CH₂C₆H₅ | CH₃ | 5-CH₃ |
| 1418 | 3-O-CH₂C₆H₅ | CH₃ | 5-CH₃ |
| 1419 | 4-O-CH₂C₆H₅ | CH₃ | 5-CH₃ |
| 1420 | 2-O-(CH₂)₃C₆H₅ | CH₃ | 5-CH₃ |
| 1421 | 4-O-(CH₂)₃C₆H₅ | CH₃ | 5-CH₃ |
| 1422 | 2,3-(OCH₃)₂ | CH₃ | 5-CH₃ |
| 1423 | 2,4-(OCH₃)₂ | CH₃ | 5-CH₃ |
| 1424 | 2,5-(OCH₃)₂ | CH₃ | 5-CH₃ |
| 1425 | 2,6-(OCH₃)₂ | CH₃ | 5-CH₃ |
| 1426 | 3,4-(OCH₃)₂ | CH₃ | 5-CH₃ |
| 1427 | 3,5-(OCH₃)₂ | CH₃ | 5-CH₃ |
| 1428 | 2-O-t-C₄H₉ | CH₃ | 5-CH₃ |
| 1429 | 3-O-t-C₄H₉ | CH₃ | 5-CH₃ |
| 1430 | 4-O-t-C₄H₉ | CH₃ | 5-CH₃ |
| 1431 | 3-(3'-Cl-C₆H₄) | CH₃ | 5-CH₃ |
| 1432 | 4-(4'-CH₃-C₆H₄) | CH₃ | 5-CH₃ |
| 1433 | 2-O-C₆H₅ | CH₃ | 5-CH₃ |
| 1434 | 3-O-C₆H₅ | CH₃ | 5-CH₃ |
| 1435 | 4-O-C₆H₅ | CH₃ | 5-CH₃ |
| 1436 | 2-O-(2'-F-C₆H₄) | CH₃ | 5-CH₃ |
| 1437 | 3-O-(3'-Cl-C₆H₄) | CH₃ | 5-CH₃ |
| 1438 | 4-O-(4'-CH₃-C₆H₄) | CH₃ | 5-CH₃ |
| 1439 | 2,3,6-(CH₃)₃, 4-F | CH₃ | 5-CH₃ |
| 1440 | 2,3,6-(CH₃)₃, 4-Cl | CH₃ | 5-CH₃ |
| 1441 | 2,3,6-(CH₃)₃, 4-Br | CH₃ | 5-CH₃ |
| 1442 | 2,4-(CH₃)₂, 6-F | CH₃ | 5-CH₃ |
| 1443 | 2,4-(CH₃)₂, 6-Cl | CH₃ | 5-CH₃ |
| 1444 | 2,4-(CH₃)₂, 6-Br | CH₃ | 5-CH₃ |
| 1445 | 2-i-C₃H₇, 4-Cl, 5-CH₃ | CH₃ | 5-CH₃ |
| 1446 | 2-Cl, 4-NO₂ | CH₃ | 5-CH₃ |
| 1447 | 2-NO₂, 4-Cl | CH₃ | 5-CH₃ |
| 1448 | 2-OCH₃, 5-NO₂ | CH₃ | 5-CH₃ |
| 1449 | 2,4-Cl₂, 5-NO₂ | CH₃ | 5-CH₃ |
| 1450 | 2,4-Cl₂, 6-NO₂ | CH₃ | 5-CH₃ |
| 1451 | 2,6-Cl₂, 4-NO₂ | CH₃ | 5-CH₃ |
| 1452 | 2,6-Br₂, 4-NO₂ | CH₃ | 5-CH₃ |
| 1453 | 2,6-J₂, 4-NO₂ | CH₃ | 5-CH₃ |
| 1454 | 2-CH₃ , 5-i-C₃H₇, 4-Cl | CH₃ | 5-CH₃ |
| 1455 | 2-CO₂CH₃ | CH₃ | 5-CH₃ |
| 1456 | 3-CO₂CH₃ | CH₃ | 5-CH₃ |
| 1457 | 4-CO₂CH₃ | CH₃ | 5-CH₃ |
| 1458 | 2-CH₂-OCH₃ | CH₃ | 5-CH₃ |
| 1459 | 3-CH₂-OCH₃ | CH₃ | 5-CH₃ |
| 1460 | 4-CH₂-OCH₃ | CH₃ | 5-CH₃ |
| 1461 | 2-Me-4-CH₃-CH(CH₃)-CO | CH₃ | 5-CH₃ |
| 1462 | 2-CH₃-4-(CH₃-C=NOCH₃) | CH₃ | 5-CH₃ |
| 1463 | 2-CH₃-4-(CH₃-C=NOC₂H₅) | CH₃ | 5-CH₃ |
| 1464 | 2-CH₃-4-(CH₃-C=NO-n-C₃H₇) | CH₃ | 5-CH₃ |
| 1465 | 2-CH₃-4-(CH₃-C=NO-i-C₃H₇) | CH₃ | 5-CH₃ |
| 1466 | 2,5-(CH₃)₂-4-(CH₃-C=NOCH₃) | CH₃ | 5-CH₃ |
| 1467 | 2,5-(CH₃)₂-4-(CH₃-C=NOC₂H₅) | CH₃ | 5-CH₃ |
| 1468 | 2,5-(CH₃-4-(CH₃-C=NO-n-C₃H₇) | CH₃ | 5-CH₃ |
| 1469 | 2,5-(CH₃)₂-4-(CH₃-C=NO-i-C₃H₇) | CH₃ | 5-CH₃ |
| 1470 | 2-C₆H₅ | CH₃ | 5-CH₃ |
| 1471 | 3-C₆H₅ | CH₃ | 5-CH₃ |
| 1472 | 4-C₆H₅ | CH₃ | 5-CH₃ |
| 1473 | 2-(2'-F-C₆H₄) | CH₃ | 5-CH₃ |
| 1474 | 2-CH₃, 5-Br | CH₃ | 5-CH₃ |
| 1475 | 2-CH₃, 6-Br | CH₃ | 5-CH₃ |
| 1476 | 2-Cl, 3-CH₃ | CH₃ | 5-CH₃ |
| 1477 | 2-Cl, 4-CH₃ | CH₃ | 5-CH₃ |
| 1478 | 2-Cl, 5-CH₃ | CH₃ | 5-CH₃ |
| 1479 | 2-F, 3-CH₃ | CH₃ | 5-CH₃ |
| 1480 | 2-F 4-CH₃ | CH₃ | 5-CH₃ |
| 1481 | 2-F, 5-CH₃ | CH₃ | 5-CH₃ |
| 1482 | 2-Br, 3-CH₃ | CH₃ | 5-CH₃ |
| 1483 | 2-Br, 4-CH₃ | CH₃ | 5-CH₃ |
| 1484 | 2-Br, 5-CH₃ | CH₃ | 5-CH₃ |
| 1485 | 3-CH₃ , 4-Cl | CH₃ | 5-CH₃ |
| 1486 | 3-CH₃ , 5-Cl | CH₃ | 5-CH₃ |
| 1487 | 3-CH₃, 4-F | CH₃ | 5-CH₃ |
| 1488 | 3-CH₃, 5-F | CH₃ | 5-CH₃ |
| 1489 | 3-CH₃ , 4-Br | CH₃ | 5-CH₃ |
| 1490 | 3-CH₃ , 5-Br | CH₃ | 5-CH₃ |
| 1491 | 3-F, 4-CH₃ | CH₃ | 5-CH₃ |
| 1492 | 3-Cl, 4-CH₃ | CH₃ | 5-CH₃ |
| 1493 | 3-Br, 4-CH₃ | CH₃ | 5-CH₃ |
| 1494 | 2-Cl, 4,5-(CH₃)₂ | CH₃ | 5-CH₃ |
| 1495 | 2-Br, 4,5-(CH₃)₂ | CH₃ | 5-CH₃ |
| 1496 | 2-Cl, 3,5-(CH₃)₂ | CH₃ | 5-CH₃ |
| 1497 | 2-Br, 3,5-(CH₃)₂ | CH₃ | 5-CH₃ |
| 1498 | 2,6-Cl₂, 4-CH₃ | CH₃ | 5-CH₃ |
| 1499 | 2,6-F₂, 4-CH₃ | CH₃ | 5-CH₃ |
| 1500 | 2,6-Br₂,4-CH₃ | CH₃ | 5-CH₃ |
| 1501 | 2,4-Br₂, 6-CH₃ | CH₃ | 5-CH₃ |
| 1502 | 2,4-F₂, 6-CH₃ | CH₃ | 5-CH₃ |
| 1503 | 2,4-Br₂, 6-CH₃ | CH₃ | 5-CH₃ |
| 1504 | 2,6-(CH₃)₂, 4-F | CH₃ | 5-CH₃ |
| 1505 | 2,6-(CH₃)₂, 4-Cl | CH₃ | 5-CH₃ |
| 1506 | 2,6-(CH₃)₂, 4-Br | CH₃ | 5-CH₃ |
| 1507 | 3,5-(CH₃)₂, 4-F | CH₃ | 5-CH₃ |
| 1508 | 3,5-(CH₃)₂, 4-Cl | CH₃ | 5-CH₃ |
| 1509 | 3,5-(CH₃)₂, 4-Br | CH₃ | 5-CH₃ |
| 1510 | 2-CF₃ | CH₃ | 5-CH₃ |
| 1511 | 3-CF₃ | CH₃ | 5-CH₃ |
| 1512 | 4-CF₃ | CH₃ | 5-CH₃ |
| 1513 | 2-OCF₃ | CH₃ | 5-CH₃ |
| 1514 | 3-OCF₃ | CH₃ | 5-CH₃ |
| 1515 | 4-OCF₃ | CH₃ | 5-CH₃ |
| 1516 | 3-OCH₂CHF₂ | CH₃ | 5-CH₃ |
| 1517 | 2-NO₂ | CH₃ | 5-CH₃ |
| 1518 | 3-NO₂ | CH₃ | 5-CH₃ |
| 1519 | 4-NO₂ | CH₃ | 5-CH₃ |
| 1520 | 2-CN | CH₃ | 5-CH₃ |
| 1521 | 3-CN | CH₃ | 5-CH₃ |
| 1522 | 4-CN | CH₃ | 5-CH₃ |
| 1523 | 2-CH₃, 3-Cl | CH₃ | 5-CH₃ |
| 1524 | 2-CH₃, 4-Cl | CH₃ | 5-CH₃ |
| 1525 | 2-CH₃, 5-Cl | CH₃ | 5-CH₃ |
| 1526 | 2-CH₃, 6-Cl | CH₃ | 5-CH₃ |
| 1527 | 2-CH₃, 3-F | CH₃ | 5-CH₃ |
| 1528 | 2-CH₃ , 4-F | CH₃ | 5-CH₃ |
| 1529 | 2-CH₃ , 5-F | CH₃ | 5-CH₃ |
| 1530 | 2-CH₃ , 6-F | CH₃ | 5-CH₃ |
| 1531 | 2-CH₃, 3-Br | CH₃ | 5-CH₃ |
| 1532 | 2-CH₃, 4-Br | CH₃ | 5-CH₃ |
| 1533 | 2-Pyridyl-2' | CH₃ | 5-CH₃ |
| 1534 | 3-Pyridyl-3' | CH₃ | 5-CH₃ |
| 1535 | 4-Pyridyl-4' | CH₃ | 5-CH₃ |
| 1536 | 2-CO-CH₃ | CH₃ | 5-CH₃ |
| 1537 | 3-CO-CH₃ | CH₃ | 5-CH₃ |
| 1538 | 4-CO-CH₃ | CH₃ | 5-CH₃ |
| 1539 | 2-C(=N-OCH₃)-CH₃ | CH₃ | 5-CH₃ |
| 1540 | 3-C(=N-OCH₃)-CH₃ | CH₃ | 5-CH₃ |
| 1541 | 4-C(=N-OCH₃)-CH₃ | CH₃ | 5-CH₃ |

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Verbindungen I können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die Verbindungen der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zukkerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz z.B. zum Schutz von Holz, Papier und Textilien eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-VolumeVerfahren (ULV) ausgebracht werden, wobei sogar der Wirksoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

### Beispiele für solche Zubereitungen sind:

I. eine Lösung aus 90 Gew.-Teilen einer erfindungsgemäßen Verbindung I und 10 Gew.-Teilen N-Methyl-α-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-a-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schadlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von zink-(N,N'-propylen-bis-dithiocarbamat), Zink-(N,N'-propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthals°ure-di-isopropylester;
heterocyclische Substanzen, wie 2-Heptadecyl-2-imidazolin-acetat, 2,4-Dichlor-6- (o-chloranilino)-s-triazin, O,O-Diethyl-phthalimidophosphonothioat, 5-Amino-1-β-[bis-(dimethylamino)-phosphinyl]-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N- [3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N- (2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3- (3,5-dichlorphenyl)-2,4-dioxo-l,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.

1. N-Methoxy-N-(2-(4'-(2"-fluorphenyl)-2'-methyl-phenoxymethyl)-phenyl)-carbaminsäuremethylester 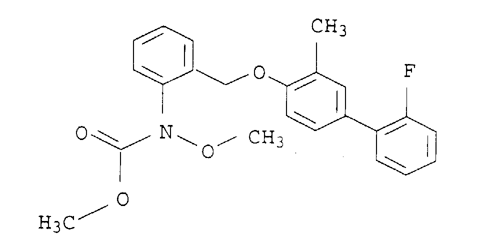 Eine Mischung von 1 g (2,6 mmol) N-Methoxy-N-(2-(4'-brom-2'-methyl-phenoxymethyl)-phenyl)-carbaminsäuremethylester (Herstellung analog WO 93/15046), 0,55 g (3,9 mmol) 2-Fluorphenylboronsäure und 0,33 g (3,9 mmol) NaHCO₃ in 4 ml Dimethoxyethan/Wasser 1:1 wird ca. 5 Stunden zum Rückfluß erhitzt und gerührt. Anschließend kühlt man ab, verdünnt die Reaktionsmischung mit Wasser und extrahiert die wäßrige Phase dreimal mit Methylenchlorid. Die vereinigten organischen Phasen werden mit Wasser extrahiert, über MgSO₄ getrocknet und eingeengt. Als Rückstand erhält man 1 g (95 %) der Titelverbindung als hellgelbe Kristalle (Fp= 71°C).
¹H-NMR (CDCl₃; δ in ppm):
7,7(d,1H,Phenyl); 7,35(m,8H,Phenyl); 6,95(m,2H,Phenyl);
5,2(s,2H,OCH₂); 3,82(s,3H,OCH₃); 3,75(s,3H,OCH₃);
2,4(s,3H,CH₃)

**Tabelle B**

| Physikalische Daten einiger Verbindungen | | | | |
|---|---|---|---|---|
| Nr. | Rₘ | R² | R¹ₙ | FP[°C] oder NMR (CDCl₃; δ in ppm) |
| 1 | 4-F | F | H | 3,8(s,3H); 3,73(s,3H) |
| 2 | 2-F | CH₃ | H | 71 |
| 3 | 3-OCH₃ | CH₃ | H | 3,85(s,3H); 3,8(s,3H); 3,73(s,3H) |
| 4 | 2-CH₃ | CH₃ | H | 3,8(s,3H); 3,73(s,3H) |
| 5 | 2,4-Cl₂ | CH₃ | H | 3,82(s,3H); 3,75(s,3H) |
| 6 | 4-OCH₃ | CH₃ | H | 113 |
| 7 | 2-OCH₃ | CH₃ | H | 3,8(2s, je 3H); 3,75(s,3H) |
| 8 | 3,4-Cl₂ | CH₃ | H | 3,8(s,3H); 3,73(s,3H) |
| 9 | 3,5-Cl₂ | CH₃ | H | 3,83(s,3H); 3,77(s,3H) |
| 10 | 4-CH₃ | CH₃ | H | 3,7(s,3H); 3,66(s,3H) (in DMSO-d₆) |

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirksamkeit gegen Plasmopara viticola

Topfreben (Sorte: "Müller Thurgau") wurden mit der Wirkstoffaufbereitung tropfnaß gespritzt. Nach 8 Tagen wurden die Pflanzen mit einer Zoosporenaufschwemmung des Pilzes Plasmopara viticola besprüht und 5 Tage bei 20-30°C bei hoher Luftfeuchtigkeit bewahrt. Vor der Beurteilung wurden die Pflanzen danach für 16 h bei hoher Luftfeuchtigkeit bewahrt. Die Auswertung erfolgte visuell.

In diesem Test zeigten die mit den erfindungsgemäßen Wirkstoffen 2, 3, 4, 5, 6, 7, 9 und 10 behandelten Pflanzen einen Pilzbefall von 15 % und weniger während die unbehandelten Pflanzen (Kontrollversuch) zu 70 % befallen waren.

### Wirksamkeit gegen Puccinia recondita (Weizenbraunrost)

Blätter von Weizensämlingen (Sorte "Kanzler") wurden mit Sporen des Braunrosts (Puccinia recondita) bestäubt. Die so behandelten Pflanzen wurden 24 h bei 20-22°C und einer relativen Luftfeuchtigkeit von 90-95 % inkubiert und anschließend mit der wäßrigen Wirkstoffaufbereitung behandelt. Nach weiteren 8 Tagen bei 20-22°C und 65-70 % relativer Luftfeuchtigkeit wurde das Ausmaß der Pilzentwicklung ermittelt. Die Auswertung erfolgte visuell.

In diesem Test zeigten die mit den erfindungsgemäßen Wirkstoffen 1, 2, 3, 4, 5, 6, 7, 8, 9 und 10 behandelten Pflanzen einen Pilzbefall von 5 % und weniger während die unbehandelten Pflanzen (Kontrollversuch) zu 65 % befallen waren.

### Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. 2-[4-Biphenyl-oxymethylen]-anilide der Formel I 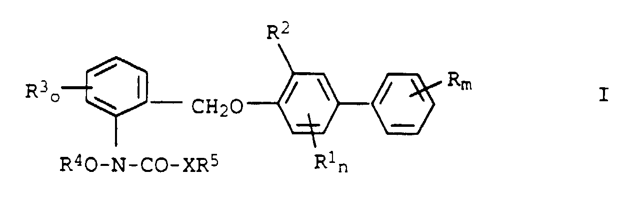 in der die Indices und die Substituenten die folgende Bedeutung haben:
R Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino, C₁-C₄-Alkylcarbonyl, C₁-C₄-Alkoxycarbonyl, C₁-C₄-Alkylaminocarbonyl, Di-(C₁-C₄-alkyl)aminocarbonyl, C₁-C₄-Alkylcarbonylamino, C₁-C₄-Alkylcarbonyl-(C₁-C₄-alkylamino), C₂-C₄-Alkenyl, C₃-C₄-Alkenyloxy, C₂-C₄-Alkinyl, C₃-C₄-Alkinyloxy, C₃-C₆-Cycloalkyl oder C(R^{a})=NOR^{b};
R^{a},R^{b} Wasserstoff, Alkyl, Alkenyl, Alkinyl, Cycloalkyl oder Cycloalkenyl;
m 1, 2, 3, 4 oder 5, wobei die Reste R verschieden sein können, wenn m größer als 1 ist;
R¹ Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino, C₁-C₄-Alkylcarbonyl, C₁-C₄-Alkoxycarbonyl, C₁-C₄-Alkylaminocarbonyl, Di-(C₁-C₄-alkyl)aminocarbonyl, C₁-C₄-Alkylcarbonylamino, C₁-C₄-Alkylcarbonyl-(C₁-C₄-alkylamino), C₂-C₄-Alkenyl, C₃-C₄-Alkenyloxy, C₂-C₄-Alkinyl, C₃-C₄-Alkinyloxy, C₃-C₆-Cycloalkyl oder C(R^{a}) =NOR^{b};
n 0, 1 oder 2, wobei die Reste R¹ verschieden sein können, wenn n für 2 steht;
R² Cyano, Nitro, Halogen, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylamino, Di-C₁-C₄-alkylamino, C₁-C₄-Alkylcarbonyl, C₁-C₄-Alkoxycarbonyl, C₁-C₄-Alkylaminocarbonyl, Di-(C₁-C₄-alkyl)aminocarbonyl, C₁-C₄-Alkylcarbonylamino, C₁-C₄-Alkylcarbonyl-(C₁-C₄-alkylamino), C₂-C₄-Alkenyl, C₃-C₄-Alkenyloxy, C₂-C₄-Alkinyl, C₃-C₄-Alkinyloxy, C₃-C₆-Cycloalkyl oder C(R^{a})=NOR^{b};
R³ Nitro, Cyano, Halogen,
ggf. subst. Alkyl, Alkenyl, Alkinyl, Alkoxy, Alkenyloxy, Alkinyloxy oder
C₁-C₄-Alkylcarbonyl, C₁-C₄-Alkoxycarbonyl, C₁-C₄-Alkylaminocarbonyl, Di-(C₁-C₄-alkyl)aminocarbonyl, C₁-C₄-Alkylcarbonylamino, C₁-C₄-Alkylcarbonyl-(C₁-C₄-alkylamino), C(R^{a})=NOR^{b} oder
für den Fall, daß o größer als 1 ist, zusätzlich eine an zwei benachbarte Ringatome gebundene ggf. subst. Brücke, welche drei bis vier Glieder aus der Gruppe 3 oder 4 Kohlenstoffatome, 2 oder 3 Kohlenstoffatome und 1 oder 2 Stickstoff-, Sauerstoff- und/oder Schwefelatome, wobei diese Brücke gemeinsam mit dem Ring an den sie gebunden ist einen partiell ungesättigten oder aromatischen Rest bilden kann;
o 0, 1, 2, 3 oder 4, wobei die Substituenten R³ verschieden sein können, wenn n größer als 1 ist;
R⁴ Wasserstoff,
ggf. subst. Alkyl, Alkenyl, Alkinyl, Cycloalkyl, Cycloalkenyl, Alkylcarbonyl oder Alkoxycarbonyl;
X eine direkte Bindung oder CH₂, O oder NR^{c};
R^{c} Wasserstoff, Alkyl, Alkenyl, Alkinyl, Cycloalkyl oder Cycloalkenyl;
R⁵ Alkyl, Alkenyl, Alkinyl, Cycloalkyl oder Cycloalkenyl, oder
für den Fall, daß X für NR^{c} steht, zusätzlich Wasserstoff.

2. Verfahren zur Herstellung der Verbindungen der Formel I gemäß Anspruch 1, in denen R⁴ Wasserstoff bedeutet und X für eine direkte Bindung oder Sauerstoff steht, dadurch gekennzeichnet, daß man ein Benzylderivat der Formel II 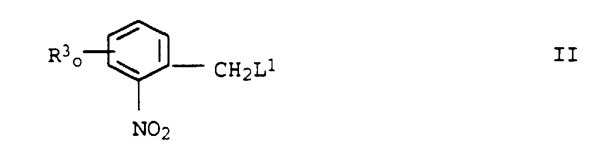 in der L¹ eine nucleophil austauschbare Gruppe bedeutet, in Gegenwart einer Base mit einem 4-Biphenol der Formel III 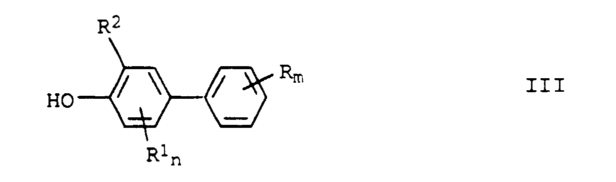 in das entsprechende 2-[4-Biphenyl-oxymethylen]-nitrobenzol der Formel IV 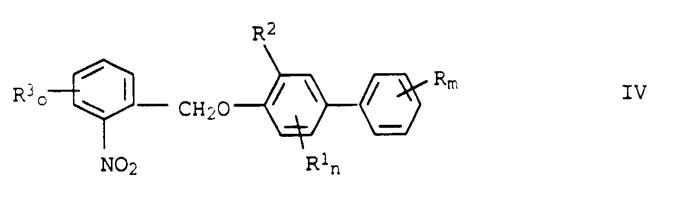 überführt, IV anschließend zum N-Hydroxylanilin der Formel Va 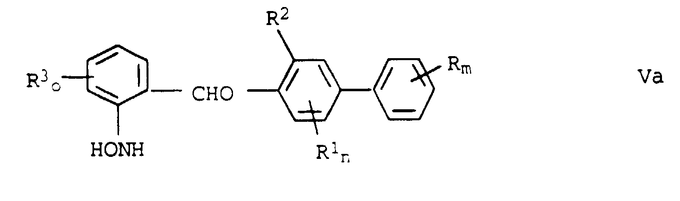 reduziert und Va mit einer Carbonylverbindung der Formel VI
L²-CO-X-R⁵ VI
in der L² Halogen bedeutet, in I umwandelt.

3. Verfahren zur Herstellung der Verbindungen der Formel I gemäß Anspruch 1, in denen R⁴ nicht Wasserstoff bedeutet, dadurch gekennzeichnet, daß man ein Benzylderivat der Formel IIa 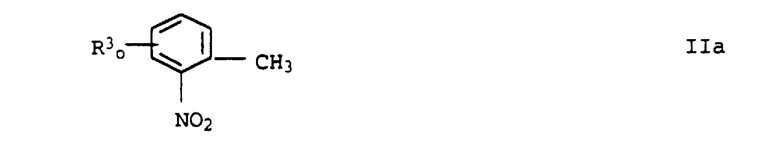 zunächst zum entsprechenden N-Hydroxyanilin der Formel Vb 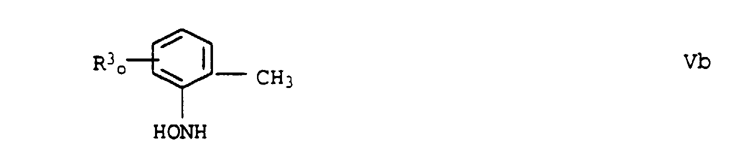 reduziert, Vb mit einer Carbonylverbindung der Formel VI gemäß Anspruch 2 in das entsprechende Anilid der Formel VII 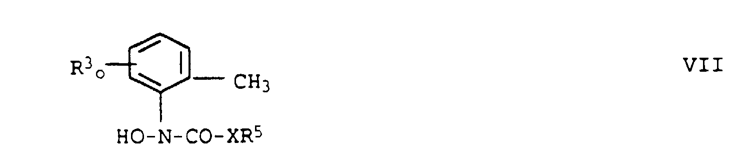 überführt und VII anschließend mit einer Verbindung VIII
L³-R⁴ VIII
in der L³ eine nucleophil austauschbare Gruppe bedeutet und R⁴ nicht für Wasserstoff steht, in das Amid der Formel IX 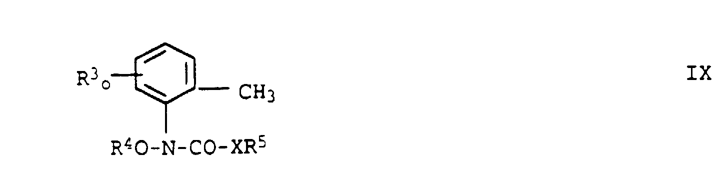 überführt, IX anschließend in das entsprechende Benzylhalogenid der Formel X 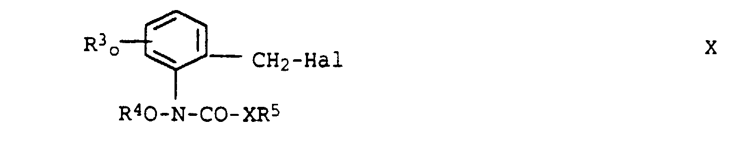 in der Hal für ein Halogenatom steht, überführt und X in Gegenwart einer Base mit einem 4-Biphenol der Formel III gemäß Anspruch 2 in I umwandelt.

4. Verfahren zur Herstellung der Verbindungen I gemäß Anspruch 1, in denen R⁴ nicht Wasserstoff bedeutet, dadurch gekennzeichnet, daß man eine entsprechende Verbindung der Formel I, in der R⁴ Wasserstoff bedeutet, mit einer entsprechenden Verbindung der Formel VIII gemäß Anspruch 3 umsetzt.

5. Verfahren zur Herstellung der Verbindungen der Formel I, in denen X für NR^{c} steht, dadurch gekennzeichnet, daß man ein Benzanilid der Formel IXa 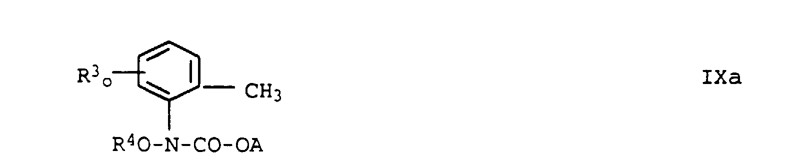 in der A für Alkyl oder Phenyl steht, in das entsprechende Benzylhalogenid der Formel Xa 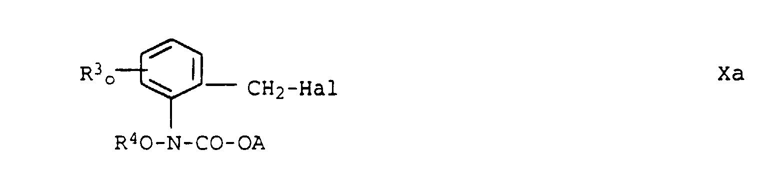 in der Hal für ein Halogenatom steht, überführt, Xa in Gegenwart einer Base mit einem 4-Biphenol der Formel III gemäß Anspruch 2 in eine Verbindung der Formel I.A 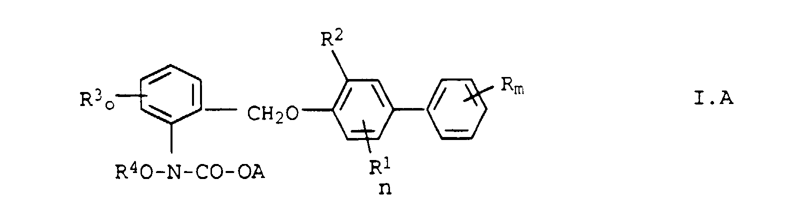 überführt und I.A anschließend mit einem Amin der Formel XIa bzw. XIb 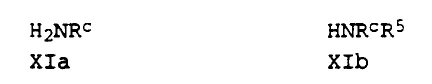 zu I umsetzt.

6. Verfahren zur Herstellung von Verbindungen der Formel I.A gemäß Anspruch 5, in denen A Phenyl bedeutet, dadurch gekennzeichnet, daß man ein N-Hydroxylanilin der Formel VA gemäß Anspruch 2 mit einem Kohlensäurephenylester der Formel VIA
L²-CO₂-C₆H₅ VIA
in dem L² für ein Halogenatom steht, in das entsprechende N-Hydroxycarbamat der Formel IB 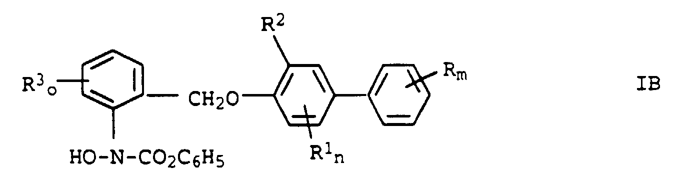 überführt und IB anschließend mit einer Verbindung VIII gemäß Anspruch 3 zu IA umsetzt.

7. Verfahren zur Herstellung der Verbindung I gemäß Anspruch 1, dadurch gekennzeichnet, daß man eine Verbindung der Formel XIV 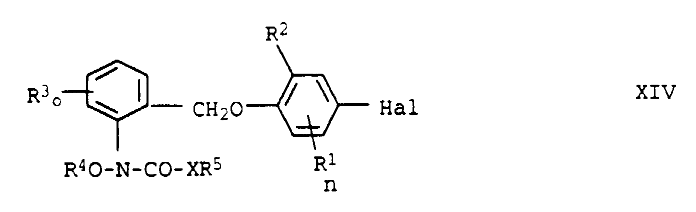 in der Hal für ein Halogenatom steht, in Gegenwart eines Palladium-Katalysators mit einer metallorganischen Verbindung der Formel XV 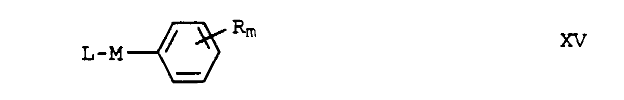 in der L-M einen metallhaltigen Rest bedeutet, umsetzt.

8. Zwischenprodukte der Formel XII 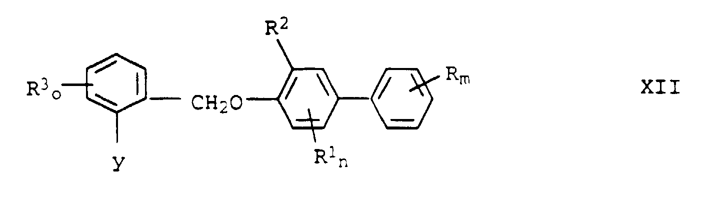 in der Y für NO₂, NHOH, NHOR⁴, N(OH)-CO₂C₆H₅ oder N(OR⁴)-CO₂C₆H₅ steht und die Indices m, n und o und die Substituenten R, R¹, R², R³ und R⁴ die in Anspruch 1 gegebene Bedeutung haben.

9. Zur Bekämpfung von tierischen Schädlingen oder Schadpilzen geeignetes Mittel, enthaltend einen festen oder flüssigen Trägerstoff und eine Verbindung der allgemeinen Formel I gemäß Anspruch 1.

10. Verwendung der Verbindungen I gemäß Anspruch 1 zur Herstellung eines zur Bekämpfung von tierischen Schädlingen oder Schadpilzen geeigneten Mittels.

11. Verfahren zur Bekämpfung von tierischen Schädlingen oder Schadpilzen, dadurch gekennzeichnet, daß man die zu bekämpfenden Schädlinge oder Pilze oder die vor ihnen zu schützenden Materialien, Pflanzen, Boden oder Saatgüter mit einer wirksamen Menge einer Verbindung der allgemeinen Formel I gemäß Anspruch 1 behandelt.

## Claims

1. A 2-[4-biphenyloxymethylene]anilide of the formula I 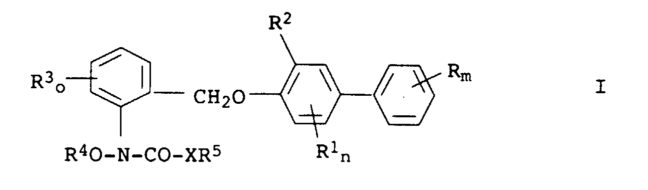 where the indices and the substituents have the following meanings:
R is cyano, nitro, halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylamino, di-C₁-C₄-alkylamino, C₁-C₄-alkylcarbonyl, C₁-C₄-alkoxycarbonyl, C₁-C₄-alkylaminocarbonyl, di(C₁-C₄-alkyl)aminocarbonyl, C₁-C₄-alkylcarbonylamino, C₁-C₄-alkylcarbonyl(C₁-C₄-alkylamino), C₂-C₄-alkenyl, C₃-C₄-alkenyloxy, C₂-C₄-alkynyl, C₃-C₄-alkynyloxy, C₃-C₆-cycloalkyl or C(R^{a})=NOR^{b};
R^{a} and R^{b} are hydrogen, alkyl, alkenyl, alkynyl, cycloalkyl or cycloalkenyl;
m is 1, 2, 3, 4 or 5, it being possible for the radicals R to be different if m is greater than 1;
R¹ is cyano, nitro, halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylamino, di-C₁-C₄-alkylamino, C₁-C₄-alkylcarbonyl, C₁-C⁴-alkoxycarbonyl, C₁-C₄-alkylaminocarbonyl, di(C₁-C₄-alkyl)aminocarbonyl, C₁-C₄-alkylcarbonylamino, C₁-C₄-alkylcarbonyl(C₁-C₄-alkylamino), C₂-C₄-alkenyl, C₃-C₄-alkenyloxy, C₂-C₄-alkynyl, C₃-C₄-alkynyloxy, C₃-C₆-cycloalkyl or C(R^{a})=NOR^{b};
n is 0, 1 or 2, it being possible for the radicals R¹ to be different if n is 2;
R² is cyano, nitro, halogen, C₁-C₄-alkyl, C₁-C₄-haloalkyl, C₁-C₄-alkoxy, C₁-C₄-haloalkoxy, C₁-C₄-alkylthio, C₁-C₄-haloalkylthio, C₁-C₄-alkylamino, di-C₁-C₄-alkylamino, C₁-C₄-alkylcarbonyl, C₁-C₄-alkoxycarbonyl, C₁-C₄-alkylaminocarbonyl, di(C₁-C₄-alkyl)aminocarbonyl, C₁-C₄-alkylcarbonylamino, C₁-C₄-alkylcarbonyl(C₁-C₄-alkylamino), C₂-C₄-alkenyl, C₃-C₄-alkenyloxy, C₂-C₄-alkynyl, C₃-C₄-alkynyloxy, C₃-C₆-cycloalkyl or C(R^{a})=NOR^{b};
R³ is nitro, cyano, halogen,
unsubst. or subst. alkyl, alkenyl, alkynyl, alkoxy, alkenyloxy, alkynyloxy or
C₁-C₄-alkylcarbonyl, C₁-C₄-alkoxycarbonyl, C₁-C₄-alkylaminocarbonyl, di(C₁-C₄-alkyl)aminocarbonyl, C₁-C₄-alkylcarbonylamino, C₁-C₄-alkylcarbonyl(C₁-C₄-alkylamino), C(R^{a})=NOR^{b} or
in the case where o is greater than 1, is additionally an unsubst. or subst. bridge bonded to two adjacent ring atoms, which can carry three to four members from the group consisting of 3 or 4 carbon atoms, 2 or 3 carbon atoms and I or 2 nitrogen, oxygen and/or sulfur atoms, it being possible for this bridge, together with the ring to which it is bonded, to form a partially unsaturated or aromatic radical;
o is 0, 1, 2, 3 or 4, it being possible for the substituents R³ to be different if n is greater than 1;
R⁴ is hydrogen,
unsubst. or subst. alkyl, alkenyl, alkynyl, cycloalkyl, cycloalkenyl, alkylcarbonyl or alkoxycarbonyl;
X is a direct bond or CH₂, O or NR^{c};
R^{c} is hydrogen, alkyl, alkenyl, alkynyl, cycloalkyl or cycloalkenyl;
R⁵ is alkyl, alkenyl, alkynyl, cycloalkyl or cycloalkenyl, or
in the case where X is NR^{c}, is additionally hydrogen.

2. A process for preparing the compounds of the formula I as claimed in claim 1, where R⁴ is hydrogen and X is a direct bond or oxygen, which comprises converting a benzyl derivative of the formula II 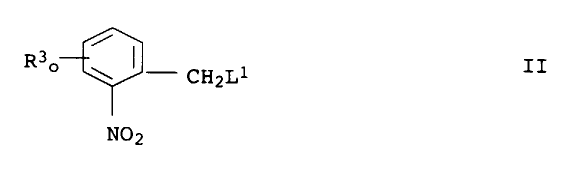 where L¹ is a nucleophilically replaceable group, in the presence of a base using a 4-biphenol of the formula III 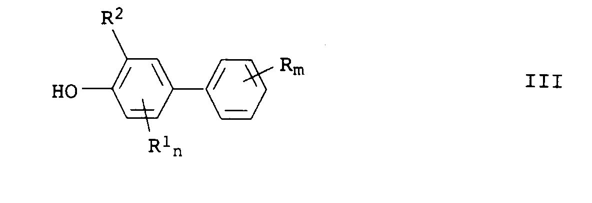 into the corresponding 2-[4-biphenyloxymethylene]nitrobenzene of the formula IV 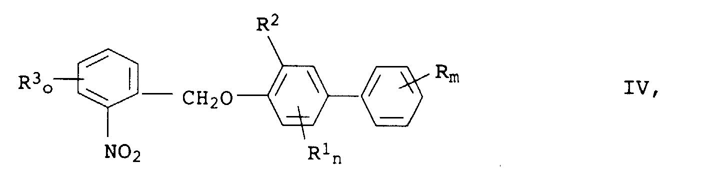 then reducing IV to the N-hydroxyaniline of the formula Va 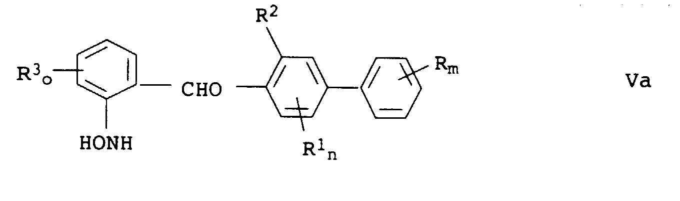 and converting Va into I using a carbonyl compound of the formula VI
L²-CO-X-R⁵ VI
where L² is halogen.

3. A process for preparing the compounds of the formula I as claimed in claim I, where R⁴ is not hydrogen, which comprises reducing a benzyl derivative of the formula IIa 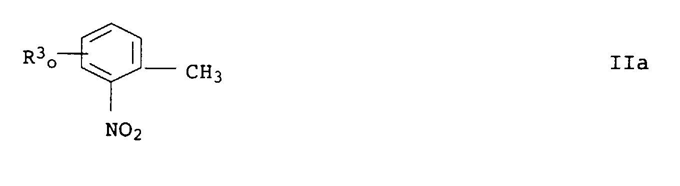 first to the corresponding N-hydroxyaniline of the formula Vb 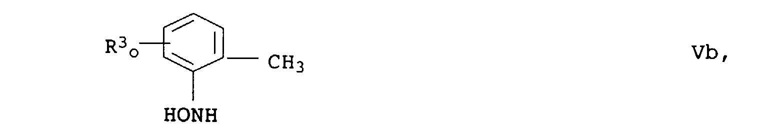 converting Vb into the corresponding anilide of the formula VII 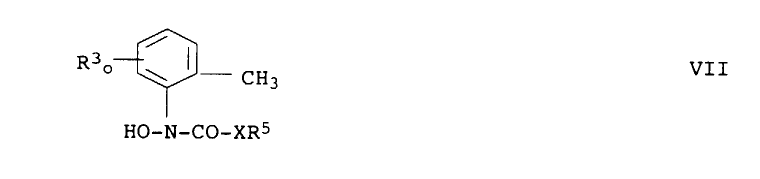 using a carbonyl compound of the formula VI as set forth in claim 2 and then converting VII using a compound VIII
L³-R⁴ VIII
where L³ is a nucleophilically replaceable group and R⁴ is not hydrogen, into the amide of the formula IX 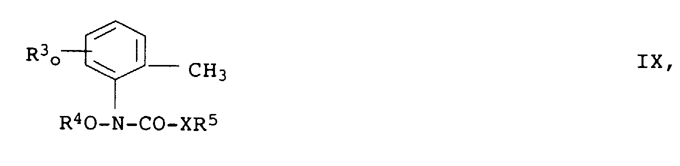 then converting IX into the corresponding benzyl halide of the formula X 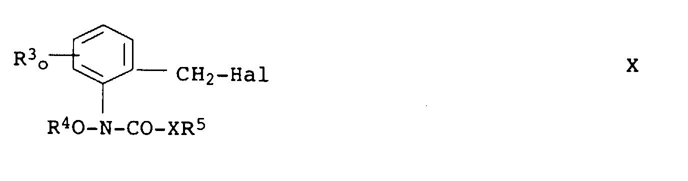 where Hal is a halogen atom, and converting X into I in the presence of a base using a 4-biphenol of the formula III as set forth in claim 2.

4. A process for preparing the compounds I as claimed in claim 1, where R⁴ is not hydrogen, which comprises reacting a corresponding compound of the formula I where R⁴ is hydrogen with an appropriate compound of the formula VIII as set forth in claim 3.

5. A process for preparing the compounds of the formula I where X is NR^{c}, which comprises converting a benzanilide of the formula IXa 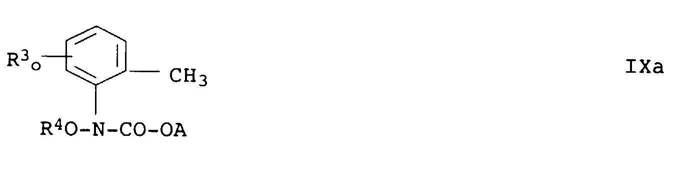 where A is alkyl or phenyl, into the corresponding benzyl halide of the formula Xa 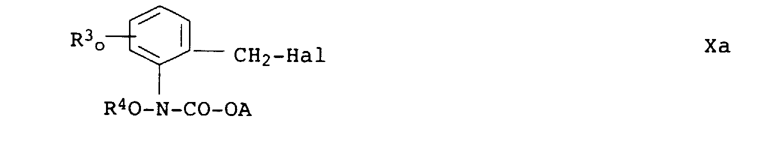 where Hal is a halogen atom, converting Xa into a compound of the formula IA 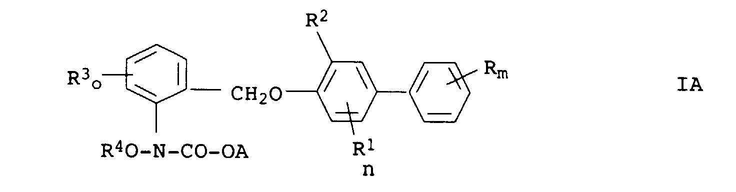 in the presence of a base using a 4-biphenol of the formula III as set forth in claim 2 and then reacting I.A with an amine of the formula XIa or XIb 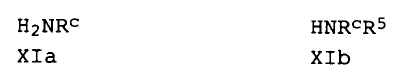 to give I.

6. A process for preparing compounds of the formula I.A as set forth in claim 5, where A is phenyl, which comprises converting an N-hydroxyaniline of the formula VA as set forth in claim 2 using a phenyl carbonate of the formula VIA
L²-CO₂-C₆H₅ VIA
where L² is a halogen atom, into the corresponding N-hydroxycarbamate of the formula IB 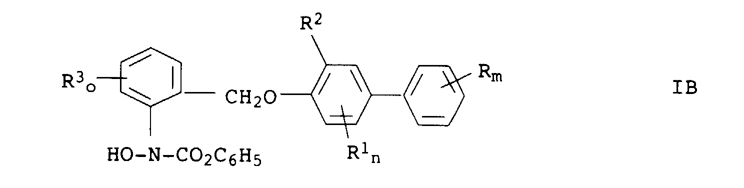 and then reacting IB with a compound VIII as set forth in claim 3 to give IA.

7. A process for preparing the compound I as claimed in claim 1, which comprises reacting a compound of the formula XIV 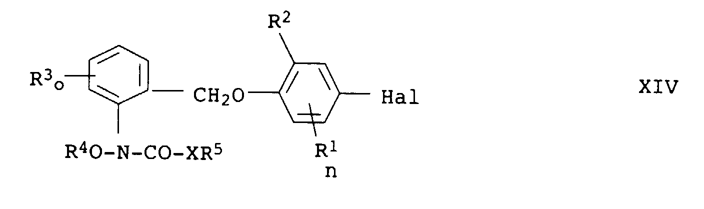 where Hal is a halogen atom, with an organometallic compound of the formula XV 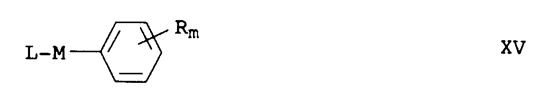 where L-M is a metal-containing radical, in the presence of a palladium catalyst.

8. An intermediate of the formula XII 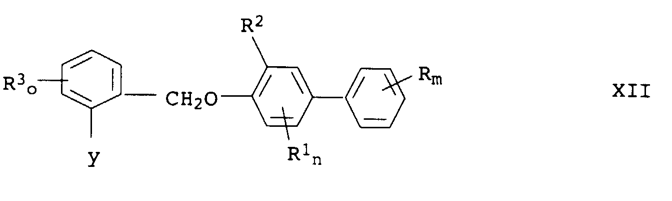 where Y is NO₂, NHOH, NHOR⁴, N(OH)-CO₂C₆H₅ or N(OR⁴)-CO₂C₆H₅ and the indices m, n and o and the substituents R, R¹, R², R³ and R⁴ have the meanings given in claim 1.

9. A composition suitable for controlling animal pests or harmful fungi, containing a solid or liquid carrier and a compound of the general formula I as claimed in claim 1.

10. The use of the compounds I as claimed in claim 1 for preparing a composition suitable for controlling animal pests or harmful fungi.

11. A method of controlling animal pests or harmful fungi, which comprises treating the pests or fungi to be controlled or the materials, plants, soil or seed to be protected from them with an effective amount of a compound of the general formula I as claimed in claim 1.

## Revendications

1. 2-[4-biphényl-oxyméthylène]-anilides de formule I 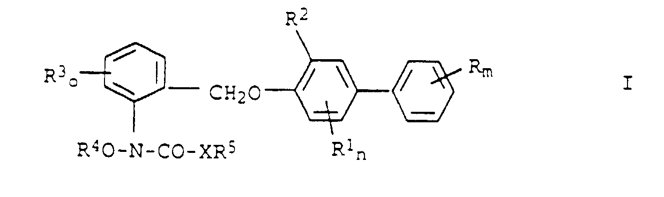 dans laquelle les indices et les substituants ont les significations suivantes:
R cyano, nitro, halogéno, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄, alkylamino en C₁-C₄, di-alkyl(C₁-C₄)amino, alkyl(C₁-C₄)carbonyle, alcoxy(C₁-C₄)carbonyle, alkyl(C₁-C₄)aminocarbonyle, di-(alkyl en C₁-C₄)aminocarbonyle, alkyl(C₁-C₄)carbonylamino, alkyl(C₁-C₄)carbonyl-(alkyl (C₁-C₄)amino), alcényle en C₂-C₄, alcényloxy en C₃-C₄, alcynyle en C₂-C₄, alcynyloxy en C₃-C₄, cycloalkyle en C₃-C₆ ou C(R^{a})=NOR^{b};
R^{a},R^{b} hydrogène, alkyle, alcényle, alcynyle, cycloalkyle ou cycloalcényle;
m 1, 2, 3, 4 ou 5, tandis que les restes R peuvent être différents, lorsque m est supérieur à 1;
R¹ cyano, nitro, halogéno, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄, alkylamino en C₁-C₄, di-alkyl(C₁-C₄)amino, alkyl(C₁-C₄)carbonyle, alcoxy(C₁-C₄)carbonyle, alkyl(C₁-C₄)aminocarbonyle, di-(alkyl en C₁-C₄)aminocarbonyle, alkyl(C₁-C₄)carbonylamino, alkyl(C₁-C₄)carbonyl-(alkyl (C₁-C₄)amino), alcényle en C₂-C₄, alcényloxy en C₃-C₄, alcynyle en C₂-C₄, alcynyloxy en C₃-C₄, cycloalkyle en C₃-C₆ ou C(R^{a})=NOR^{b};
n 0, 1 ou 2, tandis que les restes R¹ peuvent être différents, lorsque n vaut 2;
R² cyano, nitro, halogéno, alkyle en C₁-C₄, halogénoalkyle en C₁-C₄, alcoxy en C₁-C₄, halogénoalcoxy en C₁-C₄, alkylthio en C₁-C₄, halogénoalkylthio en C₁-C₄, alkylamino en C₁-C₄, di-alkyl(C₁-C₄)amino, alkyl(C₁-C₄)carbonyle, alcoxy(C₁-C₄)carbonyle, alkyl(C₁-C₄)aminocarbonyle, di-(alkyl en C₁-C₄)aminocarbonyle, alkyl(C₁-C₄)carbonylamino, alkyl(C₁-C₄)carbonyl-(alkyl(C₁-C₄)amine), alcényle en C₂-C₄, alcényloxy en C₃-C₄, alcynyle en C₂-C₄, alcynyloxy en C₃-C₄, cycloalkyle en C₃-C₆ ou C(R^{a})=NOR^{b};
R³ nitro, cyano, halogéno,
alkyle, alcényle, alcynyle, alcoxy, alcényloxy, alcynyloxy, éventuellement substitués, ou
alkyl(C₁-C₄)carbonyle, alcoxy(C₁-C₄)carbonyle, alkyl(C₁-C₄)aminocarbonyle, di-(alkyl en C₁-C₄)aminocarbonyle, alkyl(C₁-C₄)carbonylamino, alkyl(C₁-C₄)carbonyl-(alkyl(C₁-C₄)amine), C(R^{a}) =NOR^{b} ou dans le cas où o est supérieur à 1, en outre un pont éventuellement substitué relié à deux atomes de cycle voisins, et qui possède trois à quatre membres du groupe à 3 ou 4 atomes de carbone ou à 2 ou 3 atomes de carbone et 1 ou 2 atomes d'azote, d'oxygène et/ou de soufre, tandis que ce pont peut former conjointement avec le cycle auquel il est relié un reste partiellement insaturé ou aromatique;
o 0, 1, 2, 3 ou 4, tandis que les substituants R³ peuvent être différents, lorsque n est supérieur à 1;
R⁴ hydrogène,
alkyle, alcényle, alcynyle, cycloalkyle, cycloalcényle, alkylcarbonyle ou alcoxycarbonyle, éventuellement substitués;
X une liaison directe ou CH₂, O ou NR^{c};
R^{c} hydrogène, alkyle, alcényle, alcynyle, cycloalkyle ou cycloalcényle;
R⁵ alkyle, alcényle, alcynyle, cycloalkyle ou cycloalcényle, ou
dans le cas où X désigne NR^{c}, en outre hydrogène.

2. Procédé pour la préparation de composés de formule I selon la revendication 1, dans lesquels R⁴ représente l'hydrogène et X désigne une liaison directe ou l'oxygène, caractérisé par le fait qu'on convertit un dérivé benzylique de formule II 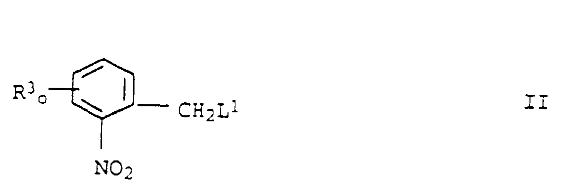 où L¹ désigne un groupe échangeable par voie nucléophile, en présence d'une base avec un 4-biphénol de formule III 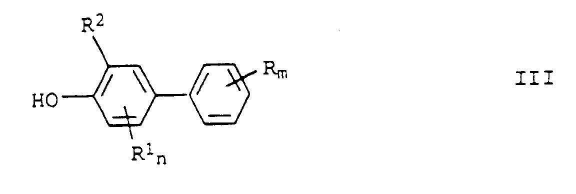 en le 2-[4-biphényl-oxyméthylène]-nitrobenzène correspondant de formule IV 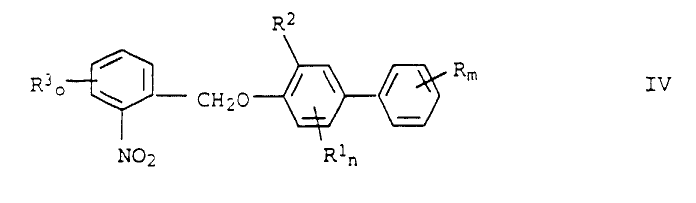 on réduit ensuite IV en la N-hydroxylaniline de formule Va 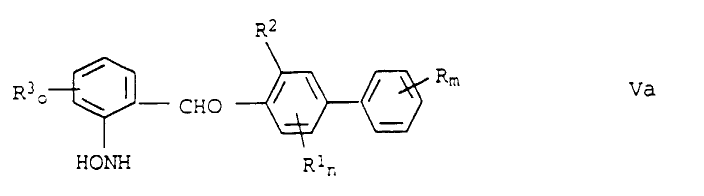 et on transforme Va en I avec un composé carbonylé de formule VI
L²-CO-X-R⁵ VI
où L² représente un halogène.

3. Procédé pour la préparation des composés de formule I selon la revendication 1 dans lesquels R⁴ ne représente pas l'hydrogène, caractérisé par le fait qu'on réduit un dérivé benzylique de formule IIa 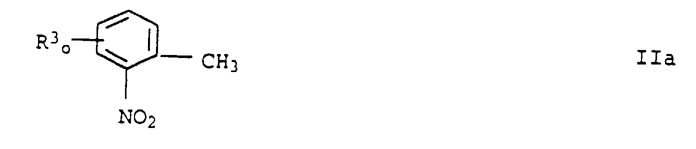 d'abord en la N-hydroxyaniline correspondante de formule Ib 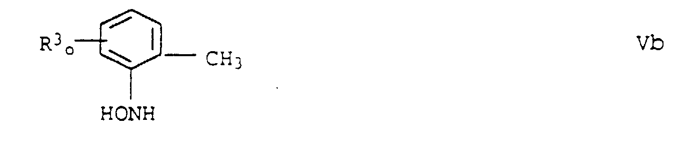 on transforme Vb avec un composé carbonylé de formule VI selon la revendication 2 en l'anilide correspondant de formule VII 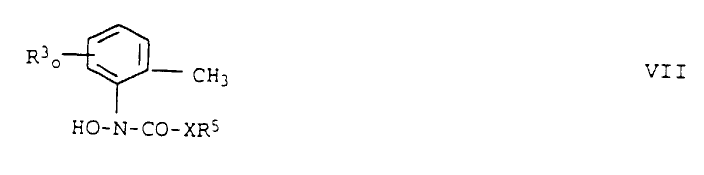 et on convertit ensuite VII avec un composé VIII
L³-R⁴ VIII
dans lequel L³ désigne un groupe échangeable par voie nucléophile et R⁴ ne représente pas l'hydrogène, en l'amide de formule IX 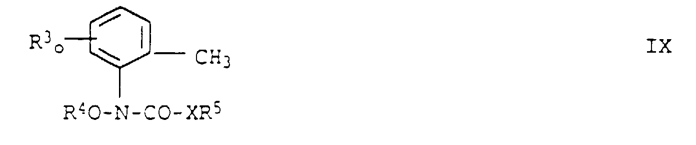 on convertit ensuite IX en l'halogénure de benzyle correspondant de formule X 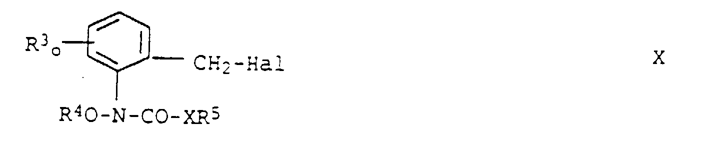 où Hal désigne un atome d'halogène, et on convertit X en I, en présence d'une base, avec un 4-biphénol de formule III selon la revendication 2.

4. Procédé pour la préparation des composés I selon la revendication 1 dans lesquels R⁴ ne représente pas l'hydrogène, caractérisé par le fait qu'on fait réagir un composé correspondant de formule I, dans lequel R⁴ désigne l'hydrogène, avec un composé correspondant de formule VIII selon la revendication 3.

5. Procédé pour la préparation des composés de formule I dans lesquels X représente NR^{c}, caractérisé par le fait qu'on convertit un benzanilide de formule IXa 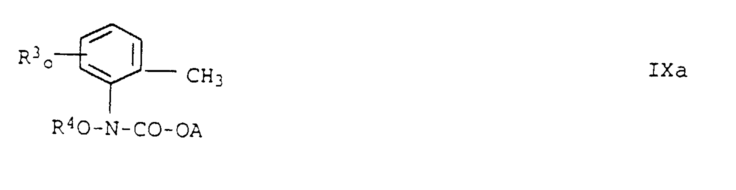 dans lequel A représente un groupe alkyle ou phényle, en l'halogénure de benzyle correspondant de formule Xa 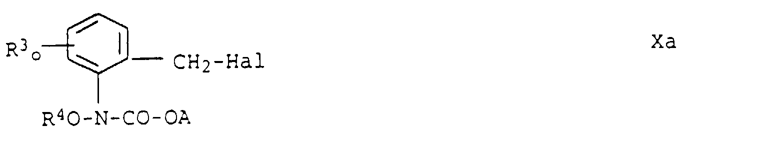 où Hal désigne un atome d'halogène, on convertit Xa, en présence d'une base, avec un 4-biphénol de formule III selon la revendication 2 en un composé de formule I.A 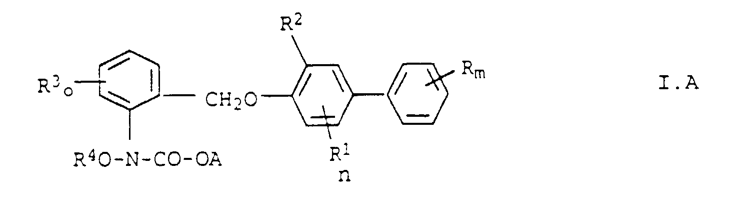 et on fait ensuite réagir I.A avec une amine de formule XIa ou XIb 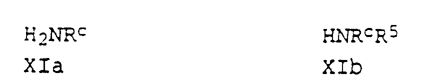 pour obtenir I.

6. Procédé pour la préparation de composés de formule I.A selon la revendication 5, dans lesquels A représente un groupe phényle, caractérisé par le fait qu'on convertit une N-hydroxylaniline de formule VA selon la revendication 2 avec un ester phénylique d'acide carboxylique de formule VIA
L²-CO₂-C₆H₅ VIA
où L² désigne un atome d'halogène, en le N-hydroxycarbamate correspondant de formule IB 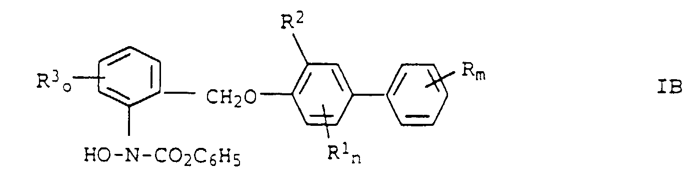 et on fait ensuite réagir IB avec un composé VIII selon la revendication 3 pour obtenir IA.

7. Procédé pour la préparation du composé I selon la revendication 1, caractérisé par le fait qu'on fait réagir un composé de formule XIV 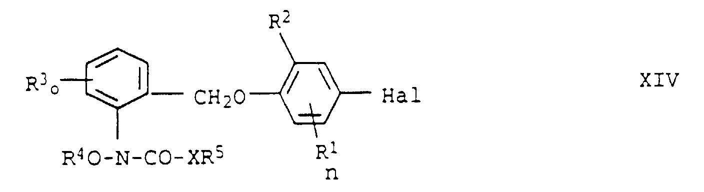 où Hal désigne un atome d'halogène, en présence d'un catalyseur au palladium, avec un composé organométallique de formule XV 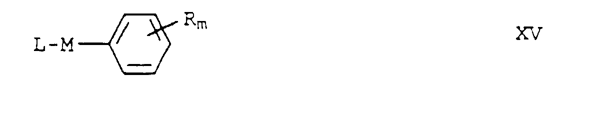 dans laquelle L-M représente un reste contenant des métaux.

8. Produits intermédiaires de formule XII 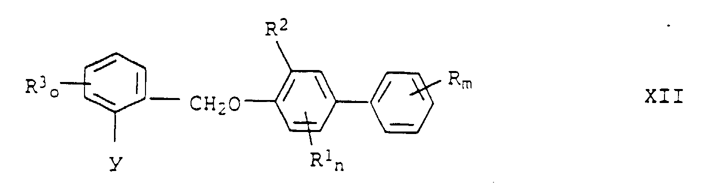 dans laquelle Y désigne NO₂, NHOH, NHOR⁴, N(OH)-CO₂C₆H₅ ou N(OR⁴)-CO₂C₆H₅ et les indices m, n et o et les substituants R, R¹, R², R³ et R⁴ ont la signification indiquée dans la revendication 1.

9. Moyens appropriés pour la lutte contre les parasites animaux ou les champignons nuisibles, contenant un support solide ou liquide et un composé de formule générale I selon la revendication 1.

10. Utilisation des composés I selon la revendication 1 pour la préparation d'un moyen approprié pour la lutte contre les parasites animaux ou les champignons nuisibles.

11. Procédé pour la lutte contre les parasites animaux ou les champignons nuisibles, caractérisé par le fait qu'on traite les parasites ou champignons à combattre ou les matériels, plantes, sols ou semences à protéger contre eux avec une quantité efficace d'un composé de formule générale I selon la revendication 1.
